# EUROPÄISCHE PATENTSCHRIFT

(11) **EP 1 129 071 B1**
(45) Veröffentlichungstag und Bekanntmachung des Hinweises auf die Patenterteilung: **26.10.2005**
(21) Anmeldenummer: 99955944.6
(22) Anmeldetag: 02.11.1999
(51) Int. Cl.: C07D 207/20, A01N 43/36, A01N 43/40, A01N 43/78, C07D 211/70, C07D 401/06, C07D 417/14, C07D 207/22, C07D 401/10, C07D 223/04, C07D 215/14, C07D 409/06, C07D 417/06, C07D 403/06, C07D 405/06, C07C 255/40, C07C 323/62, C07C 317/44

(54) **PHENYL-SUBSTITUIERTE ZYKLISCHE ENAMINONE**
PHENYL-SUBSTITUTED CYCLIC ENAMINONES
ENAMINOMES CYCLIQUES A GROUPE PHENYLE SUBSTITUE

(30) Priorität: 11.11.1998 DE 19851986
(43) Veröffentlichungstag der Anmeldung: 05.09.2001
(73) Patentinhaber: Bayer CropScience AG, 40789 Monheim (DE)
(72) Erfinder: FISCHER, Reiner, D-40789 Monheim (DE); WISCHNAT, Ralf, D-51371 Leverkusen (DE); DREWES, Mark, Wilhelm, D-40764 Langenfeld (DE); DOLLINGER, Markus, Kansas, KS 66213 (US); ERDELEN, Christoph, D-42799 Leichlingen (DE); FEUCHT, Dieter, D-40789 Monheim (DE); WETCHOLOWSKY, Ingo, CEP 13280-000 Vinhedo, SP (BR); WACHENDORFF-NEUMANN, Ulrike, D-56566 Neuwied (DE); PHILIPP, Ulrich, D-50674 Köln (DE); RAUCH, Olga-Tatjana, D-40764 Langenfeld (DE)
(86) Internationale Anmeldenummer: PCT/EP1999/008366
(87) Internationale Veröffentlichungsnummer: WO 2000/027812

(56) Entgegenhaltungen:
- EP-A- 0 434 132
- EP-A- 0 490 220
- DE-A- 3 529 259
- US-A- 4 297 361
- DATABASE CHEMABS [Online] CHEMICAL ABSTRACTS SERVICE, COLUMBUS, OHIO, US GLUSHKOV, R. G. ET AL: "Synthesis and antibacterial activity of 1,2-polymethylene-4-quinolone-3- carboxylic acids" retrieved from STN Database accession no. 113:148774 XP002127173 & KHIM.-FARM. ZH. (1990), 24(7), 24-7 ,

## Beschreibung

Die Erfindung betrifft neue Phenyl-substituierte zyklische Enaminone, mehrere Verfahren zu ihrer Herstellung, Zwischenprodukte und ihre Verwendung als Pflanzenschutzmittel, besonders als Herbizide, Akarizide, Nematizide und Insektizide.

Es sind bereits bestimmte im Phenylring substituierte zyklische Enaminone als Zwischenprodukte für antibakteriell wirksame Chinolone bekannt (R. G. Glushkov, N. B. Marchenko, A. N. Padeiskaya, L. D. Shipilova, Pharm. Chem. J. (Engl. Transl.) 24, 460-465, (1990)). Weiterhin bekannt sind im Phenylring unsubstituierte zyklische Enaminone (M. V. Mezentseva, A. V. Kadushkin, L. M. Alekseeva, A. S. Sokolova, V. G. Granik, Pharm. Chem. J. (Engl. Transl.) 25, 858-864 (1991); G. M. Coppola, R. Damon, A. D. Kahle, M. J. Shapiro, J. Org. Chem. 46, 1221-1222, (1981); D. Brillon, G. Sauvé, J. Org. Chem. 55, 2246-2249, (1990)). Eine Verwendung dieser Verbindungen als Pflanzenschutzmittel wurde bisher nicht beschrieben.

Die US 4,297,361 beschreibt insektizid wirksame Propionitrile, die jedoch eine Thiazoliden-Gruppe enthalten und sich dadurch von den erfindungsgemäßen Verbindungen unterscheiden.

Die neuen zyklischen Enaminone werden allgemein durch die Formel (I) beschrieben, 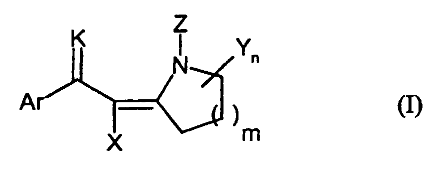 in welcher
- Ar: für Ar¹ steht, wobei Ar¹ für jeweils gegebenenfalls (jedoch im Falle von Phenyl und Naphthyl nicht für unsubstituiertes) einfach bis fünffach durch Halogen, C₁-C₈-Alkyl, C₂-C₈-Alkenyl, C₂-C₈-Alkinyl, C₁-C₈-Alkoxy, C₂-C₈-Alkenyloxy, C₃-C₈-Alkinyloxy, C₁-C₈-Alkylthio, C₁-C₆-Alkylsulfinyl, C₁-C₆-Alkylsulfonyl, C₁-C₆-Halogenalkyl, C₁-C₆-Halogen-alkoxy, C₂-C₈-Halo-genalkenyloxy, C₁-C₂-Alkylidendiyl-dioxy, C₁-C₂-Halogenalkylidendiyl-di-oxy, Halogen-C₁-C₄-alkylthio, Halogen-C₁-C₄-alkylsulfinyl, Halogen-C₁-C₄-alkylsulfonyl, Hydroxy, Mercapto, Nitro, Cyano, Amino oder durch die Gruppen 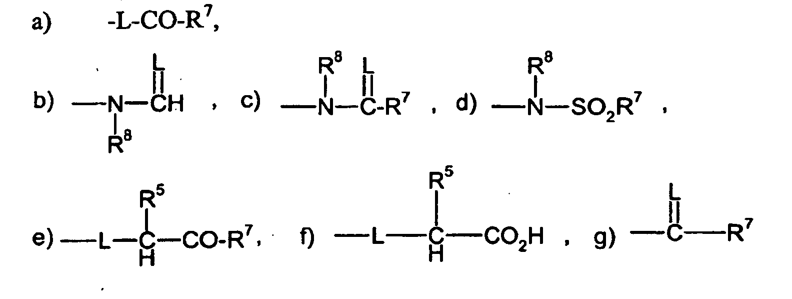 substituiertes Phenyl, Naphtyl oder mono- oder bicyclisches Hetaryl mit fünf bis zehn Ringatomen steht,
oder für Ar² steht, wobei Ar² für Ar¹ steht, welches zusätzlich durch Phenyl, Naphthyl, fünf- oder sechsgliedriges Hetaryl, Phenyl-C₁-C₄-alkyl, Phenoxy, Phenyl-S(O)_{g}-, fünf- oder sechsgliedriges Hetaryloxy oder Hetaryl-S(O)_{g} substituiert ist, wobei diese Substituenten ihrerseits jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Nitro oder Cyano substituiert sind.
- K: für Sauerstoff oder Schwefel steht,
- L: für Sauerstoff oder Schwefel steht,
- X: für

CN,

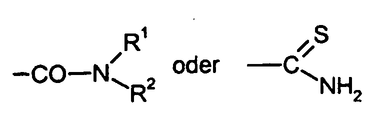

steht,
- Y: für Halogen, C₁-C₆-Alkyl, C₁-C₄-Halogenalkyl, C₁-C₆-Alkoxy, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Phenyl-C₁-C₄-alkyl, fünf- oder sechsgliedriges Hetaryl oder fünf- oder sechsgliedriges Hetaryl-C₁-C₄-alkyl oder für die Gruppen

―CO₂R¹

oder 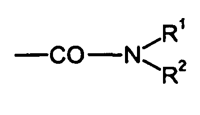 steht, oder
zwei benachbarte Yₙ für einen 5- bis 8-gliedrigen, gesättigten oder ungesättigten Cyclus stehen, der durch 1 bis 3 Heteroatome aus der Reihe N, O, S unterbrochen sein kann und gegebenenfalls einfach bis dreifach durch Halogen, C₁-C₄-Alkyl, C₁-C₄-Alkoxy, C₁-C₄-Halogenalkyl, C₁-C₄-Halogenalkoxy, Cyano oder Nitro substituiert sein kann, und
- Z: für Wasserstoff, für jeweils gegebenenfalls durch Fluor und/oder Chlor substituiertes C₁-C₈-Alkyl, Cyano-C₁-C₆-alkyl, C₃-C₈-Alkenyl, C₃-C₈-Alkinyl, C₃-C₈-Cycloalkyl, C₃-C₈-Cycloalkyl- C₁-C₂-alkyl, C₁-C₄-Alkoxy-C₁-C₄-alkyl, C₁-C₄-Halogenalkoxy-C₁-C₄-alkyl, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenoxy-C₁-C₄-alkyl, Phenyl-C₁-C₄-alkyloxy-C₁-C₄-alkyl, Phenylthio-C₁-C₄-alkyl, Phenyl-C₁-C₄-alkylthio-C₁-C₄-alkyl, Phenyl, Phenyl-C₁-C₄-alkyl, fünf- oder sechsgliedriges Hetaryl, fünf- oder sechsgliedriges Hetaryl-C₁-C₄-alkyl oder für die Gruppen

―CO₂R¹ ;

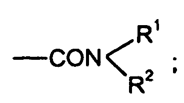

―SO₂R¹;

―COR¹;

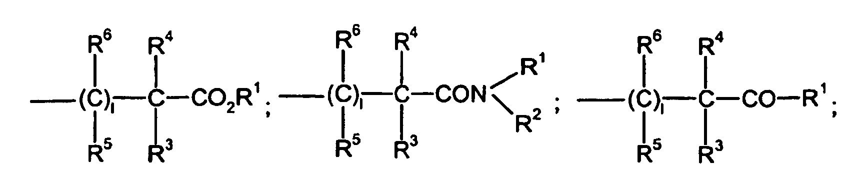

oder Cyano steht, wobei
- g: 0 bis 2 steht,
- l: 0 bis 2 steht,
- R¹: für Wasserstoff (nicht jedoch für die Reste -CO₂R¹ und -SO₂R¹), für jeweils gegebenenfalls durch Fluor und/oder Chlor substituiertes C₁-C₁₀-Alkyl, C₂-C₁₀-Alkenyl, C₃-C₆-Alkinyl, für jeweils gegebenenfalls durch Fluor, Chlor, C₁-C₄-Alkyl, C₁-C₄-Alkoxy substituiertes C₃-C₈-Cycloalkyl oder C₅-C₈-Cycloalkenyl, in welchem gegebenenfalls eine Methylengruppe durch Sauerstoff oder Schwefel unterbrochen sein kann, oder für jeweils gegebenenfalls durch einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Pyridyl, Thienyl, Pyrimidyl, Thiazolyl, Phenyl-C₁-C₄-alkyl, Pyridyl-C₁-C₂-alkyl, Thiazolyl-C₁-C₂-alkyl steht,
- R²: für Wasserstoff, für jeweils gegebenenfalls durch Fluor und/oder Chlor substituiertes C₁-C₆-Alkyl, C₃-C₆-Alkenyl, C₃-C₆-Alkinyl, C₁-C₆-Alkoxy, C₃-C₆-Alkenyloxy oder für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Phenyl-C₁-C₄-alkyl oder Phenyl-C₁-C₄-alkoxy steht, oder
- R¹,R²: gemeinsam mit dem Stickstoffatom, an das sie gebunden sind, für einen jeweils gegebenenfalls durch C₁-C₄-Alkyl substituierten fünf- bis achtgliedrigen Cyclus, bei dem gegebenenfalls eine Methylengruppe durch Sauerstoff oder Schwefel ersetzt sein kann, stehen,
- R³: für Wasserstoff, gegebenenfalls durch Halogen substituiertes C₁-C₆-Alkyl oder für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Phenyl-C₁-C₂-alkyl steht,
- R⁴: für Wasserstoff oder C₁-C₆-Alkyl steht,
- R⁵, R⁶: unabhängig voneinander für Wasserstoff oder gegebenenfalls durch Fluor und/oder Chlor substituiertes C₁-C₄-Alkyl stehen,
- R⁷: für jeweils gegebenenfalls durch Fluor und/oder Chlor substituiertes C₁-C₁₀-Alkyl, C₂-C₁₀-Alkenyl, C₃-C₁₀-Alkinyl, C₁-C₁₀-Alkoxy, C₁-C₄-Alkoxy-C₁-C₄-alkyl, C₁-C₄-Alkylthio-C₁-C₄-alkyl, jeweils gegebenenfalls durch Fluor und/oder Chlor, C₁-C₄-Alkyl, C₁-C₄-Alkoxy substituiertes C₃-C₈-Cycloalkyl oder C₃-C₈-Cycloalkoxy, in welchem gegebenenfalls eine Methylengruppe durch Sauerstoff oder Schwefel ersetzt sein kann, für jeweils gegebenenfalls einfach bis vierfach durch Halogen, C₁-C₆-Alkyl, C₁-C₆-Alkoxy, C₁-C₄-Halogen-alkyl, C₁-C₄-Halogenalkoxy, Cyano oder Nitro substituiertes Phenyl, Phenoxy, Benzyloxy, fünf- oder sechsgliedriges Hetaryl oder Phenyl-C₁-C₄-alkyl oder im Falle der unter Ar genannten Reste a) und c) auch für eine Gruppe 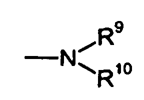 oder für den Rest g) auch für Hydroxy steht,
- R⁸: für Wasserstoff oder C₁-C₄-Alkyl steht,
- R⁹: für Wasserstoff, jeweils gegebenenfalls durch Fluor und/oder Chlor substituiertes C₁-C₁₀-Alkyl, C₃-C₈-Alkenyl, C₃-C₈-Alkinyl, C₁-C₁₀-Alkoxy, C₃-C₈-Alkenyloxy, gegebenenfalls durch Fluor, Chlor, C₁-C₄-Alkyl oder C₁-C₄-Allcoxy substituiertes C₃-C₈-Cycloalkyl, in welchem gegebenenfalls eine Methylengruppe durch Sauerstoff oder Schwefel ersetzt sein kann, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Phenyl-C₁-C₄-alkyl oder Phenyl-C₁-C₂-alkoxy steht,
- R¹⁰: für Wasserstoff, C₁-C₆-Alkyl oder C₃-C₆-Alkenyl steht, oder
- R⁹, R¹⁰: gemeinsam mit dem Stickstoffatom, an das sie gebunden sind, für einen gegebenenfalls durch C₁-C₄-Alkyl substituierten fünf- bis achtgliedrigen Cyclus, bei dem gegebenenfalls eine Methylengruppe durch Sauerstoff oder Schwefel ersetzt sein kann, stehen und
- m: für 1 bis 3 steht, und
- n: in Abhängigkeit von m für 0 bis 3 steht,
ausgenommen die Verbindungen mit der folgenden Formel: 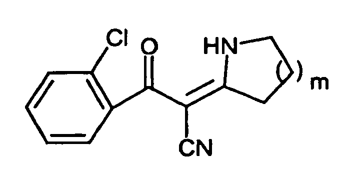 m = 1, 2, 3
- K: steht besonders bevorzugt für Sauerstoff oder Schwefel.
- Ar: steht besonders bevorzugt für Ar¹, wobei Ar¹ für jeweils gegebenenfalls einfach bis dreifach durch Fluor, Chlor, Brom, C₁-C₆-Alkyl, C₂-C₆-Alkenyl, C₂-C₆-Alkinyl, C₁-C₆-Alkoxy, C₃-C₆-Alkenyloxy, C₃-C₆-Alltinyloxy C₁-C₆-Alkylthio, C₁-C₄-Alkylsulfinyl, C₁-C₄-Alkylsulfonyl, C₁-C₄-Halogenalkyl, C₁-C₄-Halogen-alkoxy, C₂-C₄-Halogenalkenyloxy, C₁-C₂-Alkylidendiyldioxy, C₁-C₂-Halogenalkylidendiyl-dioxy, Halogen-C₁-C₂-alkylthio, Halogen-C₁-C₂-alkylsulfinyl, Halogen-C₁-C₂-alkylsulfonyl, Hydroxy, Mercapto, Nitro, Cyano, Amino oder durch eine der folgenden Gruppen 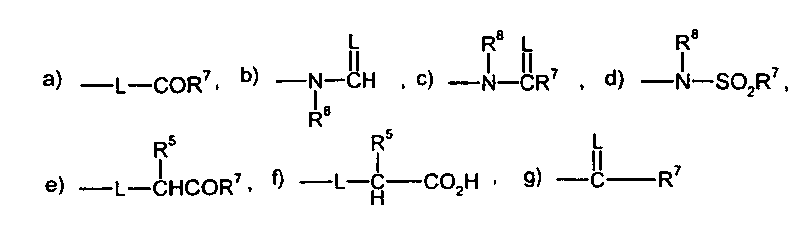 substituiertes Phenyl, Naphtyl, Chinolinyl, Thienyl, Pyrimidyl, Furanyl, Thiazolyl, Benzthiazolyl, Oxazolyl, Pyrazolyl oder Pyridyl steht,
oder für Ar², wobei Ar² für Ar¹ steht, welches zusätzlich durch Phenyl, Pyridyl, Pyrimidyl, Thienyl, Furanyl, Thiazolyl, Tetrazolyl, Triazolyl, Benzyl, Phenoxy, Phenyl-S(O)_{g}-, Pyridyloxy, Pyrimidyloxy, Thiazolyloxy, Pyridyl-S(O)_{g}-, Pyrimidyl-S(O)_{g} - oder Thiazolyl-S(O)_{g}- substituiert ist, wobei diese Substituenten ihrerseits gegebenenfalls einfach bis dreifach durch Fluor, Chlor, Brom, C₁-C₄-Alkyl, C₂C₄-Alkenyl, C₁-C₄-Alkoxy, C₁-C₂-Halogenalkyl, C₁-C₂-Halogenalkoxy, Nitro oder Cyano substituiert sind, wobei g für 0 bis 2 steht.
- L: steht besonders bevorzugt für Sauerstoff oder Schwefel.
- X: steht besonders bevorzugt für CN, 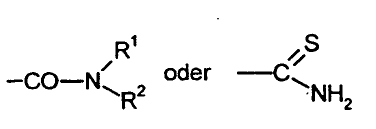
- Y: steht besonders bevorzugt für Fluor, C₁-C₄-Alkyl, C₁-C₂-Halogenalkyl, C₁-C₄-Alkoxy, für jeweils gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, Phenyl-C₁-C₂-alkyl, Thiazolylmethyl, Pyridylmethyl oder für die Gruppen

―CO₂R¹

oder 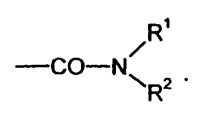 Zwei benachbarte Yₙ können weiterhin besonders bevorzugt für einen 5- bis 6-gliedrigen, gesättigten oder ungesättigten Cyclus stehen, der durch ein Heteroatom aus der Reihe N, O, S unterbrochen sein kann und gegebenenfalls einfach oder zweifach durch Fluor, Chlor, Brom, Methyl, tert-Butyl, Methoxy, Trifluormethyl, Trifluormethoxy, Cyano oder Nitro substitutiert sein kann.
- Z: steht besonders bevorzugt für Wasserstoff, für jeweils gegebenenfalls durch Fluor und/oder Chlor substituiertes C₁-C₆-Alkyl, Cyano-C₁-C₃-alkyl, C₃-C₆-Alkenyl, C₃-C₈-Alkinyl, C₃-C₆-Cycloalkyl, C₃-C₆-Cycloalkyl-C₁-C₂-alkyl, C₁-C₄-Alkoxy-C₁-C₂-alkyl, C₁-C₄-Halogen-C₁-C₂-alkyl, für jeweils gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy-C₁-C₂-alkyl, Phenyl-C₁-C₂-alkyloxy-C₁-C₂-alkyl, Phenylthio-C₁-C₂-alkyl, Phenyl-C₁-C₂-alkylthio-C₁-C₂-alkyl, Phenyl-C₁-C₂-alkyl, Phenyl, Pyridyl-C₁-C₂-alkyl, Thiazolyl-C₁-C₂-alkyl oder für die Gruppen

―CO₂R¹ ;

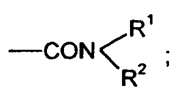

―SO₂R¹;

―COR¹;

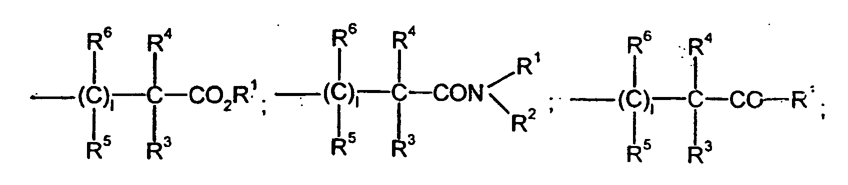

oder Cyano, wobei
- l: besonders bevorzugt für 0 bis 1 steht,
- R¹: besonders bevorzugt für Wasserstoff (jedoch nicht für die Reste -CO₂R¹ und -SO₂R¹), für jeweils gegebenenfalls durch Fluor und/oder Chlor substituiertes C₁-C₆-Alkyl, C₂-C₆-Alkenyl, C₃-C₄-Alkinyl, für gegebenenfalls durch Fluor, Chlor, C₁-C₂-Alkyl, C₁-C₂-Alkoxy substituiertes C₃-C₆-Cycloalkyl oder für jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Benzyl steht,
- R²: besonders bevorzugt für Wasserstoff, für jeweils gegebenenfalls durch Fluor und/ oder Chlor substituiertes C₁-C₄-Alkyl, C₃-C₄-Alkenyl, C₃-C₄-Alkinyl, C₁-C₄-Alkoxy, C₃-C₄-Alkenyloxy oder für jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, Benzyl oder Benzyloxy steht, oder
- R¹,R²: besonders bevorzugt weiterhin gemeinsam mit dem Stickstoffatom, an das sie gebunden sind, für einen gegebenenfalls durch C₁-C₂-Alkyl substituierten fünf- bis sechsgliedrigen Cyclus stehen können, bei dem gegebenenfalls eine Methylengruppe durch Sauerstoff ersetzt sein kann,
- R³: besonders bevorzugt für Wasserstoff, für C₁-C₄-Alkyl oder für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl oder Benzyl steht,
- R⁴, R⁵, R⁶: besonders bevorzugt für Wasserstoff, Methyl oder Ethyl stehen,
- R⁷: besonders bevorzugt für jeweils gegebenenfalls durch Fluor und/oder Chlor substituiertes C₁-C₆-Alkyl, C₂-C₆-Alkenyl, C₃-C₆-Alkinyl, C₁-C₆-Alkoxy, C₁-C₄-Alkoxy-C₁-C₂- alkyl, C₁-C₄-Alkylthio-C₁-C₂-alkyl, jeweils gegebenenfalls durch Fluor und/oder Chlor, C₁-C₂-Alkyl, C₁-C₂-Alkoxy substituiertes C₃-C₆ Cycloalkyl oder C₃-C₆-Cycloalkoxy, in welchem gegebenenfalls eine Methylengruppe durch Sauerstoff ersetzt sein kann, für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl, Phenoxy, Benzyloxy, Thienyl, Furanyl, Pyridyl, Pyrimidyl, Thiazolyl, Pyrazolyl oder Phenyl-C₁-C₂-alkyl oder im Fall der unter Ar genannten Reste a) und c) auch für eine Gruppe 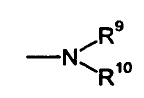 oder für den Rest g) auch für Hydroxy steht,
- R⁸: besonders bevorzugt für Wasserstoff steht,
- R⁹: besonders bevorzugt für Wasserstoff, jeweils gegebenenfalls durch Fluor und/oder Chlor substituiertes C₁-C₆-Alkyl, C₃-C₆-Alkenyl, C₃-C₆-Alkinyl, C₁-C₆-Alkoxy, C₃-C₆-Cycloalkyl, in welchem gegebenenfalls eine Methylengruppe durch Sauerstoff ersetzt sein kann, für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl oder Phenyl-C₁-C₂-alkyl steht,
- R¹⁰: besonders bevorzugt für Wasserstoff oder C₁-C₄-Alkyl steht,
- R⁹, R¹⁰: weiterhin besonders bevorzugt gemeinsam mit dem Stickstoffatom, an das sie gebunden sind, für einen gegebenenfalls durch C₁-C₂-Alkyl substituierten fünf- bis sechsgliedrigen Cyclus stehen können, bei dem gegebenenfalls eine Methylengruppe durch Sauerstoff ersetzt sein kann.
- m: steht besonders bevorzugt für 1 bis 3.
- n: steht besonders bevorzugt in Abhängigkeit von m für 0 bis 2.
- K: steht ganz besonders bevorzugt für Sauerstoff und Schwefel.
- Ar: steht ganz besonders bevorzugt für Ar¹, wobei Ar¹ für jeweils gegebenenfalls einfach bis dreifach durch Fluor, Chlor, Brom, Methyl, Ethyl, Propyl, i-Propyl, s-, n-, i- oder t-Butyl, Methoxy, Ethoxy, Propoxy, i-Propoxy, s-, n-, i- oder t-Butoxy, Allyloxy, Methallyloxy, 2-Butenyloxy, Propargyloxy, 2-Butinyloxy, Methylthio, Ethylthio, Methylsulfinyl, Ethylsulfinyl, Methylsulfonyl, Ethylsulfonyl, Trifluormethyl, Difluormethoxy, Trifluormethoxy, Methylendioxy, Difluormethylendioxy, Tetrafluorethylendioxy, Difluormethylthio, Trifluormethylthio, Trifluormethylsulfinyl, Trifluormethylsulfonyl, Hydroxy, Mercapto, Nitro, Cyano, Amino, oder durch eine der folgenden Gruppen 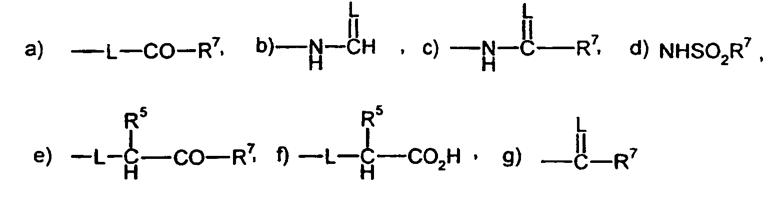 substituiertes Phenyl, Thienyl, Pyrimidyl, Furanyl oder Pyridyl steht,
oder für Ar², wobei Ar² für Ar¹ steht, welches zusätzlich durch Phenyl, Pyridyl, Thienyl, Tetrazolyl, Triazolyl oder Phenoxy substituiert ist, wobei diese Substituenten ihrerseits gegebenenfalls einfach bis zweifach durch Fluor, Chlor, Brom, Methyl, Ethyl, n- oder i-Propyl, s-, n-, i- oder t-Butyl, Methoxy, Ethoxy, i-Propoxy, s-, n- oder t-Butoxy, Trifluormethyl, Trifluormethoxy, Nitro oder Cyano substituiert sind, und
- L: ganz besonders bevorzugt für Sauerstoff oder Schwefel steht.
- X: steht ganz besonders bevorzugt für

―CN,

―CO―NH₂, 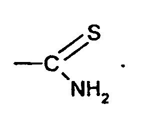
- Y: steht ganz besonders bevorzugt für Methyl, gegebenenfalls einfach bis dreifach durch Fluor, Chlor, Brom, Methyl, Methoxy, Trifluormethyl oder Trifluormethoxy substituiertes Phenyl oder für die Gruppe -CO₂R¹. Zwei benachbarte Yₙ können weiterhin ganz besonders bevorzugt für einen sechsgliedrigen, ungesättigten Cyclus stehen, der gegebenenfalls einfach durch Fluor, Chlor, Brom, Methyl, Methoxy, Trifluormethyl, Trifluormethoxy, Cyano oder Nitro substituiert sein kann
- Z: steht ganz besonders bevorzugt für Wasserstoff, Methyl, Ethyl, Allyl, Propargyl, Cyclopropyl, Cyclopentyl, Cyclohexyl, Cyclopropylmethyl, Cyclopentylmethyl, Cyclohexylmethyl, Methoxymethyl, Ethoxymethyl, für jeweils gegebenenfalls einfach bis zweifach durch Fluor, Chlor, Brom, Methyl, Methoxy, Trifluormethyl oder Trifluormethoxy, Cyano oder Nitro substituiertes Phenyl, Benzyl, Pyridylmethyl, Thiazolylmethyl oder für die Gruppen

―CO₂R¹,

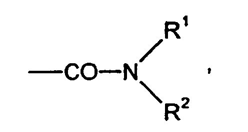

―SO₂R¹,

―CO―R¹ ,

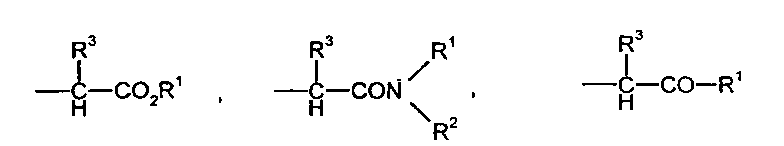

oder Cyano, wobei
- R¹: ganz besonders bevorzugt für Wasserstoff (jedoch nicht für die Reste -CO₂R¹ und -SO₂R¹), Methyl, Ethyl, Propyl, Isopropyl, n-, s-, i- oder t-Butyl, Allyl, Propargyl, Cyclopropyl, Cyclopentyl, Cyclohexyl für jeweils gegebenenfalls einfach bis zweifach durch Fluor, Chlor, Brom, Methyl, Methoxy, Trifluormethyl Trifluormethoxy, Cyano oder Nitro substituiertes Phenyl oder Benzyl steht,
- R²: ganz besonders bevorzugt für Wasserstoff, Methyl, Ethyl, Propyl, Isopropyl, Allyl, Propargyl, Methoxy, Ethoxy, Allyloxy oder für gegebenenfalls einfach bis zweifach durch Fluor, Chlor, Brom, Methyl, Methoxy, Trifluormethyl, Trifluormethoxy, Cyano oder Nitro substituiertes Benzyloxy steht, oder
- R¹, R²: gemeinsam mit dem Stickstoffatom, an das sie gebunden sind, ganz besonders bevorzugt weiterhin für einen Pyrrolidin-, Thiazin-, Piperidin- oder Morpholinrest stehen können,
- R³, R⁵: ganz besonders bevorzugt für Wasserstoff, Methyl oder Ethyl stehen,
- R⁷: ganz besonders bevorzugt für Methyl, Ethyl, Propyl, Isopropyl, n-, s-, i- oder t-Butyl, Vinyl, Trifluormethyl, Methoxy, Ethoxy, Propoxy, Isopropoxy n-, s-, i- oder t-Butyloxy, Cyclopropyl, Cyclopentyl, Cyclohexyl, Cyclopentyloxy, Cyclohexyloxy, für jeweils gegebenenfalls einfach bis zweifach durch Fluor, Chlor, Brom, Methyl, n-, s-, i- oder t-Butyl, Methoxy, Trifluormethyl, Trifluormethoxy, Cyano oder Nitro substituiertes Phenyl, Pyridyl oder Benzyl oder im Fall der unter Ar genannten Reste a) und c) auch für eine Gruppe 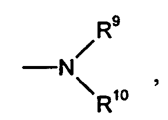 oder für den Rest g) auch für Hydroxy
- R⁹: ganz besonders bevorzugt für Wasserstoff, Methyl, Ethyl, Propyl, Isopropyl, n-, s-, i- oder t-Butyl, Cyclopropyl, Cyclopentyl, Cyclohexyl, für gegebenenfalls einfach bis zweifach durch Fluor, Chlor, Brom, Methyl, Methoxy, Trifluormethyl, Trifluormethoxy, Cyano oder Nitro substituiertes Phenyl steht,
- R¹⁰: ganz besonders bevorzugt für Wasserstoff, Methyl oder Ethyl steht,
- R⁹, R¹⁰: gemeinsam mit dem Stickstoffatom, an das sie gebunden sind, ganz besonders bevorzugt für einen Pyrrolidin-, Piperidin- oder Morpholinrest stehen.
- m: steht ganz besonders bevorzugt für 1 bis 3.
- n: steht ganz besonders bevorzugt in Abhängigkeit von m für 0 bis 1.
- K: steht insbesondere bevorzugt für Sauerstoff oder Schwefel.
- Ar: steht insbesondere bevorzugt für Ar¹, wobei Ar¹ für gegebenenfalls einfach bis dreifach durch Fluor, Chlor, Brom, Methyl, Ethyl, Propyl, Isopropyl, n-, s-, i- oder t-Butyl, Methoxy, Ethoxy, Propoxy, Isopropoxy, n-, s-, i- oder t-Butoxy, Allyloxy, Methallyloxy, 2-Butenyloxy, Propargyloxy-2-Butinyloxy, Methylthio, Ethylthio, Methylsulfinyl, Ethylsulfinyl, Methylsulfonyl, Ethylsulfonyl, Trifluormethyl, Difluormethoxy, Trifluormethoxy, Methylendioxy, Difluormethylendioxy, Tetrafluorethylendioxy, Difluormethylthio, Trifluormethylthio, Trifluormethylsulfinyl, Trifluormethylsulfonyl, Hydroxy, Nitro, Mercapto, Cyano, Amino substituiertes Phenyl, Thienyl, Pyrimidyl oder Pyridyl steht
oder für Ar², wobei Ar² für Ar¹ steht, welches zusätzlich durch Phenyl oder Phenoxy substituiert ist, wobei diese Substituenten ihrerseits gegebenenfalls einfach bis zweifach durch Fluor, Chlor, Brom, Methyl, Ethyl, Isopropyl, n-, s-, i- oder t-Butyl, Methoxy, Ethoxy, Isopropoxy, n-, s-, i- oder t-Butoxy, Trifluormethyl, Trifluormethoxy, Nitro oder Cyano substituiert sind.
- X: steht insbesondere bevorzugt für CN.
- Z: steht insbesondere bevorzugt für Wasserstoff und Methyl.
- m: steht insbesondere bevorzugt für 1 bis 3.
- n: steht insbesondere bevorzugt für 0.

Alle oben aufgeführten Verbindungen der Formel (I) können sowohl als cis- als auch trans-Isomere vorliegen. Zur Vereinfachung der Darstellung wurde bei der formelmäßigen Beschreibung der Verbindungen jeweils nur ein Isomeres angegeben. Das jeweils andere Isomer ist jedoch erfindungsgemäß ebenfalls gemeint.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- und Zwischenprodukte. Diese Restedefinitionen können untereinander, also auch zwischen den angegebenen bevorzugten Bereichen beliebig kombiniert werden.

Erfindungsgemäß besonders bevorzugt sind die Verbindungen der Formel (I), in welchen eine Kombination der vorstehend als besonders bevorzugt aufgeführten Bedeutungen vorliegt.

Erfindungsgemäß ganz besonders bevorzugt sind die Verbindungen der Formel (I), in welchen eine Kombination der vorstehend als ganz besonders bevorzugt aufgeführten Bedeutungen vorliegt.

Erfindungsgemäß insbesondere bevorzugt sind die Verbindungen der Formel (I), in welcher eine Kombination der vorstehend als insbesondere bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Beispiele für die erfindungsgemäßen Verbindungen der Formel (I) sind in den nachstehenden Gruppen aufgeführt:

### Gruppe 1:

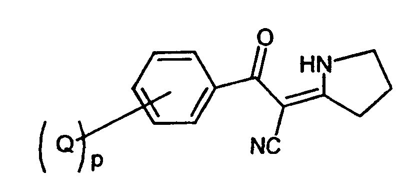

(Q)ₚ (p = 1 bis 3) hat hierbei beispielhaft die in der nachstehenden Aufzählung angegebenen Bedeutungen:
Fluor, Chlor, Brom, Methyl, Ethyl, Propyl, Isopropyl, n-, s-, i- oder t-Butyl, Methoxy, Ethoxy, Propoxy, Isopropoxy, n-, s-, i- oder t-Butoxy, Allyloxy, Methallyloxy, 2-Butenyloxy, Propargyloxy, 2-Butinyloxy, Methylthio, Ethylthio, Methylsulfinyl, Ethylsulfinyl, Methylsulfonyl, Ethylsulfonyl, Trifluormethyl, Difluormethoxy, Trifluormethoxy, Methylendioxy, Difluormethylendioxy, Tetrafluorethylendioxy, Difluormethylthio, Trifluormethylthio, Trifluormethylsulfinyl, Trifluormethylsulfonyl, Hydroxy, Mercapto, Nitro, Cyano, Amino.

### Gruppe 2:

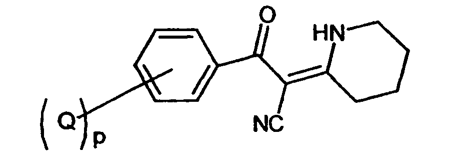

Q und p haben hierbei beispielhaft die oben in Gruppe 1 angegebenen Bedeutungen.

### Gruppe 3:

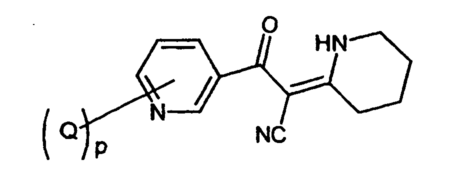

Q und p haben hierbei beispielhaft die oben in Gruppe 1 angegebenen Bedeutungen (aber p = 0 bis 2).

### Gruppe 4:

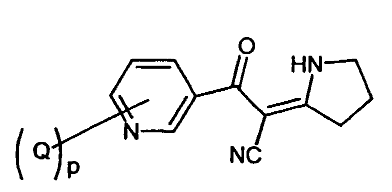

Q und p haben hierbei beispielhaft die oben in Gruppe 1 angegebenen Bedeutungen (aber p = 0 bis 2).

### Gruppe 5:

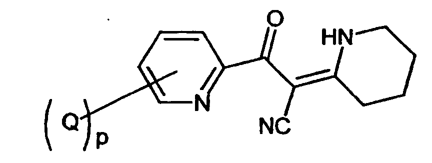

Q und p haben hierbei beispielhaft die oben in Gruppe 1 angegebenen Bedeutungen (aber p = 0 bis 2).

### Gruppe 6:

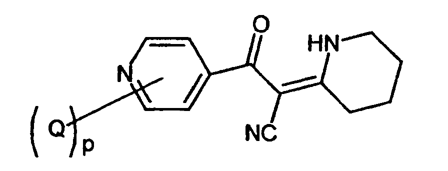

Q und p haben hierbei beispielhaft die oben in Gruppe 1 angegebenen Bedeutungen (aber p = 0 bis 2).

### Gruppe 7:

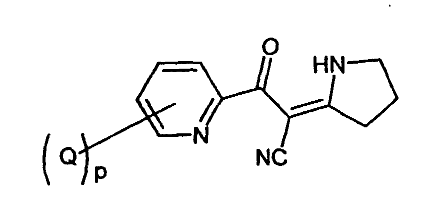

Q und p haben hierbei beispielhaft die oben in Gruppe 1 angegebenen Bedeutungen (aber p = 0 bis 2).

### Gruppe 8:

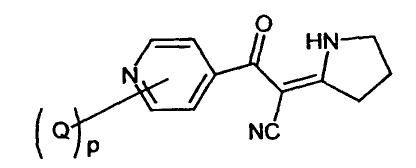

Q und p haben hierbei beispielhaft die oben in Gruppe 1 angegebenen Bedeutungen (aber p = 0 bis 2).

### Gruppe 9:

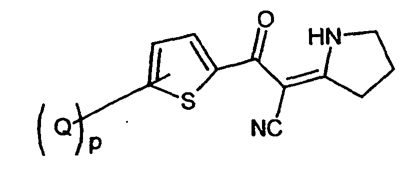

Q und p haben hierbei beispielhaft die oben in Gruppe 1 angegebenen Bedeutungen (aber p = 0 bis 2).

### Gruppe 10:

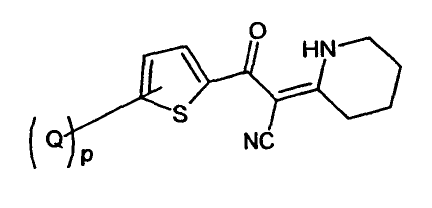

Q und p haben hierbei beispielhaft die oben in Gruppe 1 angegebenen Bedeutungen (aber p = 0 bis 2).

### Gruppe 11:

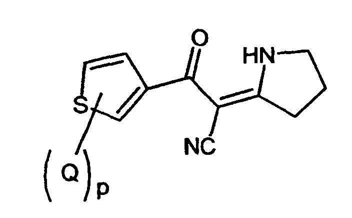

Q und p haben hierbei beispielhaft die oben in Gruppe 1 angegebenen Bedeutungen (aber p = 0 bis 2).

### Gruppe 12:

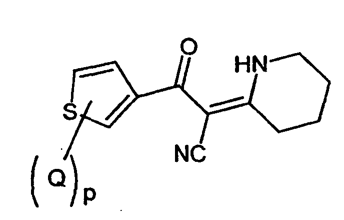

Q und p haben hierbei beispielhaft die oben in Gruppe 1 angegebenen Bedeutungen (aber p = 0 bis 2).

Es wurde gefunden, daß man die neuen Verbindungen der Formel (I) nach dem im folgenden beschriebenen Verfahren erhält:
(A) Man erhält Verbindungen der Formel (I), 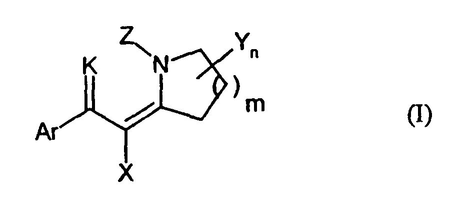 in welcher
   - Ar, X, Y, m, n: die oben angegebenen Bedeutungen haben, und
   - K: für Sauerstoff steht sowie
   - Z: für Wasserstoff steht,
   wenn man
   Verbindungen der Formel (II), 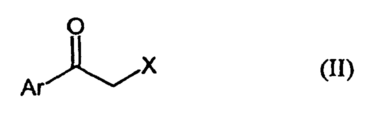 in welcher
   - Ar und X: die oben angegebenen Bedeutungen haben, mit Verbindungen der Formel (III), 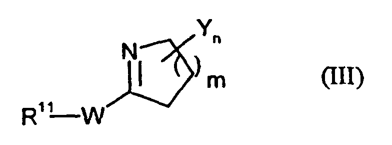 in welcher
   - Y, m, n: die oben angegebenen Bedeutungen haben, und
   - W: für O oder S(O)g, wobei g für 0 oder 2 steht, und
   - R¹¹: für Alkyl, insbesondere für C₁-C₆ -Alkyl, oder Benzyl steht,
   gegebenenfalls in Gegenwart eines Verdünnungsmittels und gegebenenfalls in Gegenwart einer Base oder einer Säure und/oder einer Metallverbindung der Formel (IIIa),

   Me(V)₂ (IIIa)

   in welcher
   - Me: für ein zweiwertiges Übergangsmetallatom, insbesondere Nickel, steht, und
   - V: für einen Chelatliganden, insbesondere für einen zweizähnigen Chelatliganden, wie z.B. Acetylacetonat, steht, umsetzt (R.G. Glushkov et al., Khim.-Farm. Zh. 24, (7), (1990), 24-27; M.V. Mezentseva et al., Khim.-Farm. Zh. 25,(12), (1991), 19-23; G. Dannhardt, A. Bauer, Pharmazie 51,(1996), 805-810).
(B) Außerdem wurde gefunden, daß man Verbindungen der Formel (I), 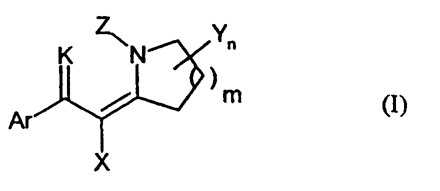 in welcher
   - Ar, Y, X, m, n: die oben angegebenen Bedeutungen haben, und
   - K: für Sauerstoff steht sowie
   - Z: für Wasserstoff steht,
   erhält, wenn man Verbindungen der Formel (IV), 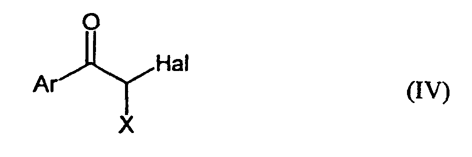 in welcher
   - Ar und X: die oben angegebene Bedeutung haben, und
   - Hal: für Halogen, insbesondere Chlor oder Brom, steht,
   mit Verbindungen der Formel (V), 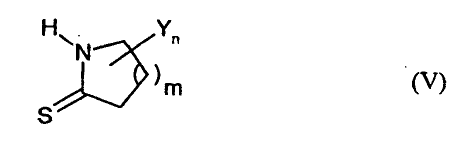 in welcher
   - Y, m und n: die oben angegebenen Bedeutungen haben, gegebenenfalls in Gegenwart eines Verdünnungsmittels zu Verbindungen der
   Formel (VI) umsetzt, 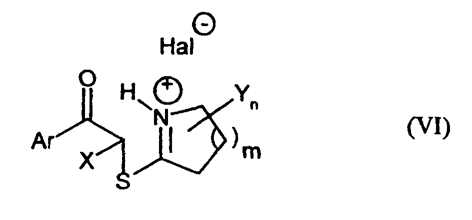 in welcher
   - Ar, X, Y, m und n: die oben angegebenen Bedeutungen haben,
   die gegebenenfalls in Gegenwart einer Base und gegebenenfalls in Gegenwart einer dreiwertigen Phosphorverbindung (z.B. Triphenylphoshin, Triethylphosphit) unter Abspaltung von Schwefel und Halogenwasserstoff zu Verbindungen der Formel (I),
   in welcher
   - Ar, X, Y, m und n: die oben angegebenen Bedeutungen haben und
   - Z: für Wasserstoff steht
   weiterreagieren (siehe A. Eschenmoser et al., Helv. Chim. Acta 54, (1971), 710-734; V. Issartel et al., C.R. Acad. Sci., Ser. II, Mec., Phys., Chim., Astron. 321, (12), (1995), 521-524).
(C) Weiterhin wurde gefunden, daß man Verbindungen der Formel (I), 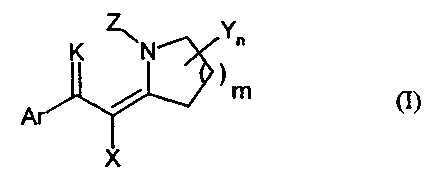 in welcher
   - Ar, Z, X, Y, m und n: eine der oben angegebenen Bedeutungen haben, und
   - K: für Sauerstoff steht, aber
   - Z: nicht für Wasserstoff steht, erhält, wenn man
   Verbindungen der Formel (VII), 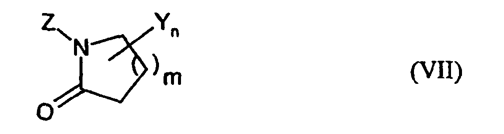 in welcher
   - Y, Z, m und n: eine der oben angegebenen Bedeutungen haben,
   und Z nicht für Wasserstoff steht,
   mit Halogenierungsmitteln, wie beispielsweise Phosgen, Diphosgen und Triphosgen, gegebenenfalls in Gegenwart eines Verdünnungsmittels zu Verbindungen der Formel (VIII), 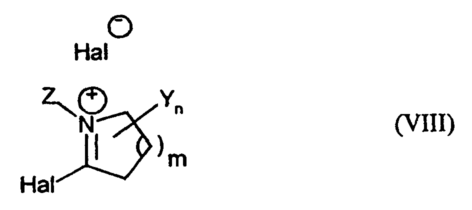 in welcher
   - Y, Z, m und n: eine der oben angegebenen Bedeutungen haben,
   und Z nicht für Wasserstoff steht, und
   - Hal: für Halogen, insbesondere Chlor oder Brom steht, umsetzt,
   die dann mit Verbindungen der Formel (II), 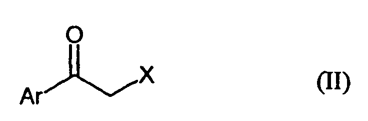 in welcher
   - Ar, X: die oben angegebenen Bedeutungen haben,
   gegebenenfalls in Gegenwart eines Verdünnungsmittels und gegebenenfalls in Gegenwart eines Säureakzeptors umgesetzt werden (siehe G. Dannhardt, A.
   Bauer, Pharmazie 51, (1996), 805-810).
(D) Weiterhin wurde gefunden, daß man Verbindungen der Formel (I), 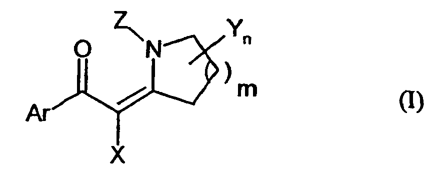 in welcher
   - Ar, Z, X,Y, m und n: eine der oben angegebenen Bedeutungen haben und
   - K: für Sauerstoff steht, aber
   - Z: nicht für Wasserstoff steht,
   erhält, wenn man
   Verbindungen der Formel (I-a), 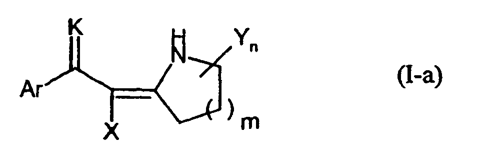 in welcher
   - Ar, X, Y, m und n: die oben angegebene Bedeutung haben,
   mit Alkylierungsmittel, Acylierungsmitteln, Sulfonylierungsmitteln oder Kondensationsmitteln der Formel (IX)

   Z-G (IX),

   in welcher
   - G: für eine Fluchtgruppe wie Halogen, insbesondere Iod, Brom, Chlor, Sulfonat, wie z.B. Mesylat, Triflat oder Toluolsulfonat, oder Alkoxy stehen,
   gegebenenfalls in Gegenwart eines Lösungsmittels und gegebenenfalls in Gegenwart einer Base umsetzt.
(E) Außerdem wurde gefunden, daß man Verbindungen der Formel (I), 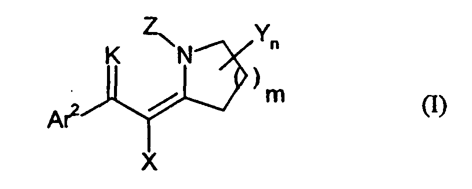 in welcher
   - Ar², X, Y, Z, m und n: die oben angegebene Bedeutung haben, und
   - K: für Sauerstoff steht,
   erhält, wenn man Verbindungen der Formel (I¹) 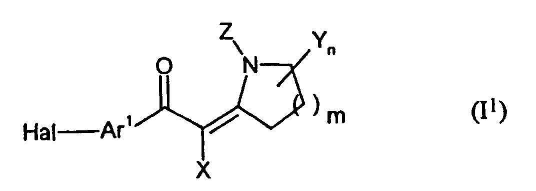 in welcher
   - Ar¹, X, Y, Z, m und n: die oben angegebene Bedeutung haben
   und
   - Hal: für Halogen, insbesondere für Brom, steht,
   mit Boronsäuren der Formel (X)

   Ar^{2'}―B(OH)₂ (X),

   in welcher
   - Ar^{2'}: für die Substituenten steht, die oben unter Ar² als zusätzliche Substituenten für Ar¹ genannt wurden,
   in Gegenwart eines Lösungsmittels, gegebenenfalls in Gegenwart einer Base und eines Edelmetallkomplexes, bevorzugt eines Palladiumkomplexes, umsetzt.
(F) Weiterhin wurde gefunden, daß man Verbindungen der Formel (I) 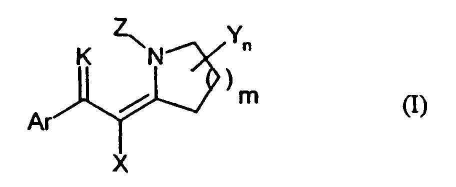 in welcher
   - Ar, X, Y, Z, m und n: die oben angegebene Bedeutung haben, und
   - K: für Schwefel steht,
   erhält, wenn man Verbindungen der Formel (I) 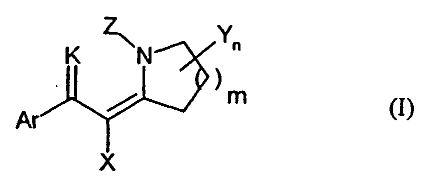 in welcher
   - Ar, X, Y, Z, m und n: die oben angegebene Bedeutung haben, und
   - K: für Sauerstoff steht,
   in Gegenwart eines Schwefelungsreagenzes wie beispielsweise Phosphorpentasulfid oder 2,4-Bis-(4-methoxyphenyl)-1,2,3,4-dithiaphosphetan-2,4-disulfid (Lawesson-Reagenz) in Gegenwart eines Lösungsmittels umsetzt.

Verwendet man gemäß Verfahren **A** z.B. 4-Methyl-benzoylacetonitril und 2-Methoxy-1-pyrrolin als Ausgangsstoffe, so kann der Reaktionsverlauf durch folgendes Reaktionsschema wiedergeben werden:

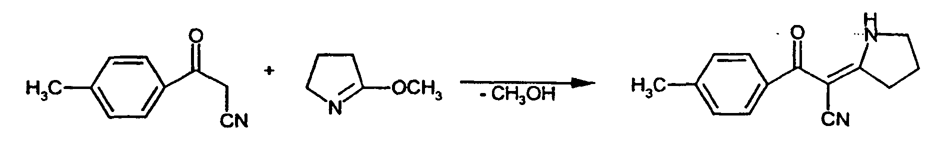

Verwendet man gemäß Verfahren **B** z.B. 2-Brom-2-(3-chlorbenzoyl)acetonitril und Pyrrolidin-2-thion als Ausgangsstoffe, so kann der Reaktionsverlauf durch folgendes Reaktionsschema wiedergeben werden:

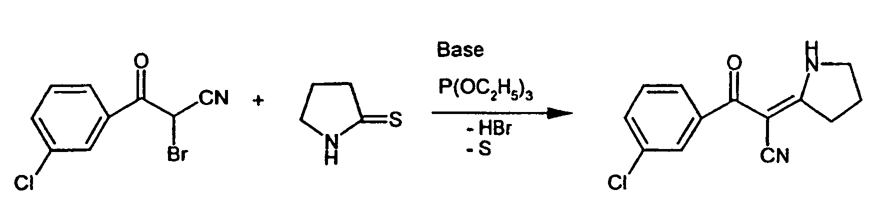

Verwendet man gemäß Verfahren **C** z.B. 3,4-Dichlor-benzoyl-acetonitril und N-Methyl-pyrrolidon als Ausgangsstoffe, so kann der Reaktionsverlauf durch folgendes Reaktionsschema wiedergeben werden:

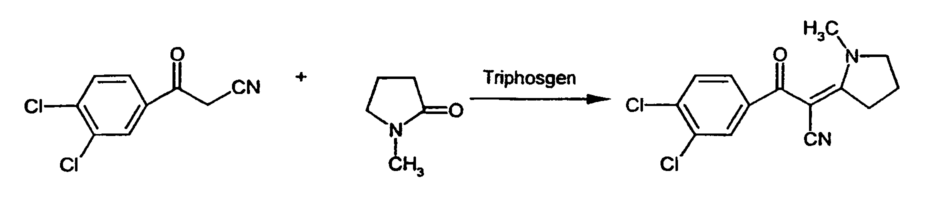

Verwendet man gemäß Verfahren **D** z.B. 3-Oxo-2-pyrrolidin-2-ylidene-3-(4-trifluormethoxy-phenyl)-propionitril und 2-Chlor-5-chlormethyl-pyridin als Ausgangsstoffe, so kann der Reaktionsverlauf durch folgendes Reaktionsschema wiedergeben werden:

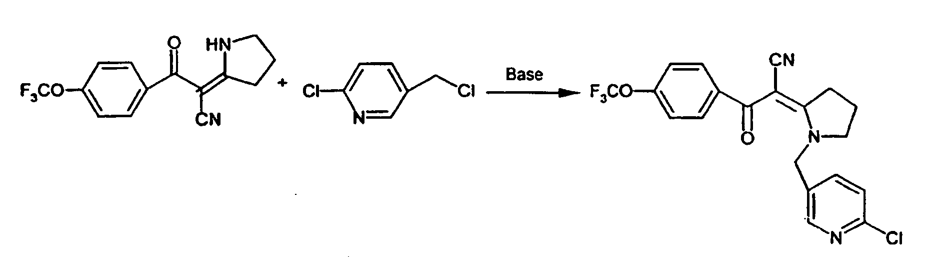

Verwendet man gemäß Verfahren **D** z.B. 3-Oxo-2-pyrrolidin-2-yliden-3-(4-trifluormethyl-phenyl)-propionitril und 4-Chlorbenzoylchlorid als Ausgangsstoffe, so kann der Reaktionsverlauf durch folgendes Reaktionsschema wiedergeben werden:

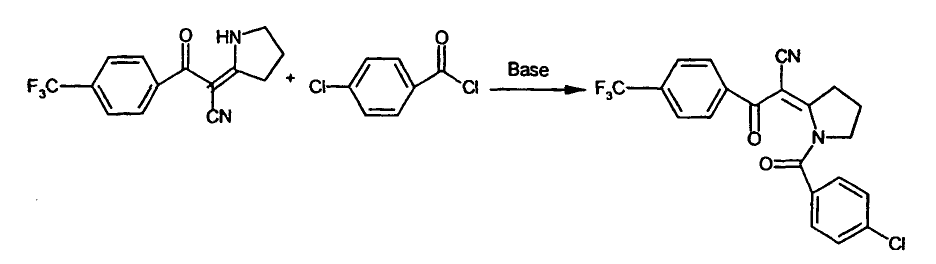

Verwendet man gemäß Verfahren **E** z.B. 3-Oxo-2-pyridin-2-yliden-3-(4-brom-phenyl)-propionitril und 4-Chlor-phenylboronsäure als Ausgangsstoffe, so kann der Reaktionsverlauf durch folgendes Reaktionsschema wiedergegeben werden:

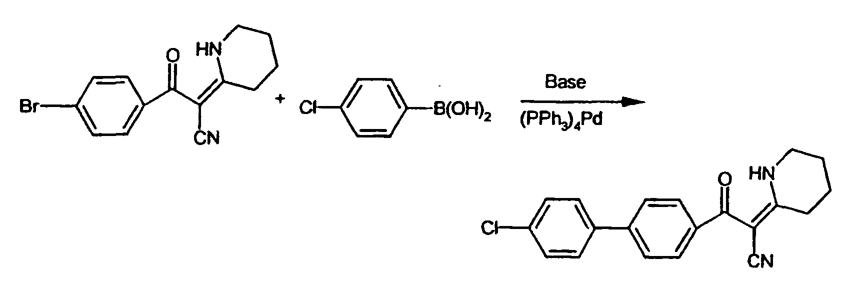

Verwendet man gemäß Verfahren **F** z.B. 3-Oxo-2-pyrrolidin-3-(4-chlor-phenyl)propionitril als Ausgangsstoff und das Lawesson-Reagenz, so kann der Reaktionsverlauf durch folgendes Reaktionsschema wiedergegeben werden:

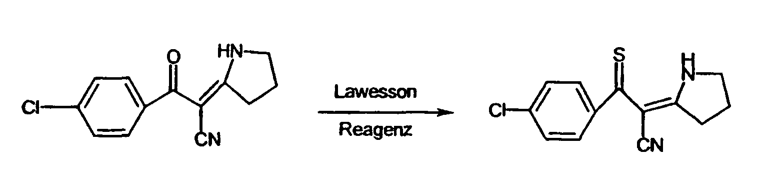

Die beim Verfahren (A) als Ausgangsstoffe benötigten Verbindungen der Formel (II), 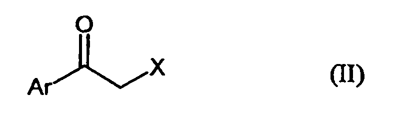 in welcher
- Ar, X: die oben angegebenen Bedeutungen haben,
sind teilweise neu und lassen sich nach im Prinzip literaturbekannten Verfahren herstellen (Organikum, 16. bearbeitete Auflage, S. 415, 417, VEB Deutscher Verlag der Wissenschaften, Berlin 1986).

Unter den Verbindungen der Formel (II) sind die neuen Verbindungen der Formel (II-1-b), 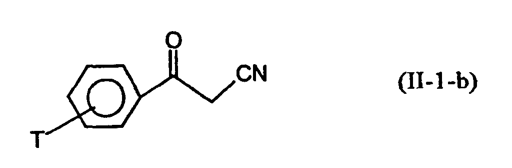 wobei
- T: die in der folgenden Tabelle angegebene Bedeutung hat:

| **Verb.-Nr.** | **T** | **Fp. °C** |
|---|---|---|
| II-1-b-1 | 3-Cl, 4-F | 78 |
| II-1-b-2 | 4-Cl, 3-F | 87 |
| II-1-b-3 | 3,5-(CF₃)₂ | 91 |
| II-1-b-4 | 2,4-Cl₂, 5-F | 106 |
| II-1-b-5 | 3,5-Cl₂, 4-F | 138-140 |
| II-1-b-6 | 4-Cl, 2-F | 90-94 |
| II-1-b-7 | 3-CF₃, 5-CH₃ | 92 |
| II-1-b-8 | 3-Cl, 4,5-F₂ | 84-88 |
| II-1-b-9 | 4-CN, 2,5-F₂ | 107-108 |
| II-1-b-10 | 2,3-F₂ | 74 |
| II-1-b-11 | 3-F, 4-CF₃ | 92 |
| II-1-b-12 | 3,4-O-CF₂-O | 70-73 |
| II-1-b-13 | 3-NO₂, 5-CF₃ | |
| II-1-b-14 | 4-Cl, 2,5-F₂ | 116 |
| II-1-b-15 | 3,4,5-(OC₂H₅)₃ | 122 |
| II-1-b-16 | 4-Br, 2-F | 118 |
| II-1-b-17 | 2,6-Cl₂, 4-CF₃ | 123 |
| II-1-b-18 | 2-F, 4-NO₂ | 168 |
| II-1-b-19 | 2,4-Cl₂, 5-NO₂ | 100 |
| II-1-b-20 | 4-Cl, 2-F, 5-NO₂ | 118 |
| II-1-b-21 | 2,4-F₂, 5-NO₂ | 128 |
| II-1-b-22 | 4-Br, 2-F, 5-NO₂ | 141 |
| II-1-b-23 | 2-F, 4-CF₃ | 62 |
| II-1-b-24 | 4-OCF₃, 3-NO₂ | 95 |
| II-1-b-25 | 4-Cl, 2-NO₂ | 130 |
| II-1-b-26 | 2-F, 3-CF₃ | 67-69 |
| II-1-b-27 | 2-Cl, 6-F | 46-48 |
| II-1-b-28 | 2-Cl, 3-CF₃ | 90-93 |
| II-1-b-29 | 3,4-O-(CF₂)₂-O- | 206 |
| II-1-b-30 | 2-Cl, 4-SCH₃ | 110 |
| II-1-b-31 | 2-Cl, 4-SO₂CH₃ | 202 |

und die Verbindung der Formel (II-2-b), 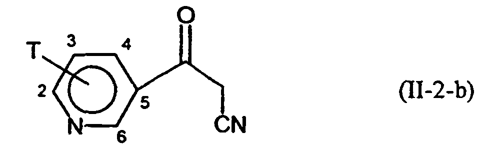 wobei T die in der folgenden Tabelle angegebene Bedeutung hat:

| **Verb.-Nr.** | **T** | **Fp. °C** |
|---|---|---|
| II-2-b-1 | 2,6-Cl₂ | > 220 |
| II-2-b-2 | 2,6-Cl₂, 4-CH₃ | 95 |
| II-2-b-3 | 6-Cl | 122 |

und die Verbindung der Formel (II-3-b), 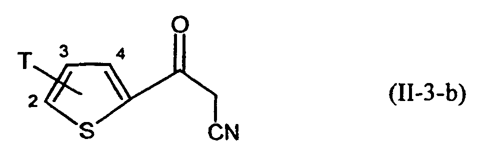 wobei T die in der folgenden Tabelle angegebene Bedeutung hat:

| **Verb.-Nr.** | **T** | **Fp. °C** |
|---|---|---|
| II-3-b-1 | 2-CF₃, 3-(4-Cl'-C₆H₄) | Öl |
| II-3-b-2 | 2-CF₃, 3-(2,4-Cl₂'-C₆H₃) | Öl |

und die Verbindung Nr. II-4-b-1 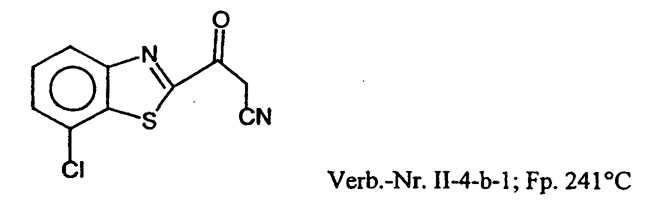 und die Verbindung Nr. II-5-b-1 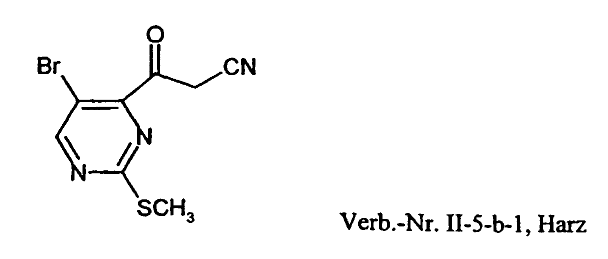 besonders gut zur Herstellung neuer pestizider, insbesondere akarizider, herbizider und insektizider, Endprodukte, geeignet.

Man erhält die Verbindungen der Formel (II) beispielsweise, indem man Verbindungen der Formel (XI), 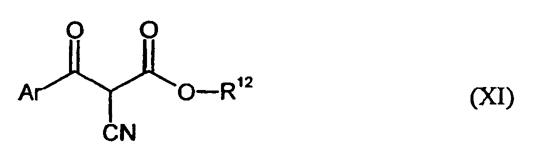 in welcher
- Ar: die oben angegebene Bedeutung hat,
- R¹²: für Alkyl, insbesondere für C₁-C₆-Alkyl, oder Benzyl steht, das gegebenenfalls substituiert sein kann,
in Gegenwart einer Säure (z.B. einer anorganischen Säure, wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 200°C, bevorzugt zwischen 20°C und 150°C hydrolysiert und dann decarboxyliert, wobei die Abspaltung des Restes R¹² wahlweise auch hydrolytisch nach bekannten Verfahren (Bowman, Fordham, J. Chem. Soc. 1951, 2758) mit molekularem Wasserstoff bei Drücken zwischen 1 und 100 bar gegebenenfalls in Gegenwart eines Lösungsmittels, wie beispielsweise Methanol, Ethanol oder Essigsäureethylester, bei Temperaturen zwischen -20 und 100°C, vorzugsweise bei Raumtemperatur, in Gegenwart eines Übergangsmetalls, wie beispielsweise Palladium, Nickel, Rhodium oder Platin, das gegebenenfalls auf einem Träger, wie beispielsweise Aktivkohle oder Bariumsulfat, immobilisiert ist, erfolgen kann.

Die Verbindungen der Formel (XI) lassen sich nach bekannten Verfahren herstellen (Organikum, 16. bearbeitete Auflage, S. 480, VEB Deutscher Verlag der Wissenschaften, Berlin 1986).

Man erhält die Verbindungen der Formel (XI) beispielsweise, indem man Verbindungen der Formel (XII), 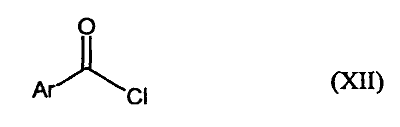 in welcher
- Ar: die oben angegebene Bedeutung hat,
mit Cyanessigsäureestern der Formel (XIII) 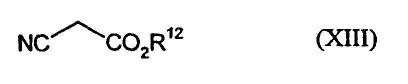 in welcher
R¹² für Alkyl, insbesondere für C₁-C₆-Alkyl, steht,
in Gegenwart einer Base (z.B. eines Metallalkoholats, wie Natriummethylat oder Natriumethylat) und gegebenenfalls in Gegenwart eines Verdünnungsmittels (z.B. Ether oder dem vom Alkoholat abgeleiteten Alkohol) bei Temperaturen von 0°C bis 150°C, bevorzugt zwischen 20°C und 120°C, umsetzt.

Die Verbindungen der Formel (XII) sind teilweise neu und lassen sich sich nach im Prinzip bekannten Verfahren herstellen (z.B. Organikum, 16. bearbeitete Auflage, S. 423, VEB Deutscher Verlag der Wissenschaften, Berlin 1986).

Man erhält die Verbindungen der Formel (XII) beispielsweise, indem man Verbindungen der Formel (XIV), 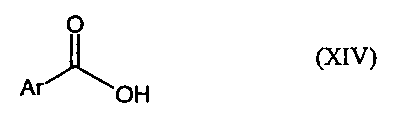 in welcher
- Ar: die oben angegebene Bedeutung hat,
mit Halogenierungsmitteln (z.B. Thionylchlorid, Phosgen, Phosphortrichlorid) gegebenenfalls in Gegenwart eines Verdünnungsmittels (z.B. gegebenenfalls chlorierten aliphatischen oder aromatischen Kohlenwasserstoffen, wie Toluol oder Methylenchlorid) bei Temperaturen von 0°C bis 150°C, bevorzugt zwischen 20°C und 100°C, umsetzt.

Cyanessigsäureester der Formel (XIII) sind bekannte Verbindungen der organischen Chemie.

Die beim Verfahren (A) ebenfalls als Ausgangsstoffe benötigten Verbindungen der Formel (III), 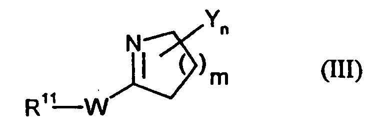 in welcher
- Y, R¹¹, m und n: die oben angegebenen Bedeutungen haben, und
- W: für Sauerstoff steht,
lassen sich nach bekannten Verfahren herstellen (Pendrak et al., J. Org. Chem. 60, (1995), 2912-2915).

Man erhält die Verbindungen der Formel (III) beispielsweise, indem man Verbindungen der Formel (VII), 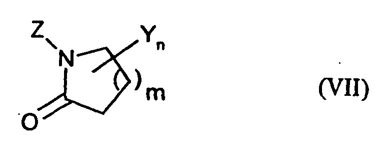 in welcher
- Y, m und n: die oben angegebenen Bedeutungen haben und
- Z: für Wasserstoff steht,
mit Alkylierungsmitteln (z.B. Dimethylsulfat, Triethyloxoniumtetrafluoroborat (Meerwein-Salz)) bei Temperaturen von -20°C bis 150°C, bevorzugt von 0°C bis 100°C, gegebenenfalls in der Gegenwart eines Verdünnungsmittels umsetzt.

Weiterhin erhält man die beim Verfahren A als Ausgangsstoffe benötigten Verbindungen der Formel (III) 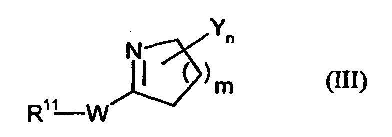 in welcher
- Y, R¹¹, m, n: die oben angegebenen Bedeutungen haben
und
- W: für Schwefel steht,
wenn man Verbindungen der Formel (VII) 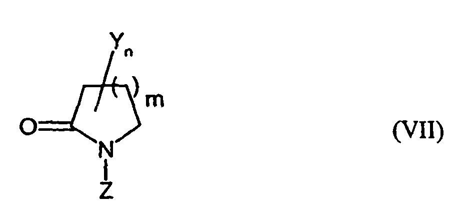 in welcher
- Y, m und n: die oben angegebenen Bedeutungen haben
und
- Z: für Wasserstoff steht
zunächst mit einem Schwefelungsreagenz, z.B. Lawesson-Reagenz, in das Thioamid der Formel (V), 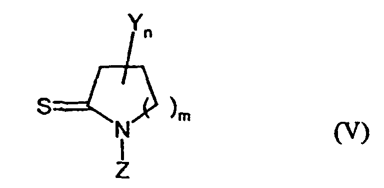 in welcher
- Y, m und n: die oben angegebenen Bedeutungen haben und Z für Wasserstoff steht,
in Gegenwart eines Lösungsmittels überftihrt und anschließend mit einem Alkylierungsmittel der Formel (XV),

R¹¹―Hal (XV)

in welcher
- R¹¹: die oben angegebene Bedeutung hat,
und
- Hal: für Halogen, insbesondere Iod und Brom, stehen,
gegebenenfalls in Gegenwart einer Base und in Gegenwart eines Lösungsmittels umsetzt.

Das Verfahren (A) ist dadurch gekennzeichnet, daß man Verbindungen der Formel (II),
in welcher
- Ar, X: die oben angegebenen Bedeutungen haben,
und Verbindungen der Formel (III),
in welcher
- R¹¹, W, Y, m, n: die oben angegebenen Bedeutungen haben,
in Gegenwart eines Verdünnungsmittels und gegebenenfalls in Gegenwart einer Base miteinander umsetzt.

Als Verdünnungsmittel können beim Verfahren (A) alle gegenüber den Reaktionsteilnehmern inerten organischen Solventien eingesetzt werden. Vorzugsweise verwendbar sind gegebenenfalls chlorierte aliphatische oder aromatischen Kohlenwasserstoffe wie Toluol, Xylol oder Methylenchlorid, weiterhin polare Solventien wie Dimethylsulfoxid, Dimethylformamid oder N-Methylpyrrolidon.

Als Basen können bei der Durchführung des Verfahrens (A) alle gegenüber den Reaktionsteilnehmern nicht hydrolysierend wirkende übliche Säureakzeptoren verwendet werden.

Vorzugsweise verwendbar sind tertiäre Amine wie Triethylamin, Pyridin oder N,N-Dimethylanilin.

Als Säuren können bei der Durchführung des Verfahrens (A) alle gegenüber den Reaktionsteilnehmern nicht hydrolysierend reagierenden Säuren eingesetzt werden. Vorzugsweise verwendbar sind organische Säuren wie p-Toluolsulfonsäure und Trifluoressigsäure.

Die Reaktionstemperatur kann bei der Durchführung des Verfahrens (A) innerhalb eines größeren Bereiches variiert werden. Zweckmäßigerweise arbeitet man bei Temperaturen zwischen -20°C und 160°C, vorzugsweise zwischen 0°C und 120°C.

Das Verfahren (A) wird vorzugsweise unter Normaldruck durchgeführt.

Bei Durchführung des Verfahrens (A) setzt man die Reaktionskomponente der Formel (III) in äquimolarer Menge oder in einem größeren Überschluß (bis zu 5 Mol) ein, vorzugsweise in 1,5- bis 2-molarer Menge im Verhältnis zu der Reaktionskomponente der Formel (II).

Die gegebenenfalls eingesetzte Base wird vorzugsweise in äquimolarer Menge zur Reaktionskomponente der Formel (II) verwendet. Die gegebenenfalls eingesetzte Säure wird vorzugsweise in katalytischen Mengen eingesetzt.

Das Verfahren (B) ist dadurch gekennzeichnet, daß man Verbindungen der Formel (IV) jeweils mit Thioamiden der Formel (V) gegebenenfalls in Gegenwart eines Verdünnungsmittels und gegebenenfalls in Gegenwart eine Säurebindemittels umsetzt.

Die Ausgangsverbindungen der Formel (IV) lassen sich nach bekannten Verfahren herstellen (Gakhar H.K. et al., J. Indian Chem. Soc. 43, (1971), 953 oder Corsaro A., Heterocycles 23, (1985), 2645). Die Verbindungen der Formel (V) sind durch Einsatz von Thionylierungsmitteln, insbesondere dem Lawesson-Reagenz, aus der entsprechenden Ketoverbindung in inerten Lösungsmitteln, wie z.B. Toluol, herstellbar (siehe Herstellungsbeispiel 9). Die substituierten 5-Phenyl-pyrrolidon-2-thione und 6-Phenyl-piperidin-2-thione der Formel (V) sind mit Ausnahme von 5-Phenyl-pyrrolidin-2-thion (siehe Lettau et al. PHARAT, Pharmazie 48 (1993) 410) und 5-(3,4-Dimethoxy-phenyl)-pyrrolidin-2-thion neu.

Als Verdünnungsmittel können bei dem erfindungsgemäßen Verfahren (B) alle gegenüber den Verbindungen der Formel (IV) inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, N-Methylpytrolidon, Dimethylsulfoxid und Sulfolan.

Als Säurebindemittel kommen bei der Umsetzung nach dem erfindungsgemäßen Verfahren (B) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethylanilin, polymere Basen wie Düsopropylaminopolystyrol, ferner Erdalkalimetalloxide, wie Magnesium- und Calciumoxid, außerdem Alkali- und Erdalkalimetall-carbonate, wie Natriumcarbonat, Kaliumcarbonat und Calciumcarbonat sowie Alkalihydroxide, wie Natriumhydroxid und Kaliumhydroxid.

Als Phosphorreagenzien können bei dem erfindungsgemäßen Verfahren (B) Alkylphosphite, wie Triethylphosphit, Tributylphosphit oder Triphenylphosphine, wie Triphenylphosphin verwendet werden.

Die Reaktionstemperatur kann beim erfindungsgemäßen Verfahren (B) innerhalb eines größeren Bereiches variiert werden. Im allgemeinen arbeitet man bei Temperaturen zwischen 0°C und 200°C, vorzugsweise zwischen +20°C und 150°C.

Bei der Durchführung des erfindungsgemäßen Verfahrens (B) werden die Ausgangsstoffe der Formel (IV) und Thioamid der Formel (V) im allgemeinen jeweils in angenähert äquivalenten Mengen verwendet. Es ist jedoch auch möglich, die eine oder andere Komponente in einem größeren Überschuß einzusetzen. Die Aufarbeitung erfolgt nach den üblichen Methoden.

Das Verfahren (C) ist dadurch gekennzeichnet, daß man Verbindungen der Formel (VII) mit Halogenierungsmittel, wie z.B. Phosgen, Diphosgen, Triphosgen, gegebenenfalls in Gegenwart eines Verdünnungsmittels zu Verbindungen der Formel (VIII) umsetzt, die dann mit Verbindungen der Formel (II) gegebenenfalls in Gegenwart eine Säurebindemittels und gegebenenfalls in Gegenwart eines Verdünnungsmittels umgesetzt werden. Die Ausgangsstoffe der Formel (VII) sind teilweise kommerziel erhältlich, wie z.B. N-Methyl-pyrrolidon oder lassen sich nach bekannten Verfahren herstellen.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethylanilin, polymere Basen wie beispielsweise Düsopropylaminopolystyrol, ferner Erdalkalimetalloxide, wie Magnesium- und Calciumoxid, außerdem Alkali- und Erdalkali-metallcarbonate, wie Natriumcarbonat, Kaliumcarbonat und Calciumcarbonat, sowie Alkalihydroxide, wie Natriumhydroxid und Kaliumhydroxid.

Als Verdünnungsmittel können bei dem erfindungsgemäßen Verfahren (C) alle gegenüber den Halogenierungsreagenzien inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylsulfoxid und Sulfolan.

Die Reaktionstemperatur kann beim erfindungsgemäßen Verfahren (C) innerhalb eines größeren Bereiches variiert werden. Im allgemeinen arbeitet man bei Temperaturen zwischen -20°C und 100°C, vorzugsweise zwischen 0°C und 80°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (VII) und das entsprechende Halogenierungsmittel im allgemeinen jeweils in angenähert äquivalenten Mengen verwendet. Es ist jedoch auch möglich, die eine oder andere Komponente in einem größeren Überschuß (bis zu 3 Mol) einzusetzen. Die Aufarbeitung erfolgt nach den üblichen Methoden. Im allgemeinen geht man so vor, daß man ausgefallene Salze entfernt und das verbleibende Reaktionsgemisch durch Abziehen des Verdünnungsmittels einengt.

Das Verfahren (D) ist dadurch gekennzeichnet, daß man Verbindungen der Formel (I) in welcher Ar, X, Y, m und n die oben angegebene Bedeutung haben und Z für Wasserstoff steht, jeweils mit Alkylierungsmittel, Acylierungsmittel, Sulfonierungsmittel oder Kondensationsmittel der Formel (IX) gegebenenfalls in Gegenwart eines Verdünnungsmittels und gegebenenfalls in Gegenwart eines Säurebindemittels umsetzt.

Die zu verwendenden Alkylierungsmittel, Acylierungsmittel, Sulfonierungsmittel oder Kondensationsmittel der Formel (IX) sind bekannte Synthesechemikalien der organischen Chemie.

Als Verdünnungsmittel können bei dem erfindungsgemäßen Verfahren (D) alle gegenüber den o.g. Reagenzien inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie beispielsweise Dimethylsulfoxid, Dimethylformamid, N-Methylpyrrolidon und Sulfolan. Wenn die Hydrolysestabilität der Acylierungsmittel und Sulfonylierungsmittel es zuläßt, kann die Umsetzung auch in Gegenwart von Wasser durchgeführt werden.

Als Säurebindemittel kommen bei der Umsetzung nach dem erfindungsgemäßen Verfahren (D) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethylanilin, ferner Erdalkalimetalloxide, wie Magnesium- und Calciumoxid, außerdem Alkali- und Erdalkali-metallcarbonate, wie Natriumcarbonat, Kaliumcarbonat und Calciumcarbonat sowie Alkalihydroxide, wie Natriumhydroxid und Kaliumhydroxid, weiterhin Alkalihydride wie Natriumhydrid, Kaliumhydrid oder Alkalialkoholate, wie Kalium-tert.-butylat.

Die Reaktionstemperaturen können bei dem erfindungsgemäßen Verfahren (D) innerhalb eines größeren Bereiches variiert werden. Im allgemeinen arbeitet man bei Temperaturen zwischen -70°C und +150°C, vorzugsweise zwischen -20°C und 100°C.

Bei der Durchführung des erfindungsgemäßen Verfahrens (D) werden die oben genannten Ausgangsstoffe der Formel (I) und die o.g. Reagenzien der Formel (IX) im allgemeinen jeweils in angenähert äquivalenten Mengen verwendet. Es ist jedoch auch möglich, die o.g. Reagenzien in einem größeren Überschuß (bis zu 5 Mol) einzusetzen. Die Aufarbeitung erfolgt nach den üblichen Methoden.

Zur Durchführung des erfindungsgemäßen Verfahrens (E) sind bevorzugt Palladium(0)-Komplexe als Katalysator geeignet. Bevorzugt wird beispielsweise Tetrakis-(triphenyl-phosphin)palladium.

Die für die Durchführung des Verfahrens (E) benötigten Arylboronsäuren sind teilweise kommerziell erhältlich, wie z.B. die 4-Chlor-phenylboronsäure, oder lassen sich nach bekannten Verfahren herstellen.

Als Säureakzeptoren zur Durchführung des erfindungsgemäßen Verfahrens (E) kommen anorganische oder organische Basen in Frage. Hierzu gehören vorzugsweise Erdalkalimetall- oder Alkalimetallhydroxide, -acetate, -carbonate oder -hydogencarbonate, wie beispielsweise Natrium-, Kalium-, Barium- oder Ammoniumhydroxid, Natrium-, Kalium-, Calcium- oder Ammoniumacetat, Natrium-. Kalium- oder Anunoniumcarbonat, Natriumhydrogen- oder Kaliumhydrogencarbonat, Alkalifluoride, wie beispielsweise Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicycloundecen (DBU), Diazabicyclononen (DBN).

Als Verdünnungsmittel zur Durchführung des erfindungsgemäßen Verfahrens (E) kommen Wasser, organische Lösungsmittel und beliebige Mischungen davon in Betracht. Beispielhaft seien genannt: aliphatische, alizyklische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether wie Diethyl-, Düsopropyl-, Methyl-tert.-butyl-, Methyl-tert.-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethan-diol, Propan-1,2-diol, Ethoxyethanol, Methoxyethanol, Diethylenglykolmono-methylether, Diethylenglykolmonoethylether; Wasser.

Die Reaktionstemperatur kann bei dem erfindungsgemäßen Verfahren (E) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (E) werden die Boronsäure der Formel (X), in welcher Ar^{2'} die oben angegebene Bedeutung hat und Verbindungen der Formel (I¹), in welcher Ar¹, X, Y, Z, m, n und Hal die oben angegebene Bedeutung haben, im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 bis 0,1 Mol pro Mol der Verbindung der Formel (I¹) ein. Die Base setzt man im allgemeinen in einem Überschuß ein.

Das Verfahren (F) ist dadurch gekennzeichnet, daß man Verbindungen der Formel (I) in welcher Ar, X, Y, Z, m und n die oben angegebene Bedeutung haben und K für Sauerstoff steht, mit Schwefelungsreagenzien gegebenenfalls in Gegenwart eines Verdünnungsmittels umsetzt.

Die zu verwendenden Schwefelungsreagenzien sind bekannte Synthesechemikalien wie beispielsweise Phosphorpentasulfid oder 2,4-Bis-(4-methoxyphenyl)-1,2,3,4-dithiaphosphetan-2,4-disulfid (Lawesson-Reagenz).

Als Verdünnungsmittel können bei dem erfindungsgemäßen Verfahren (F) alle gegenüber den o.g. Reagenzien inerten Solventien eingesetzt werden. Vorzugsweise verwendbar sind Kohlenwasserstoffe wie beispielsweise Benzin, Benzol, Toluol, Xylol und Tetralin, ferner Halogenkohlenwasserstoffe wie Methylenchlorid, Chloroform, Chlorbenzol und o-Dichlorbenzol, Ether wie Tetrahydrofuran, Dioxan, Diisopropylether oder Methyl-tert.-butylether.

Die Reaktionstemperaturen können bei dem erfindungsgemäßen Verfahren (F) innerhalb eines größeren Bereichs variiert werden. Im allgemeinen arbeitet man bei Temperaturen zwischen 0°C und 250°C, vorzugsweise zwischen 40°C und 200°C.

Bei der Durchführung des erfindungsgemäßen Verfahrens (F) werden die Ausgangsstoffe der Formel (I) und die o.g. Reagenzien im allgemeinen jeweils in angenähert äquivalenten Mengen verwendet. Es ist jedoch auch möglich, die o.g. Reagenzien in einem größeren Überschuß bis zu 5 Mol einzusetzen. Die Aufarbeitung erfolgt nach den üblichen Methoden.

Die erfindungsgemäßen Wirkstoffe eignen sich besonders gut als Defoliants, Desiccants, Krautabtötungsmittel und insbesondere als Unkrautvemichtungsmittel. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Verwendung der erfindungsgemäßen Wirkstoffe ist jedoch keineswegs auf diese Gattungen beschränkt, sondern erstreckt sich in gleicher Weise auch auf andere Pflanzen. Bei der Verwendung der erfindungsgemäßen Wirkstoffe sei die Verwendung im Zusarnmenhang mit transgenen Pflanzen besonders hervorgehoben, da in diesem Falle synergistische Wirkungsverstärkungen beobachtet werden können.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 % und daneben bevorzugt Streckmittel und/oder oberflächenaktive Mittel.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a. Auch Safener können zur Erhöhung der Kulturpflanzenverträglichkeit in bevorzugten Ausführungsformen der vorliegenden Erfindung mit den erfindungsgemäßen Verbindungen gemischt werden.

### Besonders günstige Mischpartner sind z.B. die folgenden:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Flüquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-propyl-1H-1,2,4-triazol-1-ethanol,
α-(2,4-Dichlorphenyl)-β-methoxy-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Meihomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin. Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

### Herbizide:

Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clefoxydim, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop-(-P-ethyl), Fentrazamide, Flamprop(-isopropyl), Flamprop(-isopropyl-L), Flamprop(-methyl), Flazasulfuron, Florasulam, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop-(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfiuon(-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromwon, (alpha-)Metolachlor, Metosulam, Metoxuron, Metribuzin, Metsulfuron(-methyl), Molinate, Monolinwon, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop-(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die Wirkstoffe eignen sich weiterhin zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
   Aus der Ordnung der Diplopoda z.B. Blaniulus guttulatus.
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
Aus der Ordnung der Blattaria bzw. Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
   Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
   Aus der Ordnung der Mallophaga z.B. Trichodectes spp., Damalinea spp..
   Aus der Ordnung der Thysanoptera z.B. Frankliniella occidentalis, Hercinothrips femoralis, Thrips palmi, Thrips tabaci.
   Aus der Ordnung der Heteroptera z.B. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Phylloxera vasturix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Plutella xylostella, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Aulema oryzae.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Liriomyza spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemnia spp., Liviomyza spp..
Aus der Ordnung der Siphonaptera z.B. Xenopsylla cheopis, Ceratophyllus spp..
   Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipulpus spp..

Zu den pflanzenparasitären Nematoden gehören z.B. Pratylenchus spp; Radopholus similis, Ditylenchus dipsaci, Tylenchulus semi penetrans, Heteroderma spp., Globodera spp., Meloidogyne spp., Aphelenchoides spp., Longidorus spp., Xiphinema spp., Trichodorus spp., Bursaphelenchus spp..

Die erfindungsgemäßen Wirkstoffe zeichnen sich durch eine hohe insektizide und akarizide Wirksamkeit nach Blatt- und Bodenanwendung aus.

Erfindungsgemäße Verbindungen haben in bestimmten Konzentrationen bzw. Aufwandmengen auch eine fungizide Wirkung. Sie können ferner auch als Mikrobizide oder Antimykotika verwendet werden.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp.. Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phtebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie
   Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeittingsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- -bzwf emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel-(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppel vakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypennethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octyl-isothiazolin-3-on genannt.

Die erfindungsgemäßen Wirkstoffe lassen sich besonders gut zur Bekämpfung von pflanzenschädigenden Insekten einsetzen, wie beispielsweise gegen die Larven des Meerrettichblattkäfers (Phaedon cochleariae), gegen die Larven der grünen Reiszikade (Nephotettix cincticeps) und gegen die Larven der grünen Pfirsichblattlaus (Myzus persicae).

Neben den beschriebenen akariziden, herbiziden und insektiziden Eigenschaften ist zu dem eine fungizide Wirksamkeit der erfindungsgemäßen Wirkstoffe feststellbar. Sowohl bei "in vitro-" als auch "in vivo"-Studien ist eine breite pilzabtötende Wirkung beobachtbar.

Außerdem wurde festgestellt, daß die Wirkstoffe sich besonders auch zur Bekämpfung von Mehltau, Blattflecken und Fusarien bei den damit befallenen Pflanzen eignen.

Die Herstellung und die Verwendung der erfindungsgemäßen Wirkstoffe geht aus den nachfolgenden Beispielen hervor.

### Herstellungsbeispiele:

### Beispiel 1:

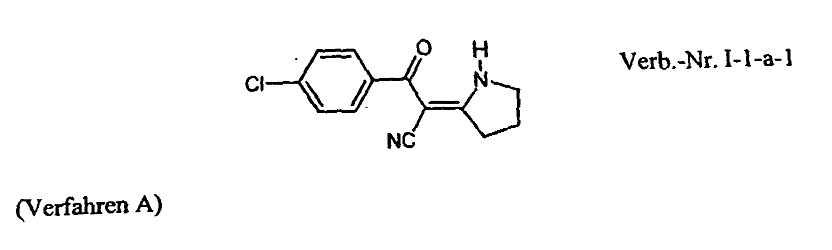

Eine Mischung von 1,2 g 2-Ethoxy-pyrrolin und 1 g 4-Chlorbenzoylacetonitril in 20 ml Toluol wird 1 Stunde bei 100°C gerührt. Der entstehende Ethylalkohol wird mit Toluol azeotrop abdestilliert. Nach Abkühlung auf 20°C wird das Reaktionsprodukt durch Zugabe von Hexan zur Kristallisation gebracht und abgesaugt.

Man erhält 1,1 g (Ausbeute: 74 % der Theorie) an 3-(4-Chlor-phenyl)-3-oxo-2-pyrrolidin-2-yliden-propionitril mit einem Schmelzpunkt von 156-158°C.

### Beispiel 2:

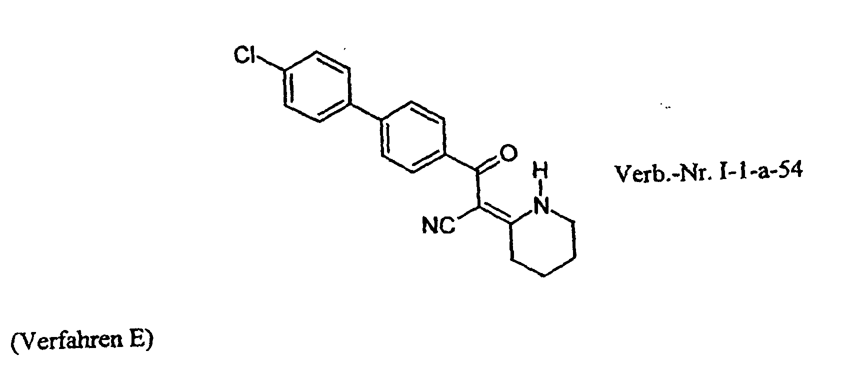

Eine bei 20°C hergestellte Lösung von 1,85 g 3-Oxo-3-(4-brom-phenyl)-2-piperidin-2-yliden-propionitril in 20 ml Dimethoxyethan wird unter Argon als Schutzgas mit 1 g 4-Chlorphenylboronsäure und 360 mg Tetrakis(triphenylphosphin)-Palladium versetzt und 15 Minuten bei 20°C gerührt. Dann werden 10 ml 20 %ige Na₂CO₃-Lösung zugegeben und die Mischung 24 h auf 80°C erwärmt. Anschließend wird die Mischung mit konz. Salzsäure angesäuert, mit Wasser verdünnt, mit Essigsäureethylester geschüttelt, die organische Phase abgetrennt, mit Wasser gewaschen, getrocknet und filtriert. Vom Filtrat wird das Lösungsmittel abdestilliert.

Man erhält 1,9 g (Ausbeute 95 % der Theorie) an 3-(4'-Chlor-biphenyl-4-yl)-3-oxo-2-piperidin-2-yliden-propionitril mit einem Schmelzpunkt von 134°C.

### Beispiel 3:

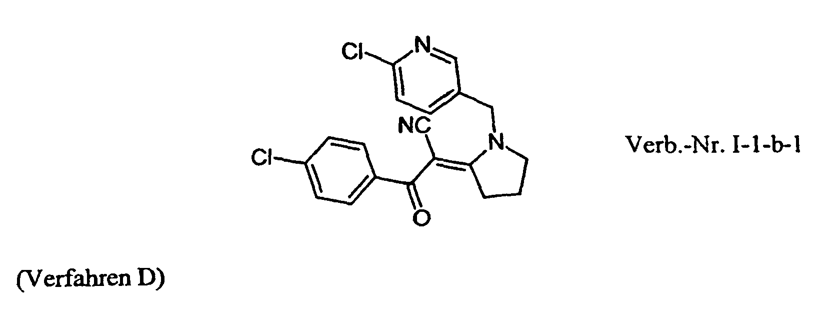

1 g des gemäß Beispiel 1 hergestellten 3-(4-Chlor-phenyl)-3-oxo-2-pyrrolidin-2-yliden-propionitril werden bei 0°C in 10 ml THF gelöst und mit 0,16 g NaH (60 %ig) versetzt und vier Stunden auf 20°C erwärmt. Dann werden 0,65 g 2-Chlor-5-chlormethyl-pyridin zugegeben und die Mischung 4 Stunden unter Rückfluß zum Sieden gebracht. Anschließend werden 100 mg Diazabicyclooctan (DABCO) zugegeben und das Gemisch weitere 24 Stunden unter Rückfluß erhitzt. Dann wird das Reaktionsgemisch auf Eiswasser gegossen, die festen Rückstände abgesaugt, die flüssige Phase mit Essigsäureethylester geschüttelt, dann die organische Phase abgetrennt, mit Wasser gewaschen und filtriert. Die in der Lösung enthaltenen Verbindungen wurden an einer Kieselgelphase mit Hexan/Aceton 7:3 als Laufmittel säulenchromatographisch getrennt.

In einer der Hauptfraktionen erhielt man nach Abdampfen des Lösungsmittels 0,65 g (Ausbeute 44 % der Theorie) des 3-(4-Chlor-phenyl)-2-[1-(6-chlor-pyridin-3-yl-methyl)-pyrrolidin-2-yliden]-3-oxo-propionitril mit einem Schmelzpunkt von 142-144°C.

### Beispiel 4:

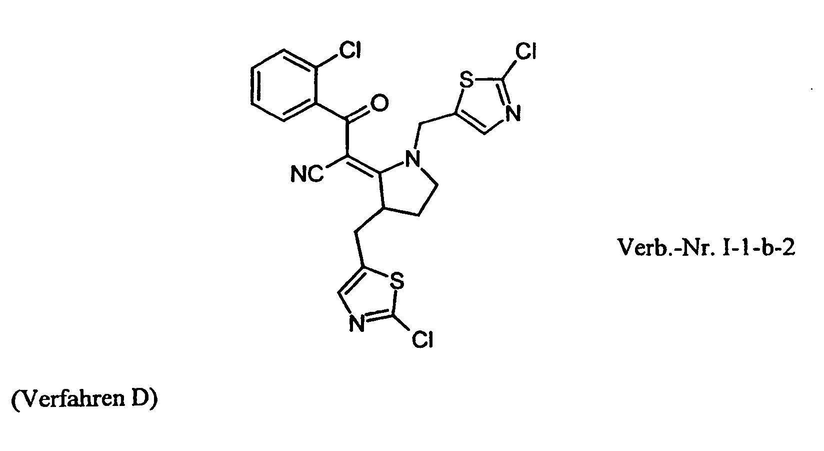

1,5 g 3-(2-Chlor-phenyl)-3-oxo-2-pyrrolidin-2-yliden-propionitril werden bei 0°C in 10 ml THF gelöst und mit 0,5 g NaH (60 %ig) versetzt und vier Stunden auf 20°C erwärmt. Dann werden 1,1 g 2-Chlor-5-chlormethyl-thiazol in 10 ml THF und 100 mg Diazabicyclooctan (DABCO) zugegeben und das Gemisch sorgfältig auf Raumtemperatur (20°C) gehalten und der Reaktionsverlauf dünnschichtchromatographisch verfolgt. Dann wird das Reaktionsgemisch auf Eiswasser gegossen, die festen Rückstände abgesaugt, die flüssige Phase mit Essigsäureethylester geschüttelt, dann die organische Phase abgetrennt, mit Wasser gewaschen und filtriert. Die in der Lösung enthaltenen Verbindungen wurden mit n-Hexan/Aceton 7:3 als Laufmittel säulenchromatographisch an einer Kieselgelphase getrennt.

In einer der Hauptfraktionen erhielt man nach Abdampfen des Lösungsmittels 0,4 g (Ausbeute 13 % der Theorie) des 3-(2-Chlorphenyl)-2-[3-(2-chlor-thiazol-5-yl)-1-(2-chlor-thiazol-5-yl-methyl)pyrrolidin-2-yliden]-3-oxo-propionitril.

### Beispiel 5:

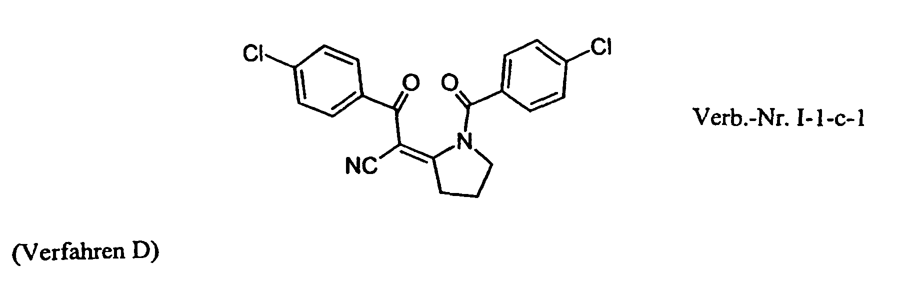

1 g des gemäß Beispiel 1 hergestellten 3-(4-Chlor-phenyl)-3-oxo-2-pyrrolidin-2-yliden-propionitril werden bei 0°C in 10 ml THF gelöst und mit 0,3 g NaH (60 %ig) versetzt und zwei Stunden auf 20°C erwärmt. Dann werden 0,8 g Chlorbenzoylchlorid in 10 ml THF zugegeben und die Mischung weitere 24 Stunden bei Raumtemperatur gerührt. Dann wird das Reaktionsgemisch auf Eiswasser gegossen, mit Essigsäureethylester extrahiert, über Magnesiumsulfat getrocknet und das Lösungsmittel im Vakuum abgedampft. Der Rückstand wurde an Kieselgel mit n-Hexan/Aceton 7:3 chromatographiert. In einer der Hauptfraktionen erhielt man nach Abdampfen des Lösungsmittels 0,2 g (Ausbeute 14 % der Theorie) des 2-[1-(4-chlorbenzoyl)pyrrolidin-2-yliden]-3-(4-chlorphenyl)-3-oxo-propionitril mit einem Schmelzpunkt von 218°C.

### Beispiel 6:

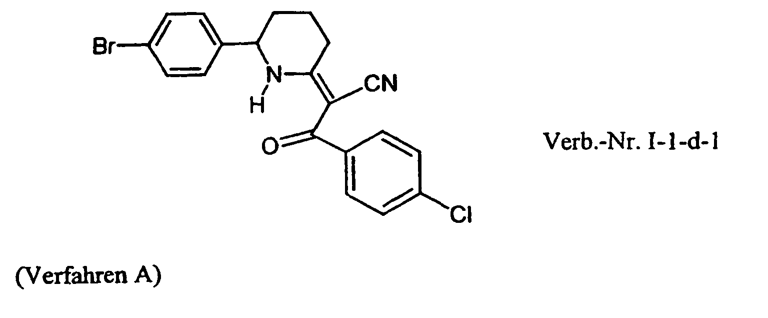

Eine Mischung von 0,9 g 6-(4-Brom-phenyl)-2-ethoxy-tetrahydropyridin und 0,5 g Chlorbenzeylacetonitril in 10 ml Toluol wird auf 120°C erhitzt und zwei Stunden bei dieser Temperatur gehalten. Die in der Lösung enthaltenen Reaktionsprodukte werden säulenchromatographisch mit Methylenchlorid/Essigester 5:3 als Laufmittel an einer Kieselgelphase getrennt.

In der Hauptfraktion erhielt man nach Abdampfen des Lösungsmittels 0,9 g (Ausbeute 77,6 % der Theorie) des 2-[6-(4-Brom-phenyl)-piperidin-2-yliden]-3-(4-chlorphenyl)-3-oxo-propionitril.

### Beispiel 7:

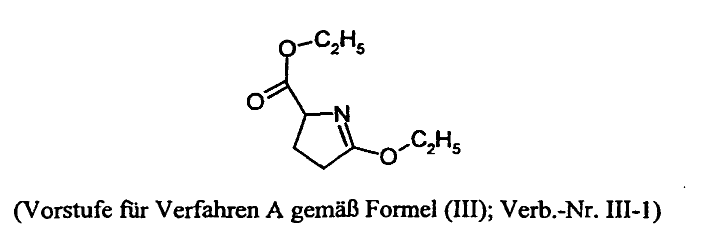

63 g DL-Pyroglutaminsäureethylester in 200 ml Methylenchlorid werden über 10 Minuten zu einer Lösung von 500 ml Triethyloxonium-tetrafluorboronat (Meerweinsalz, als einmolare Lösung in Methylenchlorid) zugetropft und die Mischung 24 Stunden unter Rückluß zum Sieden erhitzt. Dann wird die Lösung auf 0°C abgekühlt und vorsichtig mit 63 g Natriumcarbonat in 300 ml Wasser versetzt. Das Reaktionsprodukt wird mit Methylenchlorid extrahiert, die organische Phase getrocknet und filtriert und anschließend das Lösungsmittel im Wasserstrahlvakuum abgezogen.

Man erhält 12,5 g (Ausbeute 12,9 % der Theorie) an 5-Ethoxy-3,4-dihydro-2H-pyrrol-2-carbonsäureethylester.

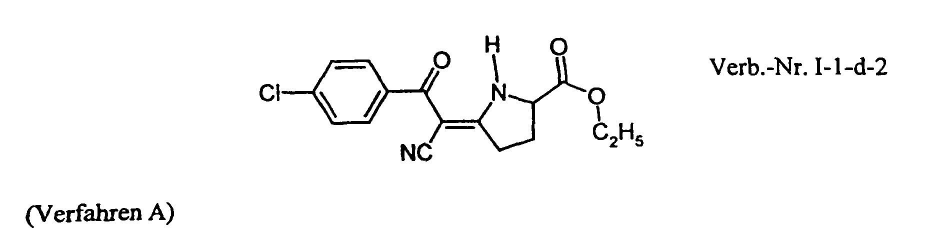

Eine Mischung von 6,1 g des hergestellten 5-Ethoxy-3,4-dihydro-2H-pyrrol-2-carbonsäureethylester und 6 g Chlorbenzoylacetonitril in 50 ml Toluol wird 1 Stunde bei 100°C gerührt. Anschließend wird der entstandene Ethylalkohol zusammen mit dem Toluol als Azeotrop abdestilliert. Nach Abkühlung auf 20°C wird das Reaktionsprodukt durch Hexanzugabe zur Kristallisation gebracht und abgesaugt. Die Abtrennung des Hauptproduktes erfolgt säulenchromatographisch an einer Kieselgelphase mit n-Hexan/Aceton 7:3.

In der Hauptfraktion erhielt man nach Abdampfen des Lösungsmittels 6,9 g (Ausbeute 67 % der Theorie) des 5-[2-(4-Chlor-phenyl)-1-cyano-2-oxo-ethyliden]-pyrrolidin-2-carbonsäureethylester mit einem Schmelzpunkt von 108-110°C.

### Beispiel 8:

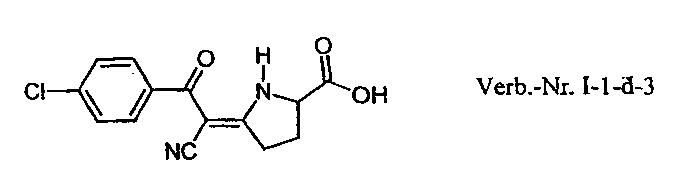

4,8 g des gemäß Beispiel 7 hergestellten 5-[2-(4-Chlor-phenyl)-1-cyano-2-oxo-ethyliden]-pyrrolidin-2-carbonsäureethylester werden in bei 40°C in 30 ml Ethanol gelöst und mit 10 ml 20 %iger NaOH versetzt. Nach einer Stunde Rühren bei der angegebenen Temperatur wird die Mischung auf unter Wasserstrahlvakuum eingeengt und mit HCl auf pH 1-2 eingestellt.

Als Rückstand erhält man 2,5 g des 5-[2-(4-Chlor-phenyl)-1-cyano-2-oxo-ethyliden]-pyrrolidin-2-carbonsäure mit einem Schmelzpunkt von 134 - 136°C.

### Beispiel 9:

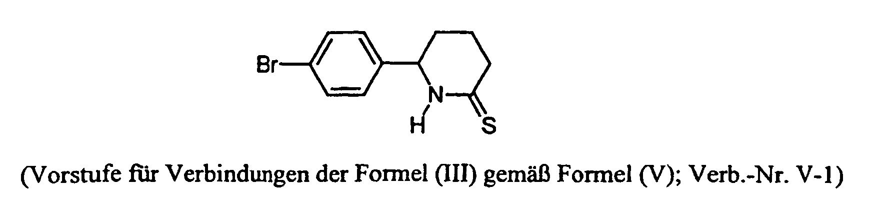

38,5 g 6-(4-Brom-phenyl)-piperidin-2-on und 31g [2,4-Bis(4-methoxyphenyl)-1,3,2,4-dithiadiphosphetan-2,4-disulfid] (Lawesson-Reagenz) werden in 200 ml wasserfreiem Toluol ca. 2 Stunden zum Rückluß erhitzt. Der Reaktionsverlauf wird dünnschichtchromatographisch überwacht. Die in der Lösung enthaltenen Reaktionsprodukte werden säulenchromatographisch mit Methylenchlorid/Essigester 5:3 als Laufmittel an einer Kieselgelphase getrennt.

In der Hauptfraktion erhielt man nach Abdampfen des Lösungsmittels 20 g (Ausbeute 50 % der Theorie) des 6-(4-Brom-phenyl)-piperidin-2-thion mit einem Schmelzpunkt von 157°C.

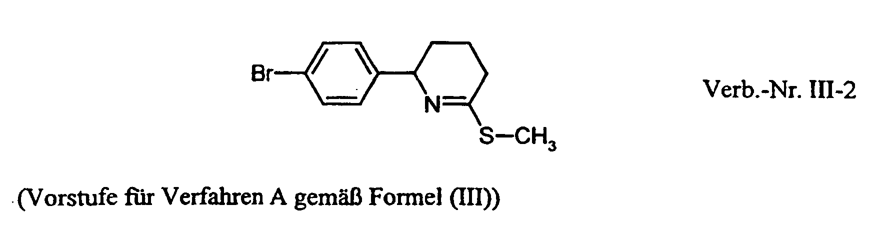

8 g 6-(4-Brom-phenyl)-piperidin-2-thion werden bei 20 °C in 150 ml Aceton gelöst und nacheinander mit 10 g Kaliumcarbonat und 10 g Methyljodid versetzt. Über ca. 24 Stunden wird der Reaktionsverlauf dünnschichtchromatographisch überwacht während das Gemisch zum Rückfluß erhitzt wird. Nach Erkalten wird der Feststoff abgesaugt und das Filtrat eingeengt. Die in der Lösung enthaltenen Reaktionsprodukte werden säulenchromatographisch mit Methylenchlorid/Essigester 5:3 als Laufmittel an einer Kieselgelphase getrennt.

In der Hauptfraktion erhielt man nach Abdampfen des Lösungsmittels 2,1 g (Ausbeute 25 % der Theorie) des 6-(4-Brom-phenyl)-2-methylmercapto-2,3,4,5-tetrahydro-pyridin als gelbes Öl.

### Beispiel 10:

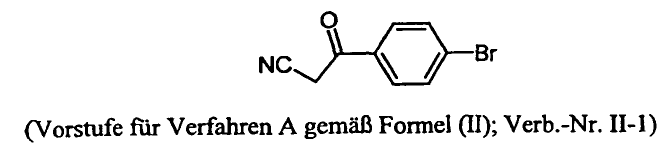

80 g 4-Brombenzoesäureethylester werden in 2000 ml Dioxan und 50 g Acetonitril vorgelegt. Nach Zusatz von 3 Tropfen Tris-[2-(2-methoxyethoxy)-ethyl]-amin (TDA-1) wird auf 90°C erwärmt. Es werden anschließend 70 g Kalium-tert.-butylat portionsweise zugegeben und das Gemisch drei Stunden zum Rückfluß erhitzt. Die Mischung wird dann unter Wasserstrahlvakuum eingeengt, der Rückstand mit Wasser aufgenommen, mit 10 %iger HCl angesäuert, mit Methylenchlorid versetzt, filtriert, die Methylenchloridphase mit gesättigter Natriumcarbonatlösung ausgeschüttelt und die getrocknete, organische Phase bis zur Trockne eingeengt.

Als Rückstand erhält man 82 g (Ausbeute 72 % der Theorie) von 3-(4-Brom-phenyl)-3-oxopropionitril.

### Beispiel 11:

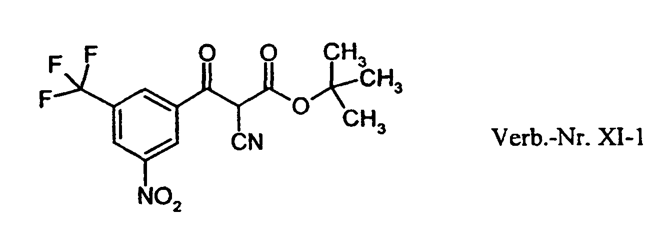

5,84 g NaH (60 %ig) werden in 147 ml Toluol unter Schutzgasbegasung (Argon) vorgelegt. Anschließend werden 10,51 g Cyanessigsäure-tert.-butylester (98 %ig) in 9 ml Toluol bei 20°C zugetropft, und die Mischung für 45 Minuten bei Raumtemperatur gerührt. Dann werden 18,5 g 3-Nitro-5-trifluormethyl-benzoylchlorid in 9 ml Toluol zugetropft und die Mischung 24 Stunden weiter gerührt. Danach werden 168 ml Wasser vorsichtig zugegeben, die Toluolphase abgetrennt und mit ca. 190 ml Wasser gewaschen. Die vereinigten wäßrigen Phasen werden mit Methylenchlorid gewaschen, mit 2 N HCl angesäuert und dann erneut zweimal mit Methylenchlorid extrahiert. Die vereinigten organischen Phasen werden mit Wasser gewaschen, über Na₂SO₄ getrocknet und im Wasserstrahlvakuum eingeengt.

Als Rückstand erhält man 13,1 g (Ausbeute 53 % der Theorie) an 2-Cyano-3-(3-nitro-5-trifluormethyl-phenyl)-3-oxo-propionsäure-tert.-butylester.

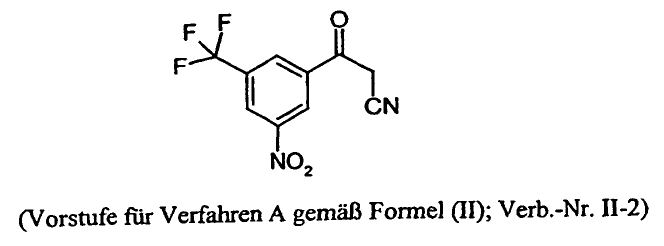

13 g von 2-Cyano-3-(3-nitro-5-trifluormethyl-phenyl)-3-oxo-propionsäure-tert.-butylester werden dann in 55 ml Toluol vorgelegt und bei 20 °C mit 0,07 g p-Toluolsulfonsäure(monohydrat) versetzt. Die Mischung wird zwei Stunden unter Rückfluß erhitzt, und der Reaktionsverlauf dünnschichtchromatographisch überwacht. Die Lösung wird anschließend eingeengt, und der Rückstand nach Aufnahme in Methylenchlorid/Methanol (99:1) über Kieselgel filtriert und dann das Lösungsmittel abgezogen.

Man erhält 5,5 g (Ausbeute 59 % der Theorie) an 3-(3-Nitro-5-trifluor-phenyl)-3-oxo-propionitril.

### Beispiel 12:

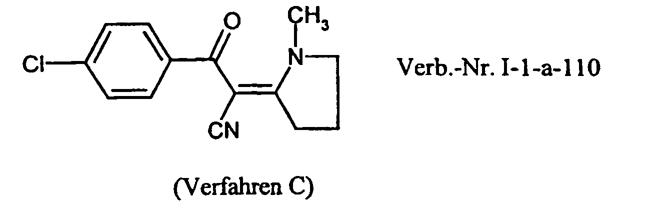

3,06 g (20mmol) 1-Methyl-2-chlor-2-pyrrolin-iminiumchlorid werden in 10 ml Toluol suspendiert. Unter Schutzgas werden 1,79 g (19 mmol) 4-Chlorbenzoylacetonitril (als Lösung in 30 ml Chloroform) und dann 3,06 ml (22 mmol) Triethylamin (als Lösung in 30 ml Chloroform) jeweils bei 0°C zugetropft. Dabei wird mit starker Kühlung gearbeitet. Nach einer Stunde Rühren bei 0°C und 2 Stunden Rühren bei Raumtemperatur werden weitere 3,06 g 1-Methyl-2-chlor-2-pyrrolin-iminiumchlorid und 4,18 ml Triethylamin bei 0°C zugegeben. Es wird 48 Stunden bei Raumtemperatur nachgerührt. Anschließend wird das Reaktionsgemisch auf 30 ml Eiswassser gegossen und dreimal mit je 50 ml Chloroform extrahiert. Die Chloroformphase wird über Magnesiumsulfat getrocknet und dann zur Trockne eingeengt.

Das erhaltene Reaktionsprodukt wird säulenchromatographisch mit Hexan/Essigester 1:1 als Laufmittel an einer Kieselgelphase getrennt.

In der Hauptfraktion erhielt man nach Abdampfen des Lösungsmittels 620 mg (Ausbeute 24 % der Theorie) des 3-(4-Chlor-phenyl)-3-oxo-2-(1-methyl-pyrrolidin-2-yliden)-propionitril mit einem Schmelzpunkt von 115°C.

### Beispiel 13:

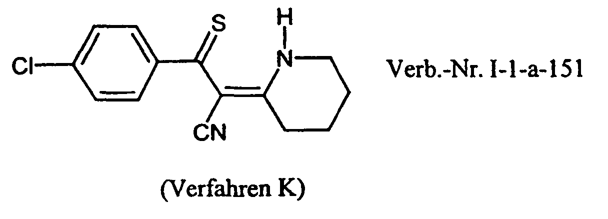

1 g der Verbindung aus Beispiel I-1-a-55 werden in 10 ml Toluol gelöst und mit 1,58 g (3,9 mmol) Lawesson-Reagenz versetzt und zum Rückfluß erhitzt. Nach 1 h kann durch Dünnschichtchromatographie mit Hexan/Essigester als Laufmittel kein Edukt mehr nachgewiesen werden. Es wurde dann das Lösungsmittel zur Hälfte abgezogen und die ausgefallenen Kristalle abgesaugt. Das nach weiterer Einengung ausgefallene Produkt wird säulenchromatographisch mit Hexan/Essigester als Laufmittel an einer Kieselgelphase getrennt.

In der Hauptfraktion erhielt man nach Abdampfen des Lösungsmittels 0,560 g (Ausbeute 53 % der Theorie) des 3-(4-Chlor-phenyl)-3-thio-2-piperidin-2-yliden-propionitril mit einem Schmelzpunkt von 137°C.

### Beispiel 14

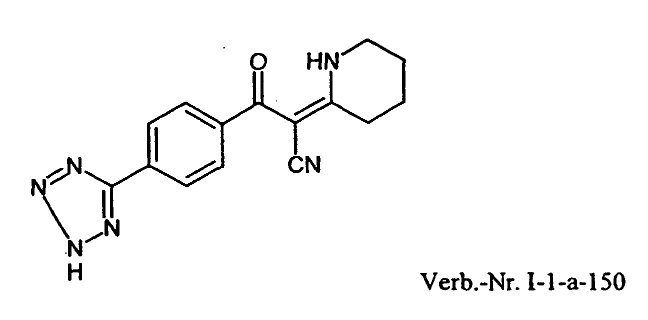

257 g (1,02 mmol) der Verbindung aus Beispiel Nr. I-1-a-62 werden zusammen mit 246 mg (1,2 mmol) Trimethylzinnazid für 24 Stunden in Toluol unter Rückfluß erhitzt. Nach 24 Stunden werden weitere 120 mg Zinnazid zugesetzt und nochmals 5 Stunden unter Rückfluß erhitzt. Anschließend wird das Zinnaddukt abgesaugt. Nach Versetzen mit 5 ml einer 2-N-NaOH-Lösung scheidet sich das Edukt ab und wird abgesaugt. Nach Einstellung des pH-Wertes auf pH = 1 mit 2-N-HCl wird das hergestellte Produkt aus der auf 0°C abgekühlten Mischung abgesaugt.

Man erhielt so als Feststoff 0,06 g (Ausbeute 18 % der Theorie) an 3-(4-Tetrazolylphenyl)-3-oxo-2-piperidin-2-yliden-propionitril mit einem Schmelzpunkt von größer 220°C.

### Beispiel 15

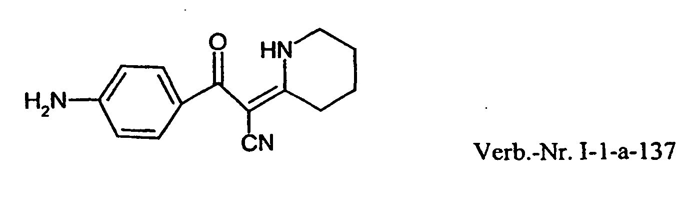

2,33 g (8,6 mmol) der Verbindung aus Beispiel Nr. I-1-a-63 werden in 100 ml Ethanol (p.a.) gelöst, und zu der Lösung werden 300 mg Raney®-Nickel gegeben. In die Reaktionsmischung wird für 5 Minuten Stickstoff eingeleitet und danach für 8 Stunden Wasserstoff bei Normaldruck eingeleitet. Anschließend wird erneut für 5 Minuten mit Stickstoff gespült. Das Raney®-Nickel wird abfiltriert, und es wird mit 20 ml Ethanol und 50 ml Dichlormethan nachgespült. Die vereinigten Filtrierlösungen werden zur Trockne eingeengt und der Rückstand am Hochvakuum getrocknet.

Man erhielt so als Feststoff 2,03 g (Ausbeute 97,8 % der Theorie) an 3-(4-Nitrophenyl)-3-oxo-2-piperidin-2-yliden-propionitril mit einem Schmelzpunkt von 182°C.

### Beispiel 16

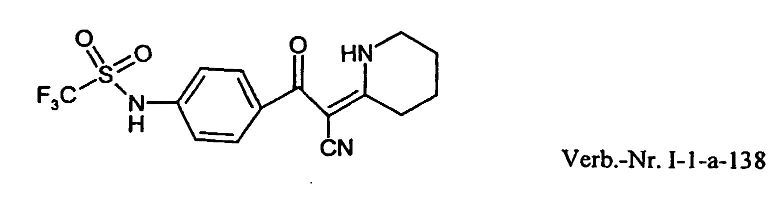

0,4 g (1,66 mmol) der Verbindung aus Beispiel Nr. I-1-a-137 werden unter Schutzgas in ausgeheizte Apparaturen in 5 ml Dichlormethan vorgelegt und auf -20°C abgekühlt. Bei dieser Temperatur werden 0,28 ml (2,0 mmol) Triethylamin zugesetzt und anschließend 0,33 ml (1,98 mmol) Trifluormethansulfonsäureanhydrid zugetropft. Es wird für 15 Minuen bei -20°C nachgerührt. Nach Aufwärmung auf 0°C wird weitere 2 Stunden gerührt. Zur Vervollständigung der Reaktion werden bei -20°C weitere 0,33 mmol Trifluormethansulfonsäureanhydrid zugesetzt. Nach Aufwärmung auf 0°C wird nochmals für 1 Stunde nachgerührt.

Die Reaktionsmischung wird mit 50 ml Dichlormethan verdünnt und auf 20 ml 0°C kalte 1-N-HCl gegossen. Die organische Phase wird abgetrennt, über Magnesiumsulfat getrocknet und am Rotationsverdampfer eingeengt. Die Reaktionsprodukte werden säulenchromatographisch mit Essigester/Cyclohexan 1:1 als Laufmittel an einer Kieselgelphase getrennt.

Man erhält 316 mg (Ausbeute 51 % der Theorie) des 3-[4-(Trifluormethansulfonylamino)-phenyl])-3-oxo-2-piperidin-2-yliden-propionitril mit einem Schmelzpunkt von 197°C.

### Beispiel 17

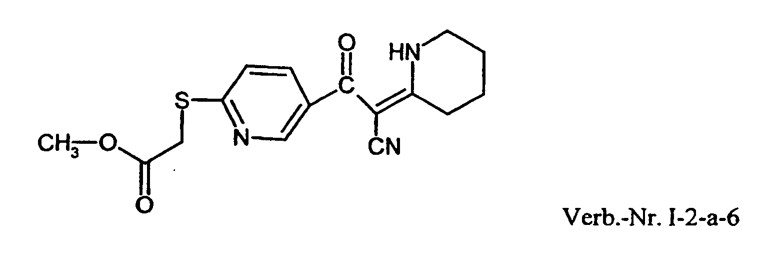

1,5 g (5,73 mmol) der Verbindung aus Beispiel Nr. I-2-a-2, 0,91 g (8.6 mmol) Thioglykolsäuremethylester und 0,91 g Natriumcarbonat werden in 10 ml wasserfreiem Aceton auf 80°C erhitzt. Nach 2 Stunden werden weitere 8,6 mmol des Thioglykolsäuremethylester und 8,6 mmol Natriummethanolat zugesetzt und für 24 Stunden unter Rückfluß erhitzt. Nach Abkühlung auf Raumtemperatur wird die Reaktionsmischung in 150 ml 1-M-HCl gegossen. Das Reaktionsprodukt wird als fester Rückstand abgesaugt und mehrmals mit 10 ml Methyl-tert.-butylether gewaschen.

Man erhielt 1,350 g 3-[(2-Methoxycarbonylmethylthio)-pyrid-5-yl)]-3-oxo-2-piperidin-2-yliden-propionitril (Ausbeute 71 % der Theorie) mit einem Schmelzpunkt von 146°C.

### Beispiel 18

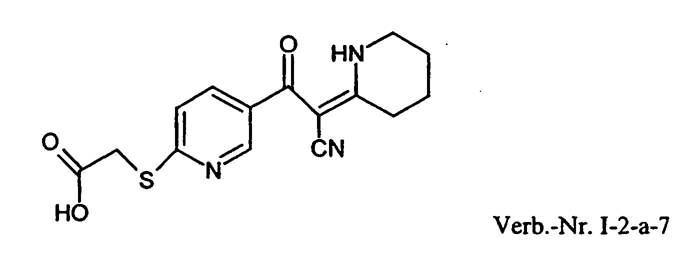

700 mg (2,1 mmol) der Verbindung aus Beispiel Nr. I-2-a-6, werden in 1,1 ml eines 1:1 Gemisches aus 2-N-NaOH und Methanol bei 0°C vorgelegt. Anschließend wird 1 Stunde bei 0°C und weitere 4 Stunden bei Raumtemperatur gerührt. Die erhaltene Reaktionslösung wird mit 6-N-HCl auf pH 6 eingestellt. Das ausgefallene Produkt wird auf Ton abgepreßt und am Hochvakuum getrocknet.

Man erhielt 551 mg (Ausbeute: 82,7 % der Theorie) 3-[4-(Essigsäurethio)-pyridyl]-3-oxo-2-pyridinyl-2-yliden-propionitril mit einem Schmelzpunkt von 155°C.

In Analogie zu den Herstellungsbeispielen 1 und 2 und gemäß den allgemeinen Angaben zur Herstellung von Verbindungen der Formel (I-1-a) erhält man folgende Verbindungen:

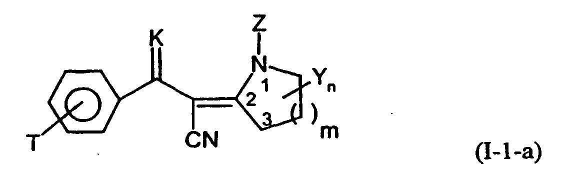

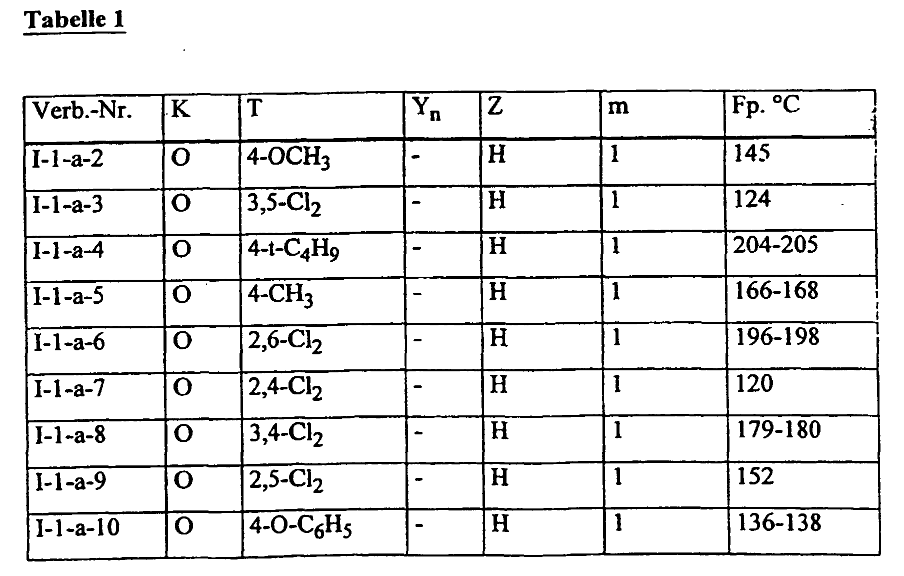

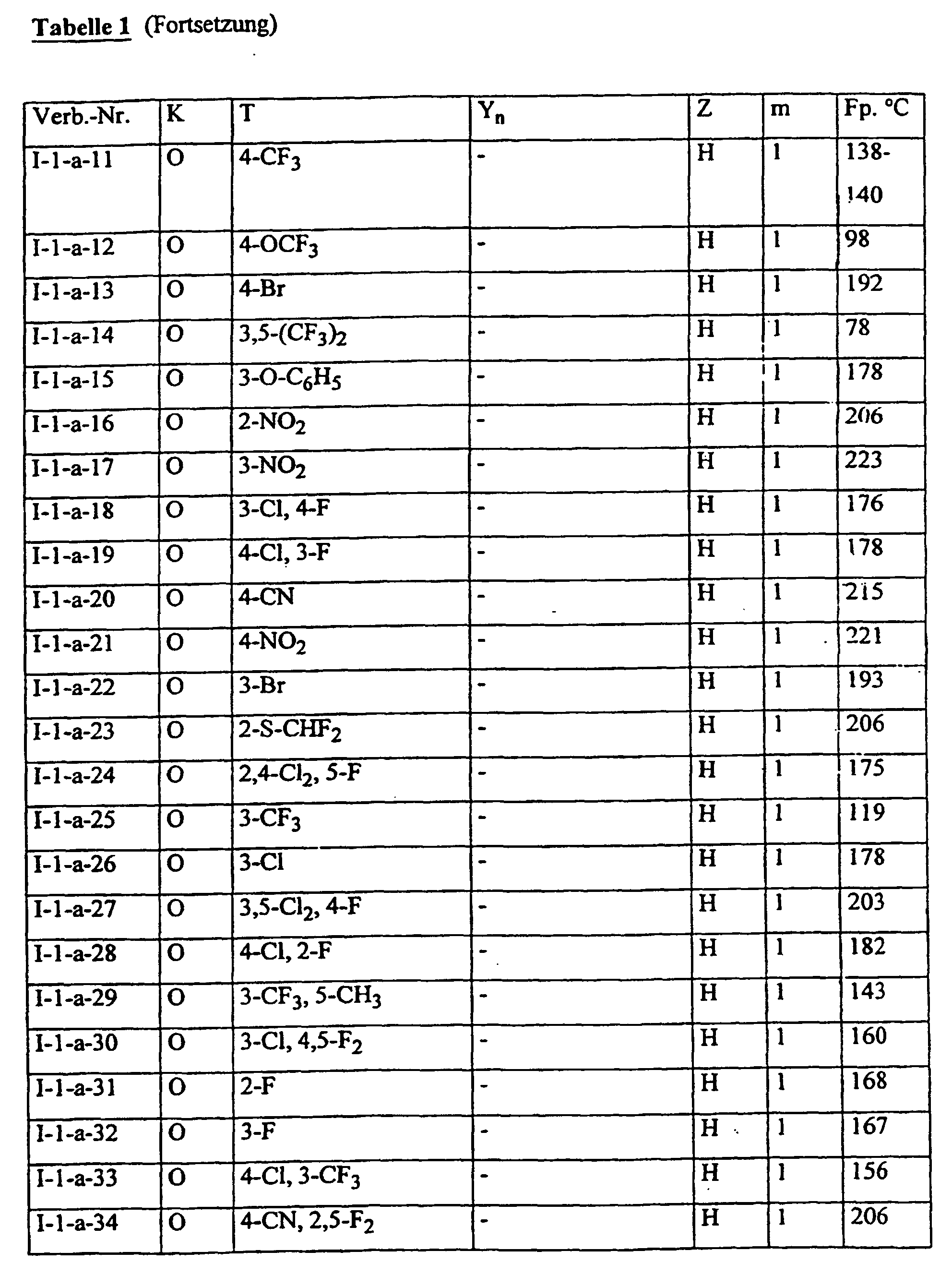

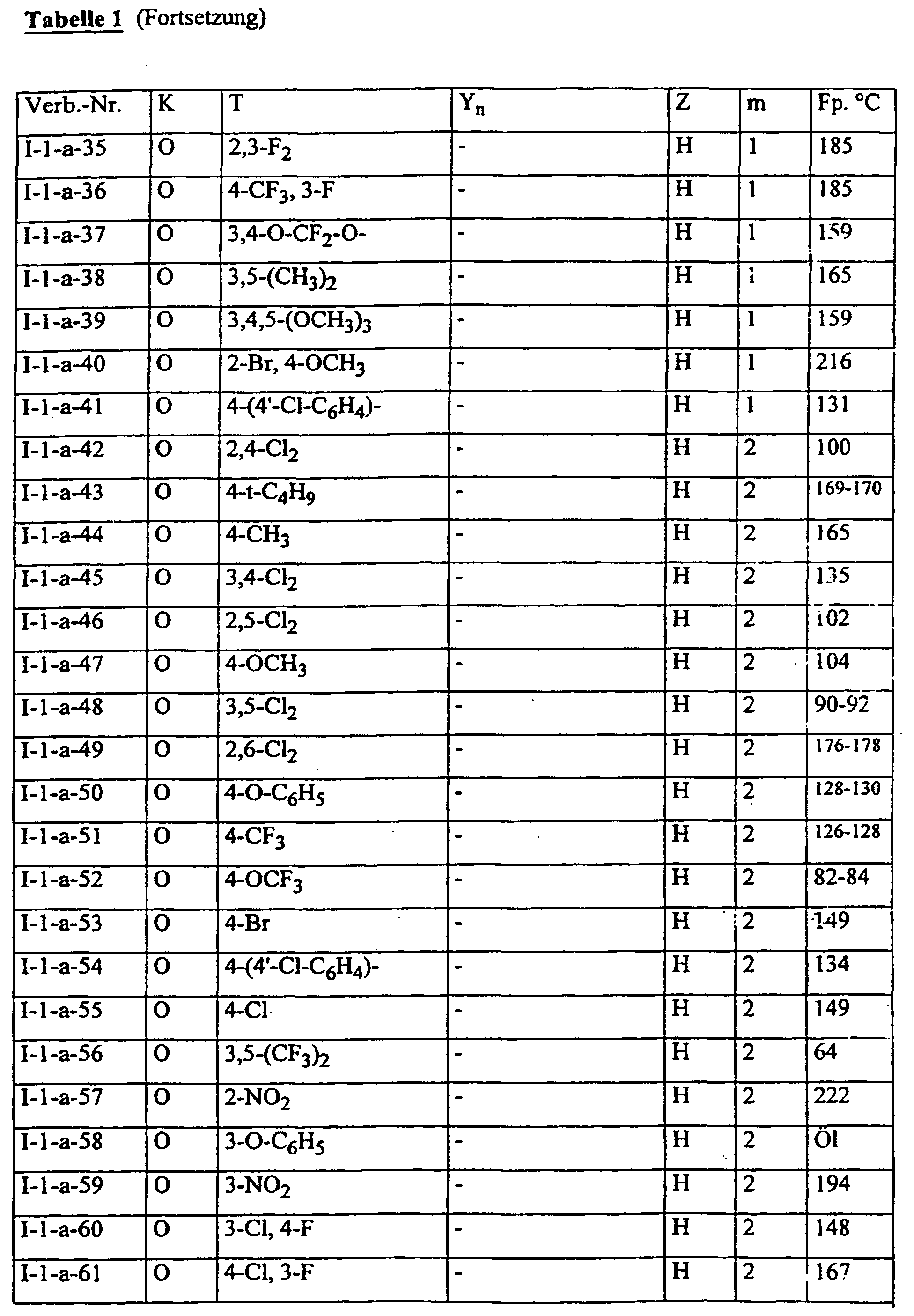

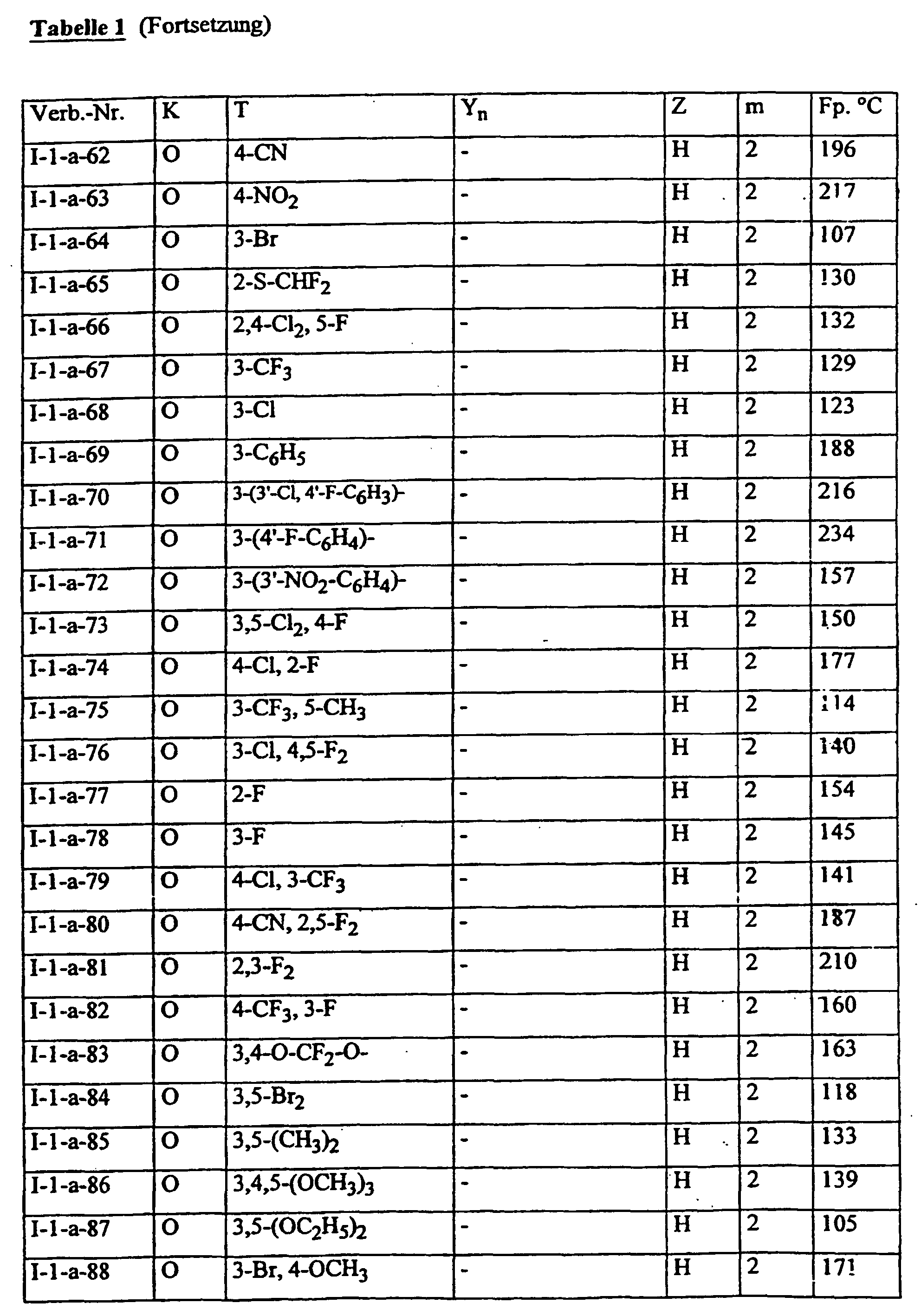

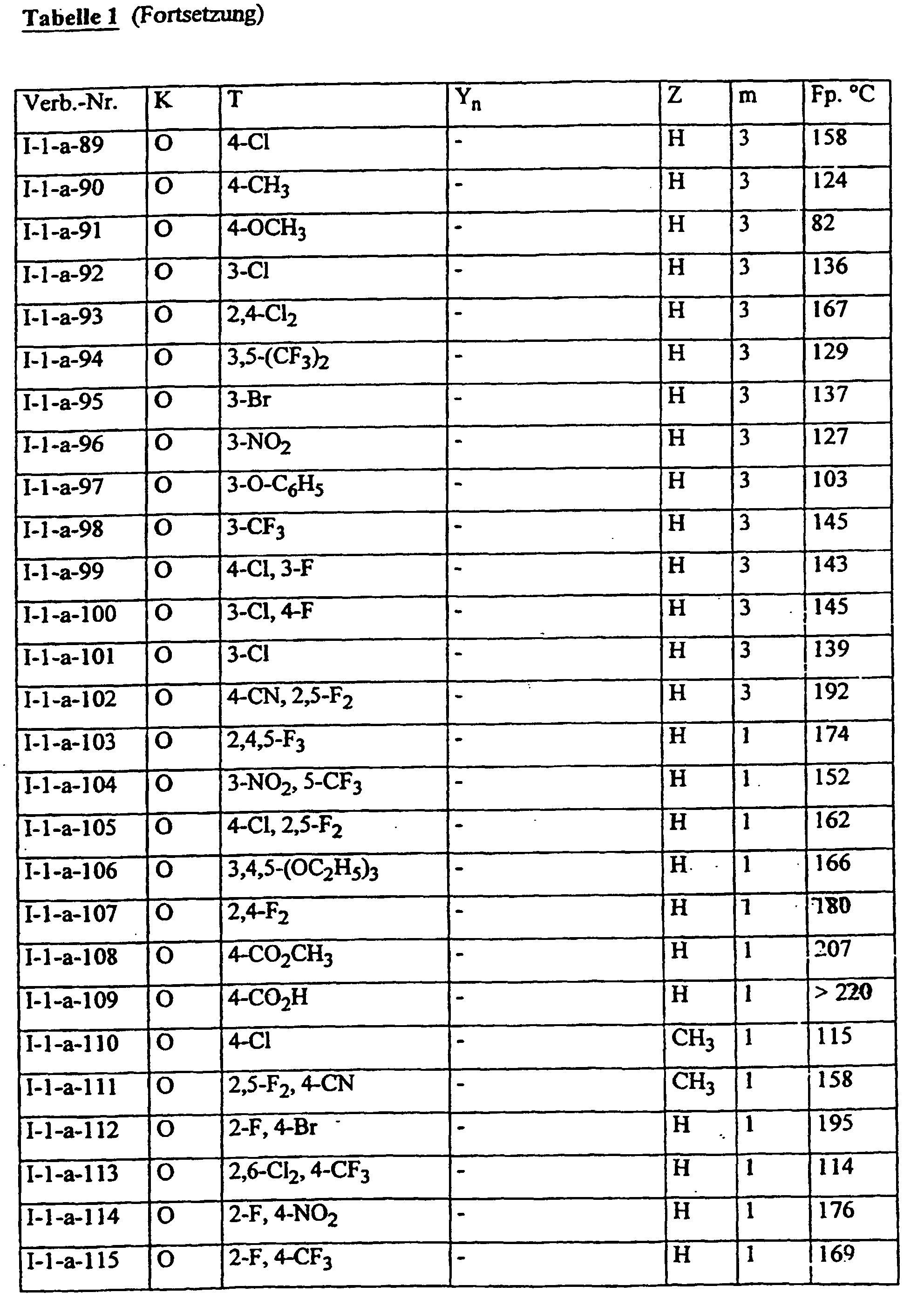

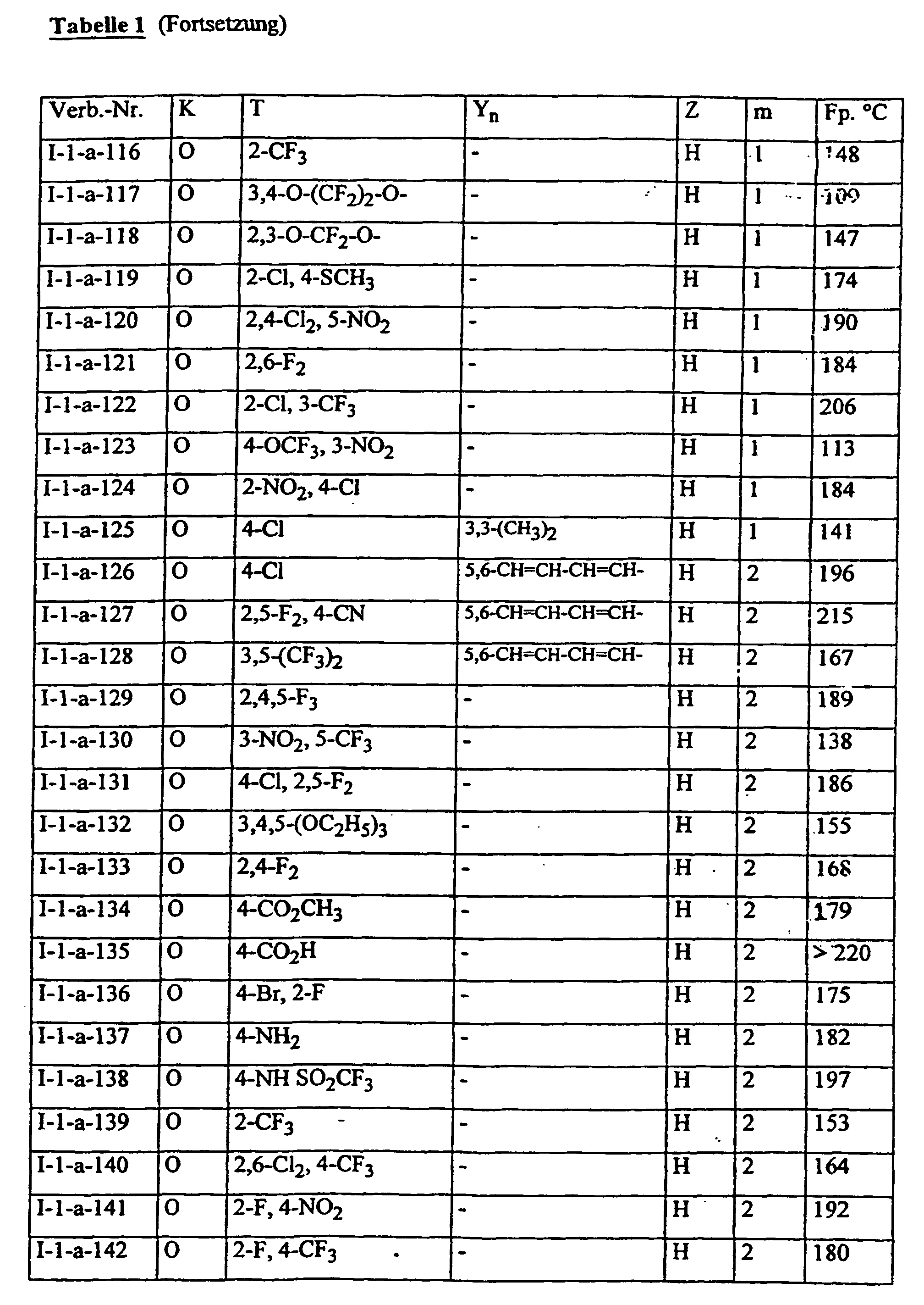

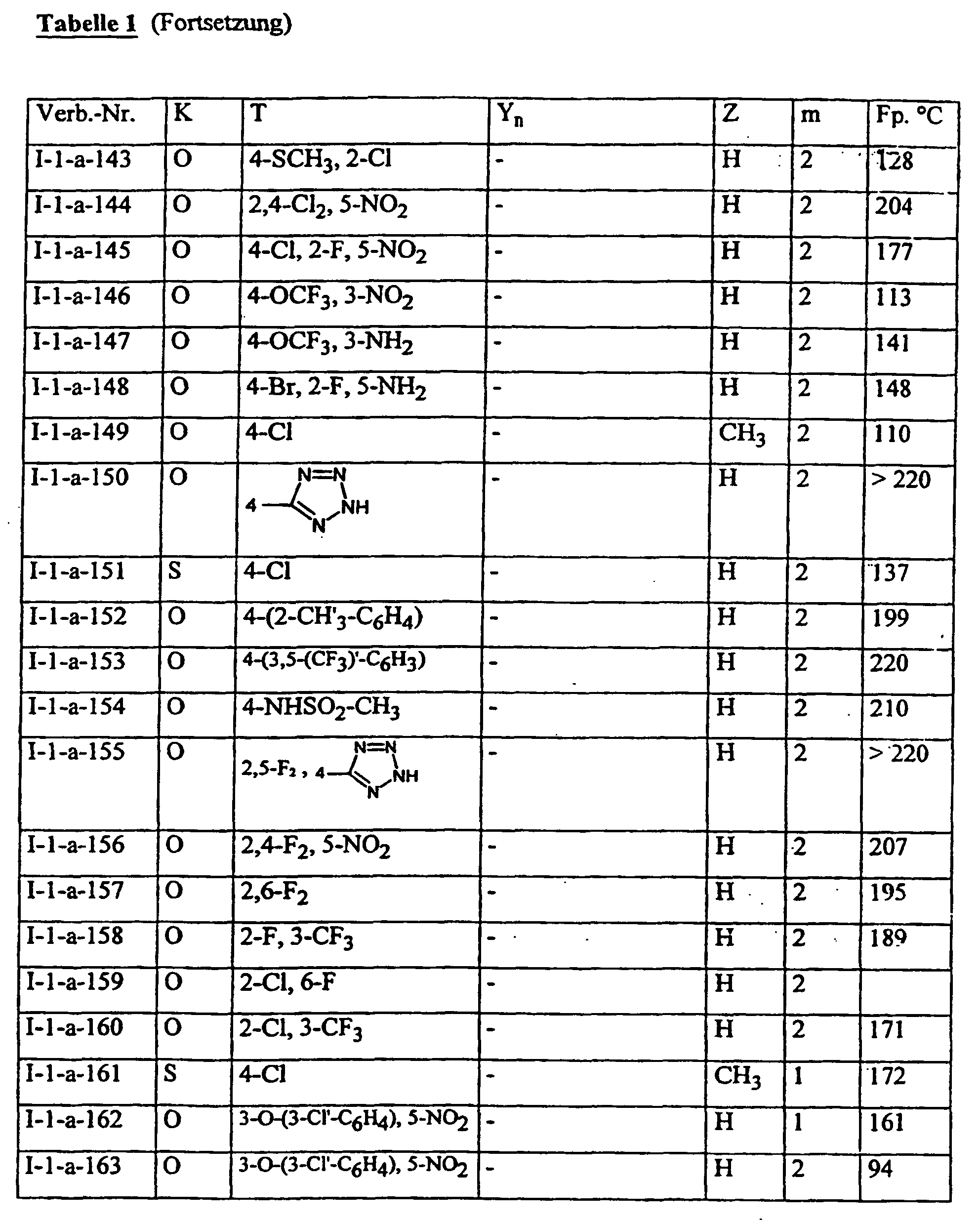

In Analogie zu Herstellungsbeispiel 1 und gemäß den allgemeinen Angaben zur Herstellung erhält man folgende Verbindungen der Formel I-2-a:

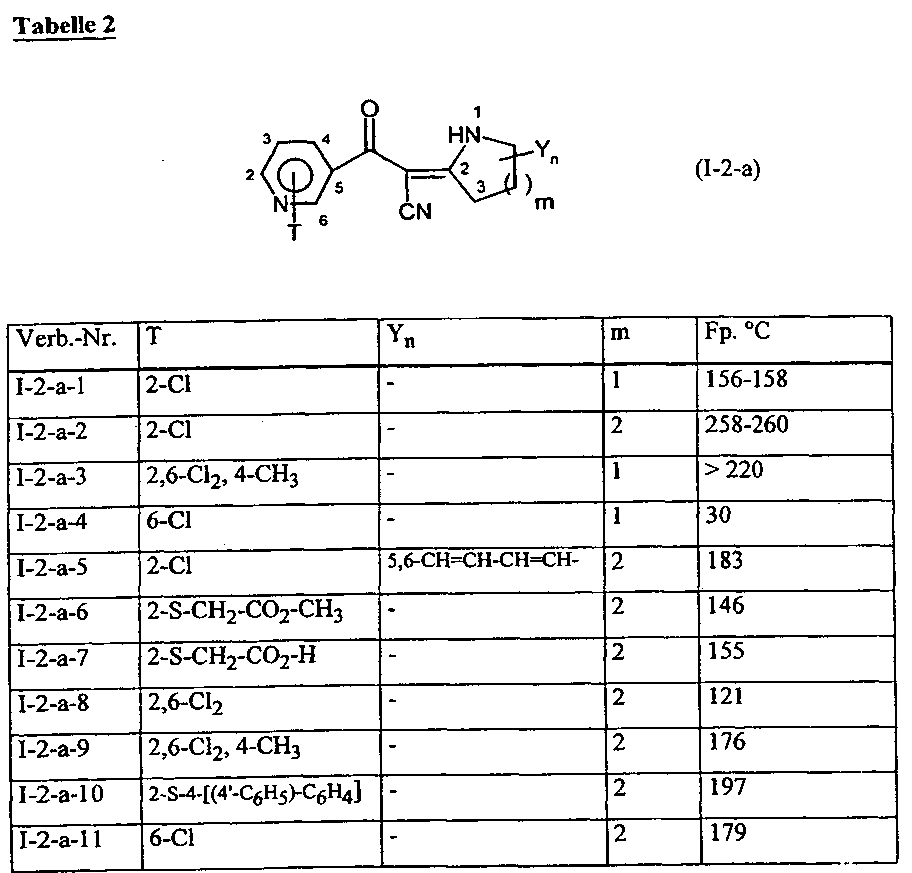

In Analogie zu Herstellungsbeispiel 1 und gemäß den allgemeinen Angaben zur Herstellung erhält man folgende Verbindungen der Formel I-3-a:

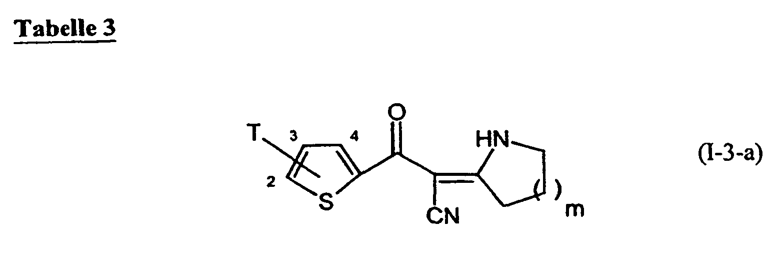

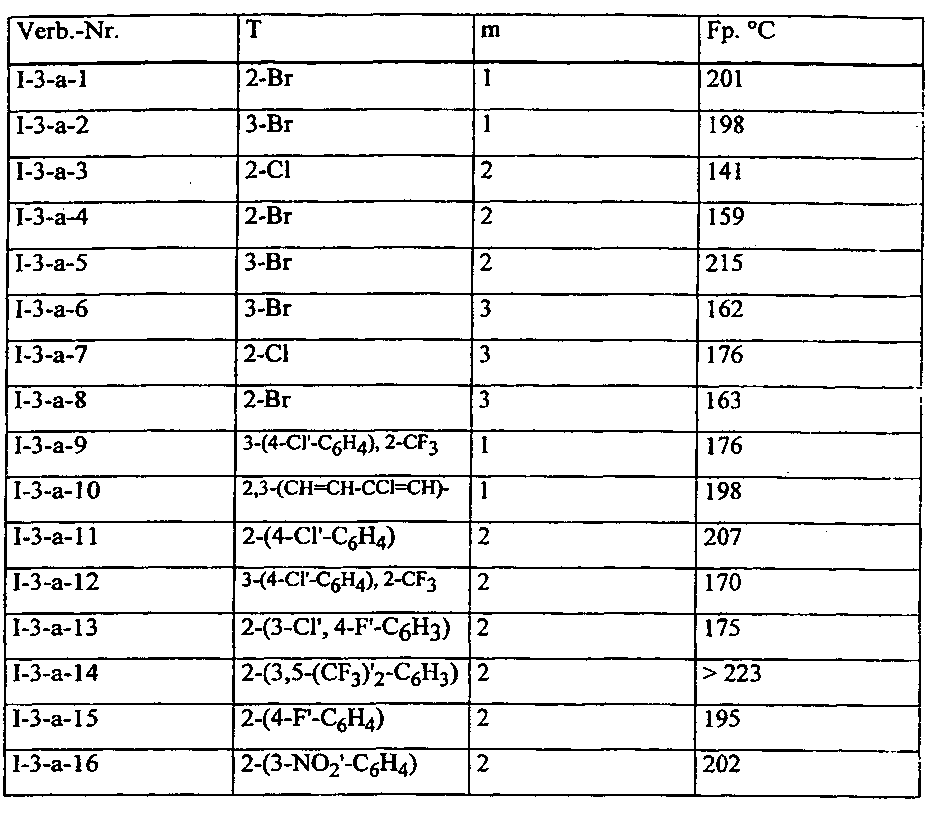

In Analogie zu Herstellungsbeispiel 1 und gemäß den allgemeinen Angaben zur Herstellung erhält man folgende Verbindungen der Formel (I-4-a)

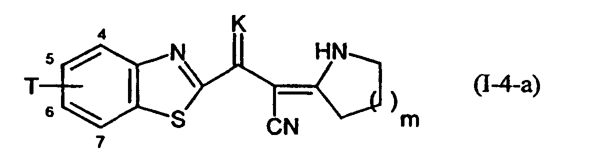

**Tabelle 4**

| Verb.-Nr. | K | T | Yₙ | m | Fp. °C |
|---|---|---|---|---|---|
| I-4-a-1 | O | 7-Cl | - | 1 | > 220 |
| I-4-a-2 | O | 7-Cl | - | 2 | > 220 |

In Analogie zu Herstellungsbeispiel 1 und gemäß den allgemeinen Angaben zur Herstellung erhält man folgende Verbindungen der Formel (I-5-a)

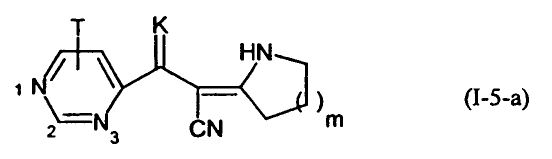

**Tabelle 5**

| Verb.-Nr. | K | T | Yₙ | m | Fp. °C |
|---|---|---|---|---|---|
| I-5-a-1 | O | 2-SCH₃, 5-Br | - | 1 | 170 |
| I-5-a-2 | O | 2-SCH₃, 5-Br | - | 2 | 180 |

In Analogie zu Herstellungsbeispiel 1 und gemäß den allgemeinen Angaben zur Herstellung erhält man folgende Verbindungen der Formel (I-6-a)

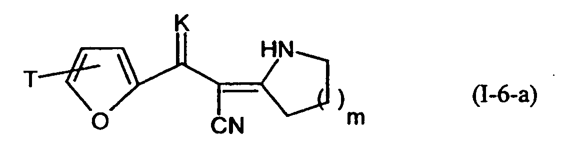

**Tabelle 6**

| Verb.-Nr. | K | T | Yₙ | m | Fp. °C |
|---|---|---|---|---|---|
| I-6-a-1 | O | H | - | 1 | 199 |
| I-6-a-2 | O | H | - | 2 | |

In Analogie zu Herstellungsbeispiel 3 und gemäß den allgemeinen Angaben zur Herstellung erhält man die Verbindungen der Formel I-1-b:

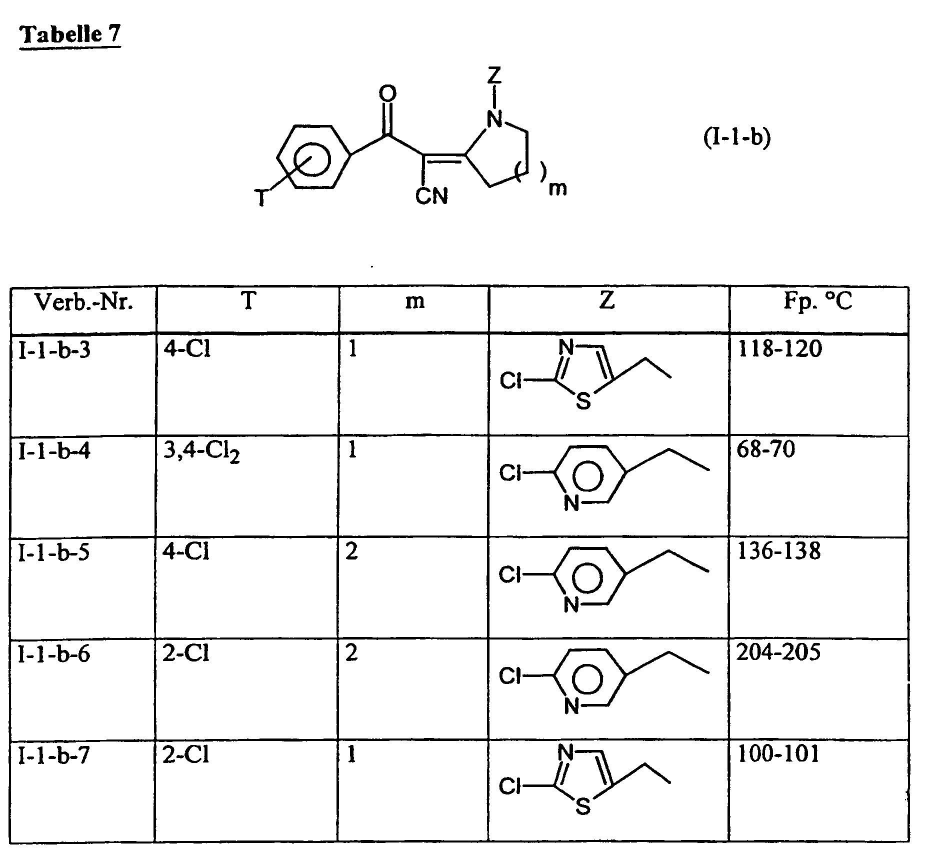

In Analogie zu Herstellungsbeispiel 7 erhält man die Verbindung I-1-d-4 vom Schmp. 222-224°C.

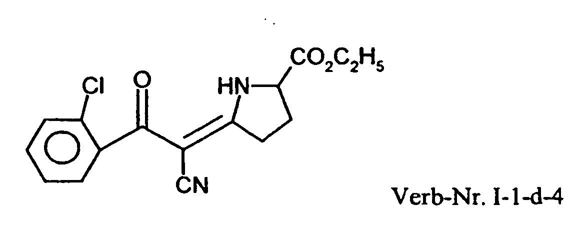

In Analogie zu Herstellungsbeispiel 3 erhält man die Verbindung I-1-d-5 vom Schmp. 130-132°C.

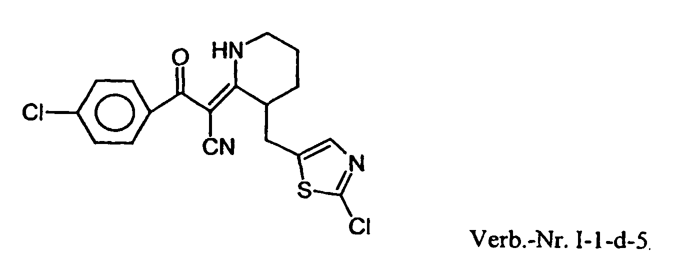

### Beispiel

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test zeigen z.B. die folgenden Verbindungen der Herstellungsbeispiele gute Wirksamkeit: I-1-a-11, I-1-a-12, I-1-a-52.

### Beispiel

### Spodoptera frugiperda-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test zeigen z.B. die folgenden Verbindungen der Herstellungsbeispiele gute Wirksamkeit: I-2-a-2, Verbindung gemäß Herstellungsbeispiel 3.

### Beispiel

### Tetranychus-Test (OP-resistent/Tauchbehandlung)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test zeigen z.B. die folgenden Verbindungen der Herstellungsbeispiele gute Wirksamkeit: I-1-a-7, I-1-a-48, I-1-a-56.

### Beispiel

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so, daß die jeweils gewünschten Wirkstoffmengen pro Flächeneinheit ausgebracht werden. Nach 3 Wochen wird der Schädigungsgrad der Pflanzen bonitiiert in % Schädigung im Vergleich zur Entwicklung der unbehandelten Kontrolle.

### Es bedeuten:

| | |
|---|---|
| 0 % | = keine Wirkung (wie unbehandelte Kontrolle) |
| 100 % | = totale Vernichtung |

In diesem Test zeigen beispielsweise die Verbindungen gemäß Herstellungsbeispiel I-1-a-9, I-1-a-14, I-1-a-20, I-1-a-54, I-1-a-55, I-1-a-62, I-2-a-2 bei teilweise guter Verträglichkeit gegenüber Kulturpflanzen, wie z.B. Weizen, starke Wirkungen gegen Unkräuter.

### Beispiel

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiiert in % Schädigung im Vergleich zur Entwicklung der unbehandelten Kontrolle.

### Es bedeuten:

| | |
|---|---|
| 0 % | = keine Wirkung (wie unbehandelte Kontrolle) |
| 100 % | = totale Vernichtung |

In diesem Test zeigen beispielsweise die Verbindungen gemäß Herstellungsbeispiel I-1-a-14, I-1-a-46, I-1-a-51, I-1-a-52, I-1-a-53, I-1-a-54, I-1-a-55, I-1-a-62, I-1-a-66, I-2-a-2, bei teilweise guter Verträglichkeit gegenüber Kulturpflanzen, wie z.B. Weizen und Soja, starke Wirkungen gegen Unkräuter.

## Patentansprüche

1. Verbindungen der allgemeinen Formel (I) 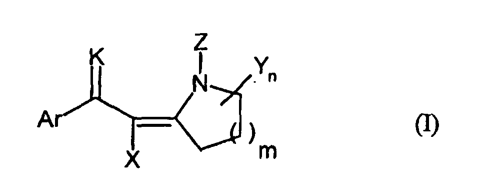 in welcher
Ar für Ar¹ steht, wobei Ar¹ für jeweils gegebenenfalls (jedoch im Falle von Phenyl und Naphtyl nicht für unsubstituiertes) einfach bis fünffach durch Halogen, C₁-C₈-Alkyl, C₂-C₈-Alkenyl, C₂-C₈-Alkinyl, C₁-C₈-Alkoxy, C₂-C₈-Alkenyloxy, C₃-C₈-Alkinyloxy, C₁-C₈-Alkylthio, C₁-C₆-Alkylsulfinyl, C₁-C₆-Alkylsulfonyl, C₁-C₆-Halogenalkyl, C₁-C₆-Halogen-alkoxy, C₂-C₈-Halogenalkenyloxy, C₁-C₂-Alkylidendiyl-dioxy, C₁-C₂-Halogenalkylidendiyl-dioxy, Halogen-C₁-C₄-alkylthio, Halogen-C₁-C₄-alkylsulfinyl, Halogen-C₁-C₄-alkylsulfonyl, Hydroxy, Mercapto, Nitro, Cyano, Amino, oder durch die Gruppen 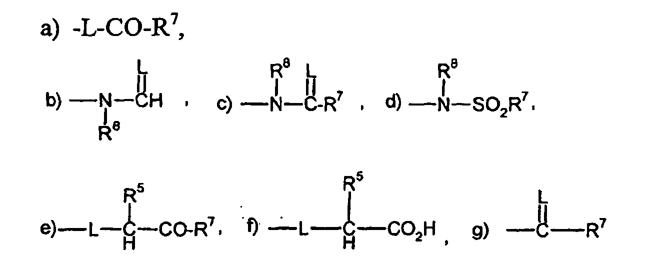 substituiertes Phenyl, Naphtyl oder mono- oder bicyclisches Hetaryl mit fünf bis zehn Ringatomen steht
oder für Ar² steht, wobei Ar² für Ar¹ steht, welches zusätzlich durch Phenyl, Naphthyl, fünf- oder sechsgliedriges Hetaryl, Phenyl-C₁-C₄-alkyl, Phenoxy, Phenyl-S(O)_{g}-, fünf- oder sechsgliedriges Hetaryloxy oder Hetaryl-S(O)_{g} substituiert ist, wobei diese Substituenten ihrerseits jeweils gegebenenfalls einfach bis vierfach durch Halogen, C₁-C₆-Alkyl, C₁-C₆-Allcoxy, C₁-C₄-Halogenalkyl, C₁-C₄-Halogenalkoxy, Nitro oder Cyano substituiert sind, und
K für Sauerstoff oder Schwefel steht,
L für Sauerstoff oder Schwefel steht,
X für
CN, 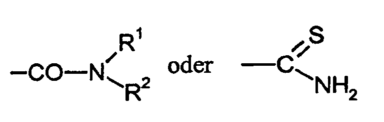
steht,
Y für Halogen, C₁-C₆-Akyl, C₁-C₄-Halogenalkyl, C₁-C₆-Alkoxy, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Phenyl-C₁-C₄-alkyl, fünf- oder sechsgliedriges Hetaryl oder fünf- oder sechsgliedriges Hetaryl-C₁-C₄-alkyl oder für die Gruppen 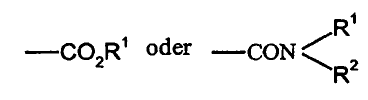 steht, oder
zwei benachbarte Yₙ für einen 5- bis 8-gliedrigen, gesättigten oder ungesättigten Cyclus stehen, der durch 1 bis 3 Heteroatome aus der Reihe N, O, S unterbrochen sein kann und gegebenenfalls einfach bis dreifach durch Halogen, C₁-C₄-Alkyl, C₁-C₄-Alkoxy, C₁-C₄-Halogenalkyl, C₁-C₄-Halogenalkoxy, Cyano oder Nitro substituiert sein kann, und
Z für Wasserstoff, für jeweils gegebenenfalls durch Fluor und/oder Chlor substituiertes C₁-C₈-Alkyl, Cyano-C₁-C₆-alkyl, C₃-C₈-Alkenyl, C₃-C₈-Alkinyl, C₃-C₈-Cycloalkyl, C₃-C₈-Cycloalkyl-C₁-C₂-alkyl, C₁-C₄-Alkoxy-C₁-C₄-alkyl, C₁-C₄-Halogenalkoxy-C₁-C₄-alkyl, für jeweils gegebenenfalls einfach bis vierfach durch Halogen, C₁-C₆-Alkyl, C₁-C₆-Alkoxy, C₁-C₄-Halogenalkyl, C₁-C₄-Halogen-alkoxy, Nitro oder Cyano substituiertes Phenoxy-C₁-C₄-alkyl, Phenyl-C₁-C₄-alkyloxy-C₁-C₄-alkyl, Phenylthio-C₁-C₄-alkyl, Phenyl-C₁-C₄-alkylthio-C₁-C₄-alkyl, Phenyl, Phenyl-C₁-C₄-alkyl, fünf- oder sechsgliedriges Hetaryl, fünf- oder sechsgliedriges Hetaryl-C₁-C₄-alkyl oder für die Gruppen
―CO₂R¹ ; 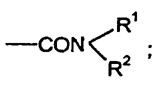
―SO₂R¹ ;
―COR¹ ; 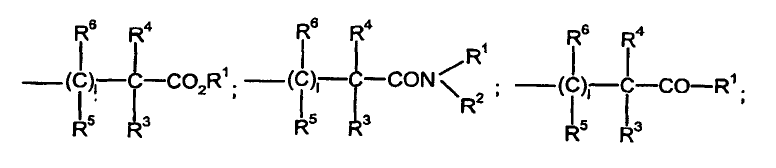
oder Cyano steht,
g für 0 bis 2 steht,
l für 0 bis 2 steht,
R¹ für Wasserstoff (nicht jedoch für die Reste -CO₂R¹ und -SO₂R¹), für jeweils gegebenenfalls durch Fluor und/oder Chlor substituiertes C₁-C₁₀-Alkyl, C₂-C₁₀-Alkenyl, C₃-C₆-Alkinyl, für jeweils gegebenenfalls durch Fluor, Chlor, C₁-C₄-Alkyl, C₁-C₄-Alkoxy substituiertes C₃-C₈-Cycloalkyl oder C₅-C₈-Cycloalkenyl, in welchem gegebenenfalls eine Methylengruppe durch Sauerstoff oder Schwefel unterbrochen sein kann, oder für jeweils gegebenenfalls durch einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Pyridyl, Thienyl, Pyrimidyl, Thiazolyl, Phenyl-C₁-C₄-alkyl, Pyridyl-C₁-C₂-alkyl, Thiazolyl-C₁-C₂-alkyl steht,
R² für Wasserstoff, für jeweils gegebenenfalls durch Fluor und/oder Chlor substituiertes C₁-C₆-Alkyl, C₃-C₆-Alkenyl, C₃-C₆-Allanyl, C₁-C₆-Alkoxy, C₃-C₆-Alkenyloxy oder für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Phenyl-C₁-C₄-alkyl oder Phenyl-C₁-C₄-alkoxy steht, oder
R¹, R² gemeinsam mit dem Stickstoffatom, an das sie gebunden sind, für einen gegebenenfalls durch C₁-C₄-Alkyl substituierten fünf- bis achtgliedrigen Cyclus, bei dem gegebenenfalls eine Methylengruppe durch Sauerstoff oder Schwefel ersetzt sein kann, stehen,
R³ für Wasserstoff, gegebenenfalls durch Halogen substituiertes C₁-C₆-Alkyl oder für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Phenyl-C₁-C₂-alkyl steht,
R⁴ für Wasserstoff oder C₁-C₆-Alkyl steht,
R⁵, R⁶ unabhängig voneinander für Wasserstoff oder gegebenenfalls durch Fluor und/oder Chlor substituiertes C₁-C₄-Alkyl stehen,
R⁷ für jeweils gegebenenfalls durch Fluor und/oder Chlor substituiertes C₁-C₁₀-Alkyl, C₂-C₁₀-Alkenyl, C₃-C₁₀-Alkinyl, C₁-C₁₀-Alkoxy, C₁-C₄-Alkoxy-C₁-C₄-alkyl, C₁-C₄-Alkylthio-C₁-C₄-alkyl, jeweils gegebenenfalls durch Fluor und/oder Chlor, C₁-C₄-Alkyl, C₁-C₄-Alkoxy substituiertes C₃-C₈-Cycloalkyl oder C₃-C₈-Cycloalkyloxy, in welchem gegebenenfalls eine Methylengruppe durch Sauerstoff oder Schwefel ersetzt sein kann, für jeweils gegebenenfalls einfach bis vierfach durch Halogen, C₁-C₆-Alkyl, C₁-C₆-Alkoxy, C₁-C₄-Halogen-alkyl, C₁-C₄-Halogenalkoxy, Cyano oder Nitro substituiertes Phenyl, Phenoxy, Benzyloxy, fünf- oder sechsgliedriges Hetaryl oder Phenyl-C₁-C₄-alkyl oder im Falle der unter Ar genannten Reste a) und c) auch für eine Gruppe 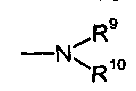 oder für den Rest g) auch für Hydroxy steht,
R⁸ für Wasserstoff oder C₁-C₄-Alkyl steht,
R⁹ für Wasserstoff, jeweils gegebenenfalls durch Fluor und/oder Chlor substituiertes C₁-C₁₀-Alkyl, C₃-C₈-Alkenyl, C₃-C₈-Alkinyl, C₁-C₁₀-Alkoxy, C₃-C₈-Alkenyl-oxy, gegebenenfalls durch Fluor, Chlor, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt sein kann, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Phenyl-C₁-C₄-alkyl oder Phenyl-C₁-C₂-alkoxy steht,
R¹⁰ für Wasserstoff, C₁-C₆-Alkyl oder C₃-C₆-Alkenyl steht, oder
R⁹, R¹⁰ gemeinsam mit dem Stickstoffatom, an das sie gebunden sind, für einen gegebenenfalls durch C₁-C₄-Alkyl substituierten fünf- bis achtgliedrigen Cyclus, bei dem gegebenenfalls eine Methylengruppe durch Sauerstoff oder Schwefel ersetzt sein kann, stehen und
m für 1 bis 3 steht, und
n in Abhängigkeit von m für 0 bis 3 steht
ausgenommen Verbindungen mit der folgenden Formel 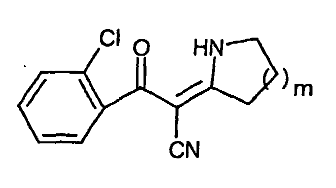 m = 1, 2, 3.

2. Verbindungen gemäß Anspruch 1, **dadurch gekennzeichnet, dass**
K für Sauerstoff oder Schwefel steht,
Ar für Ar¹ steht, wobei Ar¹ für jeweils gegebenenfalls (jedoch im Fallt von Phenyl und Naphtyl nicht für unsubstituiertes) einfach bis dreifach durch Fluor, Chlor, Brom, C₁-C₆-Alkyl, C₂-C₆-Alkenyl, C₂-C₆-Alkinyl, C₁-C₆-Alkoxy, C₃-C₆-Alkenyloxy, C₃-C₆-Alkinyloxy, C₁-C₆-Alkylthio, C₁-C₄-Alkylsulfinyl, C₁-C₄-Alkylsulfonyl, C₁-C₄-Halogenalkyl, C₁-C₄-Halogen-alkoxy, C₂-C₄-Halogenalkenyloxy, C₁-C₂-Alkylidendiyl-dioxy, C₁-C₂-Halogenalkylidendiyl-dioxy, Halogen-C₁-C₂-alkylthio, Halogen-C₁-C₂-alkylsulfinyl, Halogen-C₁-C₂-alkylsulfonyl, Hydroxy, Mercapto, Nitro, Cyano, Amino oder durch einen der folgenden Gruppen 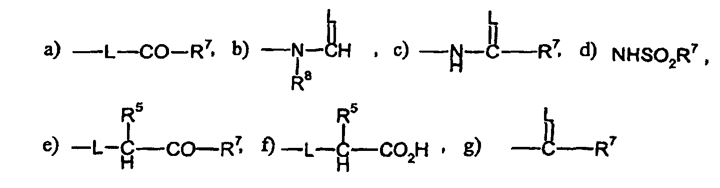 substituiertes Phenyl, Naphtyl, Chinolinyl, Thienyl, Pyrimidyl, Furanyl, Thiazolyl, Benzthiazolyl, Oxazolyl, Pyrazolyl oder Pyridyl steht
oder für Ar² steht, wobei Ar² für Ar¹ steht, welches zusätzlich durch Phenyl, Pyridyl, Pyrimidyl, Thienyl, Furanyl, Thiazolyl, Tetrazolyl, Triazolyl, Benzyl, Phenoxy, Phenyl-S(O)_{g}-, Pyridyloxy, Pyrimidyloxy, Thiazolyloxy, Pyridyl-S(O)_{g}-, Pyrimidyl-S(O)_{g}- oder Thiazolyl-S(O)_{g}- substituiert ist, wobei diese Substituenten ihrerseits gegebenenfalls einfach bis dreifach durch Fluor, Chlor, Brom, C₁-C₄-Alkyl, C₂-C₄-Alkenyl, C₁-C₄-Alkoxy, C₁-C₂-Halogenalkyl, C₁-C₂-Halogenalkoxy, Nitro oder Cyano substituiert sind, wobei g für 0 bis 2 steht, und
L für Sauerstoff oder Schwefel steht,
X für CN, 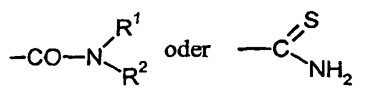 steht,
Y für Fluor, C₁-C₄-Alkyl, C₁-C₂-Halogenalkyl, C₁-C₄-Alkoxy, für jeweils gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, Phenyl-C₁-C₂-alkyl, Thiazolylinethyl, Pyridylmethyl oder für die Gruppen 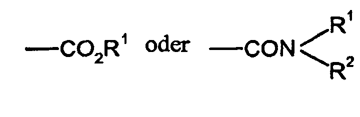 steht, oder
zwei benachbarte Yₙ weiterhin für einen 5- bis 6-gliedrigen, gesättigten oder ungesättigten Cyclus stehen, der durch ein Heteroatom aus der Reihe N, O, S unterbrochen sein kann und gegebenenfalls einfach bis zweifach durch Fluor, Chlor, Brom, Methyl, t-Butyl, Methoxy, Trifluormethyl, Trifluormethoxy, Cyano oder Nitro substituiert sein kann, und
Z für Wasserstoff, für jeweils gegebenenfalls Fluor und/oder Chlor substituiertes C₁-C₆-Alkyl, Cyano-C₁-C₃-alkyl, C₃-C₆-Alkenyl, C₃-C₈-Alkinyl, C₃-C₆-Cycloalkyl, C₃-C₆-Cycloalkyl-C₁-C₂-alkyl, C₁-C₄-Alkoxy-C₁-C₂-alkyl, C₁-C₄-Halogen-C₁-C₂-alkyl, für jeweils gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy-C₁-C₂-alkyl, Phenyl-C₁-C₂-alkyloxy-C₁-C₂-alkyl, Phenylthio-C₁-C₂-alkyl, Phenyl-C₁-C₂-alkylthio-C₁-C₂-alkyl, Phenyl-C₁-C₂-alkyl, Phenyl, Pyridyl-C₁-C₂-alkyl, Thiazolyl-C₁-C₂-alkyl oder für die Gruppen
―CO₂R¹ ; 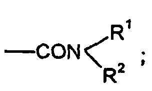
―SO₂R1;
―COR¹; 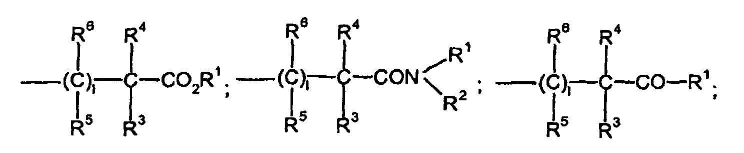
oder Cyano steht,
l für 0 bis 1 steht,
R¹ für Wasserstoff (jedoch nicht für die Reste -CO₂R¹ und -SO₂R¹), für jeweils gegebenenfalls durch Fluor und/oder Chlor substituiertes C₁-C₆-Alkyl, C₂-C₆-Alkenyl, C₃-C₄-Alkinyl, für gegebenenfalls durch Fluor, Chlor, C₁-C₂-Alkyl, C₁-C₂-Alkoxy substituiertes C₃-C₆-Cycloalkyl oder für jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Benzyl steht,
R² für Wasserstoff, für jeweils gegebenenfalls durch Fluor und/ oder Chlor substituiertes C₁-C₄-Alkyl, C₃-C₄-Alkenyl, C₃-C₄-Alkinyl, C₁-C₄-Alkoxy, C₃-C₄-Alkenyloxy oder für jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, Benzyl oder Benzyloxy steht, oder
R¹, R² gemeinsam mit dem Stickstoffatom, an das sie gebunden sind, für einen gegebenenfalls durch C₁-C₂-Alkyl substituierten fünf- bis sechsgliedrigen Cyclus stehen, bei dem gegebenenfalls eine Methylengruppe durch Sauerstoff ersetzt sein kann,
R³ für Wasserstoff, für C₁-C₄-Alkyl oder für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl oder Benzyl steht,
R⁴, R⁵, R⁶ für Wasserstoff, Methyl oder Ethyl stehen,
R⁷ für jeweils gegebenenfalls durch Fluor und/oder Chlor substituiertes C₁-C₆-Alkyl, C₂-C₆-Alkenyl, C₃-C₆-Alkinyl, C₁-C₆-Alkoxy, C₁-C₄-Alkoxy-C₁-C₂- alkyl, C₁-C₄-Alkylthio-C₁-C₂-alkyl, jeweils gegebenenfalls durch Fluor und/oder Chlor, C₁-C₂-Alkyl, C₁-C₂-Alkoxy substituiertes C₃-C₆-Cycloalkyl, in welchem gegebenenfalls eine Methylengruppe durch Sauerstoff ersetzt sein kann, für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl, Phenoxy, Benzyloxy, Thienyl, Furanyl, Pyridyl, Pyrimidyl, Thiazolyl, Pyrazolyl oder Phenyl-C₁-C₂-alkyl oder im Fall der unter Ar genannten Reste a) und c) auch für eine Gruppe 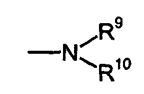 oder für den Rest g) auch für Hydroxy steht,
R⁸ für Wasserstoff steht,
R⁹ für Wasserstoff, jeweils gegebenenfalls durch Fluor und/oder Chlor substituiertes C₁-C₆-Alkyl, C₃-C₆-Alkenyl, C₃-C₆-Alkmyl, C₁-C₆-Alkoxy, C₃-C₆-Cycloalkyl, in welchem gegebenenfalls eine Methylengruppe durch Sauerstoff ersetzt sein kann, für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl oder Phenyl-C₁-C₂-alkyl steht,
R¹⁰ für Wasserstoff oder C₁-C₄-Alkyl steht, oder
R⁹, R¹⁰ gemeinsam mit dem Stickstoffatom, an das sie gebunden sind, für einen gegebenenfalls durch C₁-C₂-Alkyl substituierten fünf- bis sechsgliedrigen Cyclus stehen, bei dem gegebenenfalls eine Methylengruppe durch Sauerstoff ersetzt sein kann, und
m für 0 bis 1 steht, und
n in Abhängigkeit von m für 0 bis 2 steht.

3. Verbindungen gemäß Anspruch 1, **dadurch gekennzeichnet, dass**
K für Sauerstoff oder Schwefel steht,
Ar für Ar¹ steht, wobei Ar¹ für jeweils gegebenenfalls (jedoch im Falle von Phenyl und Naphtyl nicht für unsubstituiertes) einfach bis dreifach durch Fluor, Chlor, Brom, Methyl, Ethyl, Propyl, i-Propyl, s-, n-, i- oder t-Butyl, Methoxy, Ethoxy, Propoxy, i-Propoxy, s-, n-, i-oder t-Butoxy, Allyloxy, Methallyloxy, 2-Butenyloxy, Propargyloxy, 2-Butinyloxy, Methylthio, Ethylthio, Methylsulfinyl, Ethylsulfinyl, Methylsulfonyl, Ethylsulfonyl, Trifluormethyl, Difluormethoxy, Trifluormethoxy, Methylendioxy, Difluormethylendioxy, Tetrafluorethylendioxy, Difluormethylthio, Trifluormethylthio, Trifluormethylsulfinyl, Trifluormethylsulfonyl, Hydroxy, Mercapto, Nitro, Cyano, Amino oder durch eine der folgenden Gruppen 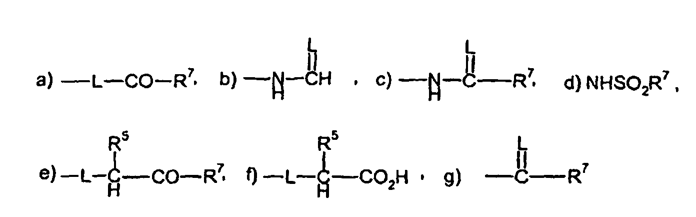 substituiertes Phenyl, Thienyl, Pyrimidyl, Furanyl, oder Pyridyl steht, oder für Ar² steht, wobei Ar² für Ar¹ steht, welches zusätzlich durch Phenyl, Pyridyl, Thienyl, Tetrazolyl, Triazolyl oder Phenoxy substituiert ist, wobei diese Substituenten ihrerseits gegebenenfalls einfach bis zweifach durch Fluor, Chlor, Brom, Methyl, Ethyl, n- oder i-Propyl, s-, n-, i- oder t-Butyl, Methoxy, Ethoxy, i-Propoxy, s-, n- oder t-Butoxy, Trifluormethyl, Trifluormethoxy, Nitro oder Cyano substituiert sind, und
L für Sauerstoff oder Schwefel steht,
X für
―CN,
―CO―NH₂, 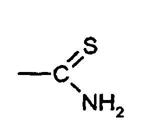
steht,
Y für Methyl, gegebenenfalls einfach bis dreifach durch Fluor, Chlor, Brom, Methyl, Methoxy, Trifluormethyl oder Trifluormethoxy substituiertes Phenyl oder für die Gruppe
―CO₂R¹ steht, oder
zwei benachbarte Yₙ für einen sechsgliedrigen, ungesättigten Cyclus stehen, der gegebenenfalls einfach durch Fluor, Chlor, Brom, Methyl, Methoxy, Trifluormethyl, Trifluormethoxy, Cyano oder Nitro substituiert sein kann, und
Z für Wasserstoff, Methyl, Ethyl, Allyl, Propargyl, Cyclopropyl, Cyclopentyl, Cyclohexyl, Cyclopropylmethyl, Cyclopentylinethyl, Cyclohexylmethyl, Methoxymethyl, Ethoxymethyl, für jeweils gegebenenfalls einfach bis zweifach durch Fluor, Chlor, Brom, Methyl, Methoxy, Trifluormethyl oder Trifluormethoxy, Cyano oder Nitro substituiertes Phenyl, Benzyl, Pyridylmethyl, Thiazolylmethyl oder für die Gruppen
―CO₂R¹, 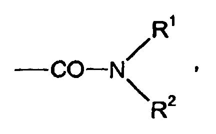
―SO₂R¹,
―CO―R¹, 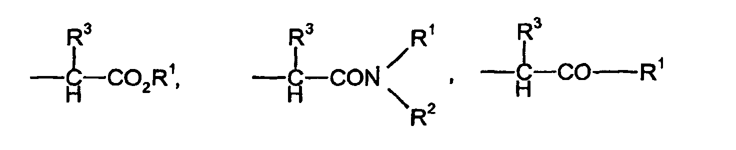
oder Cyano steht,
R¹ für Wasserstoff (jedoch nicht für die Reste -CO₂R¹ und -SO₂R¹), Methyl, Ethyl, Propyl, Isopropyl, n-, s-, i- oder t-Butyl, Allyl, Propargyl, Cyclopropyl, Cyclopentyl, Cyclohexyl, für jeweils gegebenenfalls einfach bis zweifach durch Fluor, Chlor, Brom, Methyl, Methoxy, Trifluormethyl, Trifluormethoxy, Cyano oder Nitro substituiertes Phenyl oder Benzyl steht,
R² für Wasserstoff, Methyl, Ethyl, Propyl, Isopropyl, Allyl, Propargyl, Methoxy, Ethoxy, Allyloxy oder für gegebenenfalls einfach bis zweifach durch Fluor, Chlor, Brom, Methyl, Methoxy, Trifluormethyl, Trifluormethoxy, Cyano oder Nitro substituiertes Benzyloxy steht, oder
R¹, R² gemeinsam mit dem Stickstoffatom, an das sie gebunden sind für einen Pyrrolidin-, Piperidin-, Thiazin- oder Morpholinrest stehen,
R³, R⁵ für Wasserstoff, Methyl oder Ethyl stehen,
R⁷ für Methyl, Ethyl, Propyl, Isopropyl, n-, s-, i- oder t-Butyl, Vinyl, Trifluormethyl, Methoxy, Ethoxy, Propoxy, Isopropoxy, n-, s-, i- oder t-Butyloxy, Cyclopropyl, Cyclopentyl, Cyclohexyl, Cyclopentyloxy, Cyclohexyloxy, für jeweils gegebenenfalls einfach bis zweifach durch Fluor, Chlor, Brom, Methyl, n-, s-, i- oder t-Butyl, Methoxy, Trifluormethyl, Trifluormethoxy, Cyano oder Nitro substituiertes Phenyl, Pyridyl oder Benzyl oder im Fall der unter Ar genannten Reste a) und c) auch für eine Gruppe 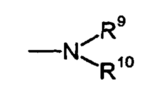 oder für den Rest g) auch für Hydroxy steht,
R⁹ für Wasserstoff, Methyl, Ethyl, Propyl, Isopropyl, n-, s-, i- oder t-Butyl, Cyclopropyl, Cyclopentyl, Cyclohexyl, für gegebenenfalls einfach bis zweifach durch Fluor, Chlor, Brom, Methyl, Methoxy, Trifluormethyl, Trifluormethoxy, Cyano oder Nitro substituiertes Phenyl steht,
R¹⁰ für Wasserstoff, Methyl oder Ethyl steht, oder
R⁹, R¹⁰ gemeinsam mit dem Stickstoffatom, an das sie gebunden sind für einen Pyrrolidin-, Piperidin-, oder Morpholinrest stehen, und
m für 1 bis 3 steht, und
n in Abhängigkeit von m für 0 bis 1 steht.

4. Verbindungen gemäß Anspruch 1, **dadurch gekennzeichnet, dass**
K für Sauerstoff oder Schwefel steht,
Ar für Ar¹ steht, wobei Ar¹ für jeweils gegebenenfalls einfach bis dreifach durch Fluor, Chlor, Brom, Methyl, Ethyl, Propyl, Isopropyl, n-, s-, i- oder t-Butyl, Methoxy, Ethoxy, Propoxy, Isopropoxy, tert.-Butoxy, Allyloxy, Methallyloxy, 2-Butenyloxy, Propargyloxy, 2-Butinyloxy, Methylthio, Ethylthio, Methylsulfinyl, Ethylsulfinyl, Methylsulfonyl, Ethylsulfonyl, Trifluormethyl, Difluormethoxy, Trifluormethoxy, Methylendioxy, Difluormethylendioxy, Tetrafluorethylendioxy, Difluormethylthio, Trifluormethylthio, Trifluormethylsulfinyl, Trifluormethylsulfonyl, Hydroxy, Mercapto, Nitro, Cyano, Amino substituiertes Phenyl, Thienyl, Pyrimidyl oder Pyridyl steht
oder für Ar² steht, wobei Ar² für Ar¹ steht, welches zusätzlich durch Phenyl oder Phenoxy substituiert ist, wobei diese Substituenten ihrerseits gegebenenfalls einfach bis zweifach durch Fluor, Chlor, Brom, Methyl, Ethyl, Isopropyl, n-, s-, i- oder t-Butyl, Methoxy, Ethoxy, Isopropoxy, n-, s-, i- oder t-Butoxy, Trifluormethyl, Trifluormethoxy, Nitro oder Cyano substituiert sind, und
X für CN steht,
Z für Wasserstoff oder Methyl steht,
m für 1 bis 3 steht, und
n für 0 steht.

5. Verfahren zur Herstellung von Verbindungen gemäß einem der Ansprüche 1 bis 4, **dadurch gekennzeichnet, dass** man
(A) für den Fall, dass K für Sauerstoff steht und Z für Wasserstoff steht,
Verbindungen der Formel (II), 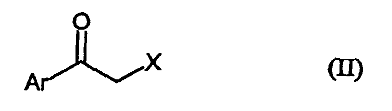 in welcher
Ar und X die in einem der Ansprüche 1 bis 5 angegebenen Bedeutungen haben,
mit Verbindungen der Formel (III), 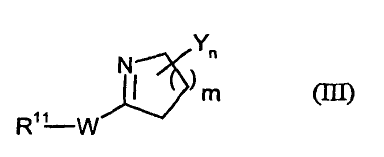 in welcher
Y, m, n die in einem der Ansprüche 1 bis 5 angegebenen Bedeutungen haben,
und
W für O oder S(O)_{g}, wobei g für 0 oder 2 steht, und
R¹¹ für Alkyl oder Benzyl steht,
gegebenenfalls in Gegenwart eines Verdünnungsmittels und gegebenenfalls in Gegenwart einer Base oder einer Metallverbindung der Formel (IIIa),
Me(V)₂ (IIIa)
in welcher
Me für ein zweiwertiges Übergangsmetallatom steht, und
V für einen Chelatliganden steht, umsetzt,
oder dass man
(B) für den Fall, dass K für Sauerstoff steht und Z für Wasserstoff steht,
Verbindungen der Formel (IV), 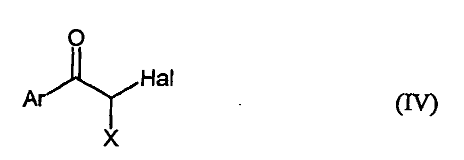 in welcher
Ar und X die in einem der Ansprüche 1 bis 5 angegebene Bedeutung haben, und
Hal für Halogen steht,
mit Verbindungen der Formel (V), 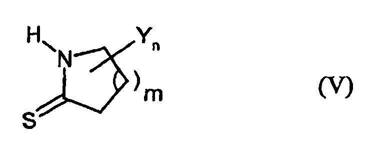
in welcher
Y, m und n die in einem der Ansprüche 1 bis 5 angegebenen Bedeutungen haben,
gegebenenfalls in Gegenwart eines Verdünnungsmittels zu Verbindungen der Formel (VI) umsetzt, 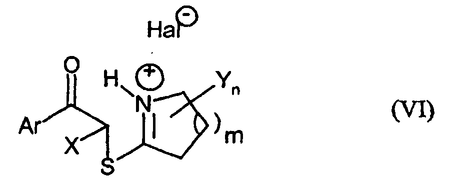 in welcher
Ar, X, Y, m und n die in einem der Ansprüche 1 bis 5 angegebenen Bedeutungen haben,
die gegebenenfalls in Gegenwart einer Base und gegebenenfalls in Gegenwart einer dreiwertigen Phosphorverbindung unter Abspaltung von Schwefel und Halogenwasserstoff zu Verbindungen der Formel (I),
in welcher
Ar, X, Y, m und n die in einem der Ansprüche 1 bis 5 angegebenen Bedeutungen haben und
Z für Wasserstoff steht,
weiterreagieren,
oder dass man
(C) für den Fall, dass K für Sauerstoff steht und Z nicht für Wasserstoff steht,
Verbindungen der Formel (VII), 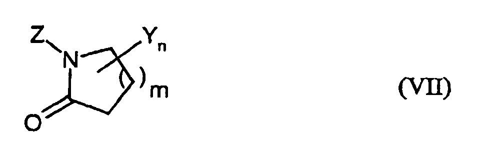 in welcher
Y, m und n die in einem der Ansprüche 1 bis 5 angegebenen Bedeutungen haben,
mit Halogenierungsmitteln gegebenenfalls in Gegenwart eines Verdünnungsmittels zu Verbindungen der Formel (VIII), 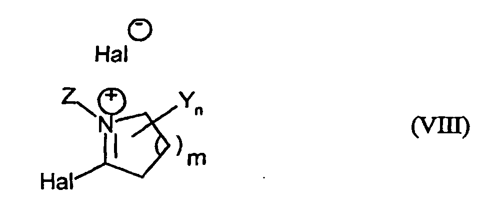 in welcher
Y, Z, m und n die in einem der Ansprüche 1 bis 5 angegebenen Bedeutungen haben,
und Z nicht für Wasserstoff steht, und
Hal für Halogen steht, umsetzt,
die dann mit Verbindungen der Formel (II), 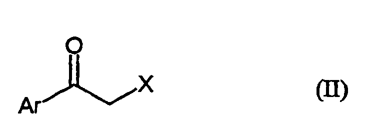 in welcher
Ar, X die in einem der Ansprüche 1 bis 5 angegebenen Bedeutungen haben,
gegebenenfalls in Gegenwart eines Verdünnungsmittels und gegebenenfalls in Gegenwart eines Säureakzeptors umgesetzt werden,
oder dass man
(D) für den Fall, dass K für Sauerstoff steht und Z nicht für Wasserstoff steht,
Verbindungen der Formel (I-a), 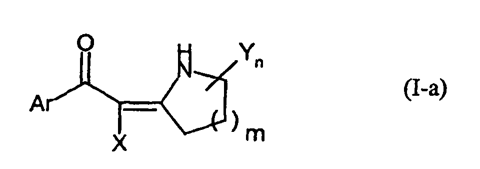 in welcher
Ar, X, Y, m und n die in einem der Ansprüche 1 bis 5 angegebene Bedeutung haben mit
Alkylierungsmittel, Acylierungsmitteln, Sulfonylierungsmitteln oder Kondensationsmitteln der Formel (IX)
Z-G (IX),
in welcher
G für eine Fluchtgruppe wie Halogen, Sulfonat oder Alkoxy stehen,
gegebenenfalls in Gegenwart eines Lösungsmittels und gegebenenfalls in Gegenwart einer Base umsetzt,
oder dass man
(E) für den Fall, dass Ar für Ar² gemäß einem der Ansprüche 1 bis 5 steht,
Verbindungen der Formel (I¹) 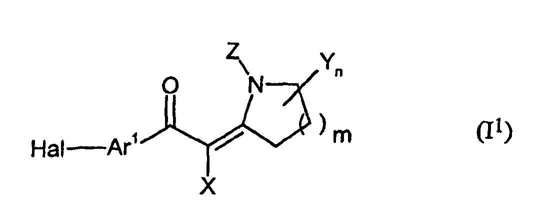 in welcher
Ar¹, X, Y, Z, m und n die in einem der Ansprüche 1 bis 5 angegebene Bedeutung haben,und
Hal für Halogen steht,
mit Boronsäuren der Formel (X)
Ar^{2'}―B(OH)₂ (X),
in welcher
Ar² wie in einem der Ansprüche 2 bis 5 definiert ist,
Ar^{2'} für die Substituenten steht, die in Anspruch 1 als zusätzliche Substituenten für Ar¹ genannt wurden
in Gegenwart eines Lösungsmittels, gegebenenfalls in Gegenwart einer Base und/oder eines Edelmetallkomplexes umsetzt,
oder dass man
(F) Verbindungen der Formel (I) 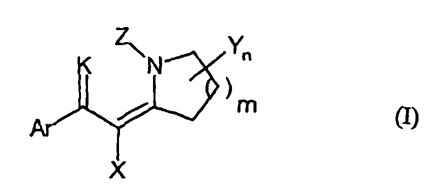 in welcher
Ar, X, Y, Z, m und n in einem der Anprüche 1 bis 5 angegebene Bedeutung haben, und K für Sauerstoff steht,
in Gegenwart eines Schwefelungsreagenz in Gegenwart eines Lösungsmittels umsetzt.

6. Herbizide, akarizide und/oder insektizide Mittel, **gekennzeichnet durch** einen Gehalt an mindestens einer Verbindung gemäß einem der Ansprüche 1 bis 5.

7. Verwendung von Verbindungen gemäß einem der Ansprüche 1 bis 5 zur Bekämpfung von unerwünschtem Pflanzenwuchs und/oder tierischen Schädlingen.

8. Verbindungen der Formel (V), 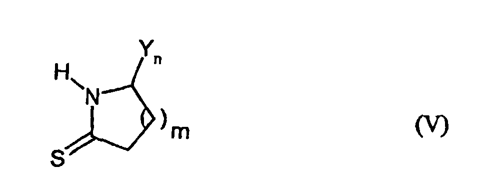 wobei, Yₙ für gegebenenfalls substituiertes Phenyl steht und m = 1 oder 2 ist,
ausgenommen die Verbindungen 5-Phenyl-pyrrolidin-2-thion und 5-(3,4-Dimethoxyphenyl)-pyrrolidin-2-thion.

9. Verbindungen der Formel (II-1-b), 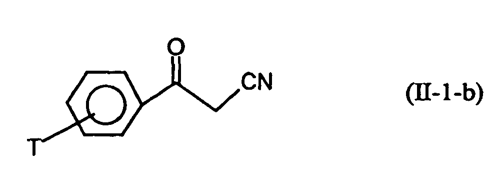 wobei
T die in der folgenden Tabelle angegebene Bedeutung hat:

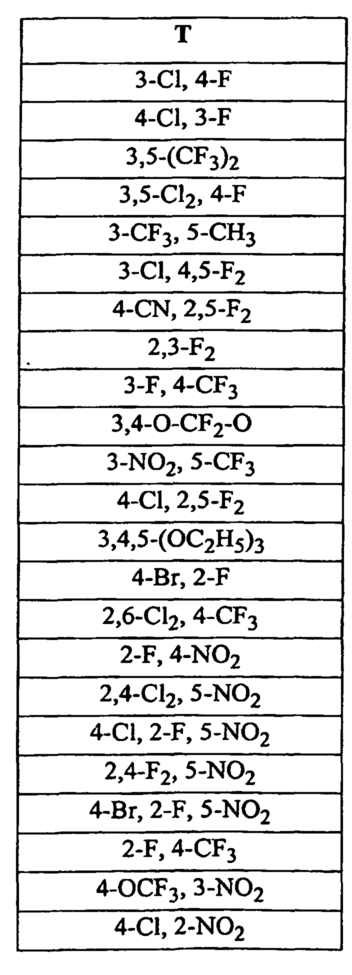

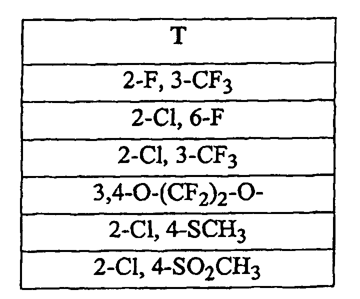

10. Verbindungen der Formel (II-2-b), 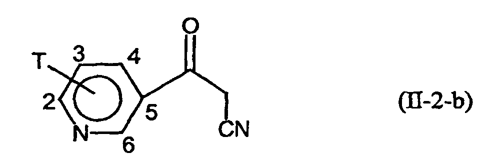 wobei T die in der folgenden Tabelle angegebene Bedeutung hat:

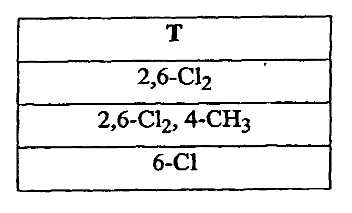

## Claims

1. Compounds of the general formula (I) 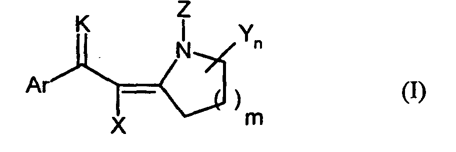 in which
Ar represents Ar¹, where Ar¹ represents phenyl, naphthyl or mono- or bicyclic hetaryl having five to ten ring atoms, each of which radicals is (but in the case of phenyl and naphthyl not unsubstituted) optionally mono- to pentasubstituted by halogen, C₁-C₈-alkyl, C₂-C₈-alkenyl, C₂-C₈-alkinyl, C₁-C₈-alkoxy, C₂-C₈-alkenyloxy, C₃-C₈-alkinyloxy, C₁-C₈-alkylthio, C₁-C₆-alkylsulfinyl, C₁-C₆-alkylsulfonyl, C₁-C₆-halogenoalkyl, C₁-C₆-halogeno-alkoxy, C₂-C₈-halogenoalkenyloxy, C₁-C₂-alkylidenediyl-dioxy, C₁-C₂-halogenoalkylidenediyl-dioxy, halogeno-C₁-C₄-alkylthio, halogeno-C₁-C₄-alkylsulfinyl, halogeno-C₁-C₄-alkylsulfonyl, hydroxyl, mercapto, nitro, cyano, amino, or by the groups 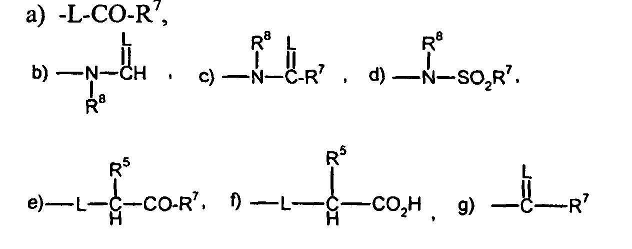 or represents Ar², where Ar² represents Ar¹ which is additionally substituted by phenyl, naphthyl, five- or six-membered hetaryl, phenyl-C₁-C₄-alkyl, phenoxy, phenyl-S(O)_{g}-, five- or six-membered hetaryloxy or hetaryl-S(O)_{g}, where these substituents for their part are in each case optionally mono- to tetrasubstituted by halogen, C₁-C₆-alkyl, C₁-C₆-alkoxy, C₁-C₄-halogenoalkyl, C₁-C₄-halogenoalkoxy, nitro or cyano, and
K represents oxygen or sulphur,
L represents oxygen or sulphur,
X represents CN, 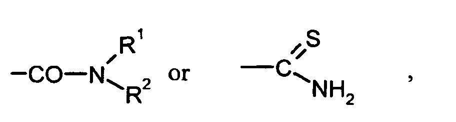
Y represents halogen, C₁-C₆-alkyl, C₁-C₄-halogenoalkyl, C₁-C₆-alkoxy, represents phenyl, phenyl-C₁-C₄-alkyl, five- or six-membered hetaryl or five- or six-membered hetaryl-C₁-C₄-alkyl, each of which is optionally mono- to tetrasubstituted by halogen, C₁-C₆-alkyl, C₁-C₆-alkoxy, C₁-C₄-halogenoalkyl, C₁-C₄-halogenoalkoxy, cyano or nitro, or represents the groups
―CO₂R¹
or 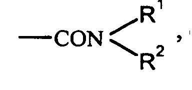 or
two adjacent Yₙ represent a 5- to 8-membered, saturated or unsaturated cycle which may be interrupted by 1 to 3 heteroatoms from the group consisting of N, O, S and which may optionally be mono- to trisubstituted by halogen, C₁-C₄-alkyl, C₁-C₄-alkoxy, C₁-C₄-halogenoalkyl, C₁-C₄-halogenoalkoxy, cyano or nitro, and
Z represents hydrogen, represents in each case optionally fluorine- and/or chlorine-substituted C₁-C₈-alkyl, cyano-C₁-C₆-alkyl, C₃-C₈-alkenyl, C₃-C₈-alkinyl, C₃-C₈-cycloalkyl, C₃-C₈-cycloalkyl-C₁-C₂-alkyl, C₁-C₄-alkoxy-C₁-C₄-alkyl, C₁-C₄-halogenoalkoxy-C₁-C₄-alkyl, represents phenoxy-C₁-C₄-alkyl, phenyl-C₁-C₄-alkyloxy-C₁-C₄-alkyl, phenylthio-C₁-C₄-alkyl, phenyl-C₁-C₄-alkylthio-C₁-C₄-alkyl, phenyl, phenyl-C₁-C₄-alkyl, five- or six-membered hetaryl, five- or six-membered hetaryl-C₁-C₄-alkyl, each of which is optionally mono- to tetrasubstituted by halogen, C₁-C₆-alkyl, C₁-C₆-alkoxy, C₁-C₄-halogenoalkyl, C₁-C₄-halogeno-alkoxy, nitro or cyano, or represents the groups
―CO₂R¹ ; 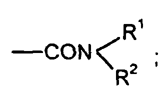
―SO₂R¹;
―COR¹; 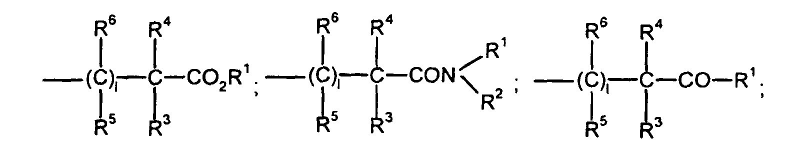
or cyano,
g represents 0 to 2,
l represents 0 to 2,
R¹ represents hydrogen (but not for the radicals -CO₂R¹ and -SO₂R¹), represents in each case fluorine- and/or chlorine-substituted C₁-C₁₀-alkyl, C₂-C₁₀-alkenyl, C₃-C₆-alkinyl, represents in each case optionally fluorine-, chlorine-, C₁-C₄-alkyl-, C₁-C₄-alkoxy-substituted C₃-C₈-cycloalkyl or C₅-C₈-cycloalkenyl in which optionally one methylene group may be interrupted by oxygen or sulphur, or represents phenyl, pyridyl, thienyl, pyrimidyl, thiazolyl, phenyl-C₁-C₄-alkyl, pyridyl-C₁-C₂-alkyl, thiazolyl-C₁-C₂-alkyl, each of which is optionally mono- to tetrasubstituted by halogen, C₁-C₆-alkyl, C₁-C₆-alkoxy, C₁-C₄-halogenoalkyl, C₁-C₄-halogenoalkoxy, cyano or nitro,
R² represents hydrogen, represents in each case optionally fluorine- and/or chlorine-substituted C₁-C₆-alkyl, C₃-C₆-alkenyl, C₃-C₆-alkinyl, C₁-C₆-alkoxy, C₃-C₆-alkenyloxy or represents phenyl, phenyl-C₁-C₄-alkyl or phenyl-C₁-C₄-alkoxy, each of which is optionally mono- to tetrasubstituted by halogen, C₁-C₆-alkyl, C₁-C₆-alkoxy, C₁-C₄-halogenoalkyl, C₁-C₄-halogenoalkoxy, cyano or nitro, or
R¹, R² together with the nitrogen atom to which they are attached represent an optionally C₁-C₄-alkyl-substituted five- to eight-membered cycle in which optionally one methylene group may be replaced by oxygen or sulphur,
R³ represents hydrogen, optionally halogen-substituted C₁-C₆-alkyl or represents phenyl or phenyl-C₁-C₂-alkyl each of which is optionally mono- to tetrasubstituted by halogen, C₁-C₆-alkyl, C₁-C₆-alkoxy, C₁-C₄-halogenoalkyl, C₁-C₄-halogenoalkoxy, cyano or nitro,
R⁴ represents hydrogen or C₁-C₆-alkyl,
R⁵, R⁶ independently of one another each represent hydrogen or optionally fluorine- and/or chlorine-substituted C₁-C₄-alkyl,
R⁷ represents in each case optionally fluorine- and/or chlorine-substituted C₁-C₁₀-alkyl, C₂-C₁₀-alkenyl, C₃-C₁₀-alkinyl, C₁-C₁₀-alkoxy, C₁-C₄-alkoxy-C₁-C₄-alkyl, C₁-C₄-alkylthio-C₁-C₄-alkyl, in each case optionally fluorine- and/or chlorine-, C₁-C₄-alkyl-, C₁-C₄-alkoxy-substituted C₃-C₈-cycloalkyl or C₃-C₈-cycloalkyloxy in which optionally one methylene group may be replaced by oxygen or sulphur, represents phenyl, phenoxy, benzyloxy, five- or six-membered hetaryl or phenyl-C₁-C₄-alkyl, each of which is optionally mono- to tetrasubstituted by halogen, C₁-C₆-alkyl, C₁-C₆-alkoxy, C₁-C₄-halogeno-alkyl, C₁-C₄-halogenoalkoxy, cyano or nitro, or, in the case of the radicals a) and c) mentioned under Ar, also represents a group 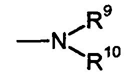 or, for the radical g), also represents hydroxyl,
R⁸ represents hydrogen or C₁-C₄-alkyl,
R⁹ represents hydrogen, in each case optionally fluorine- and/or chlorine-substituted C₁-C₁₀-alkyl, C₃-C₈-alkenyl, C₃-C₈-alkinyl, C₁-C₁₀-alkoxy, C₃-C₈-alkenyl-oxy, optionally fluorine-, chlorine-, C₁-C₄-alkyl- or C₁-C₄-alkoxy-substituted C₃-C₈-cycloalkyl in which optionally one methylene group may be replaced by oxygen or sulphur, represents phenyl, phenyl-C₁-C₄-alkyl or phenyl-C₁-C₂-alkoxy, each of which is optionally mono- to tetrasubstituted by halogen, C₁-C₆-alkyl, C₁-C₆-alkoxy, C₁-C₄-halogenoalkyl, C₁-C₄-halogenoalkoxy, cyano or nitro,
R¹⁰ represents hydrogen, C₁-C₆-alkyl or C₃-C₆-alkenyl, or
R⁹, R¹⁰ together with the nitrogen atom to which they are attached represent an optionally C₁-C₄-alkyl-substituted five- to eight-membered cycle in which optionally one methylene group may be replaced by oxygen or sulphur, and
m represents 1 to 3, and
n depending on m, represents 0 to 3,
with the exception of compounds having the following formula 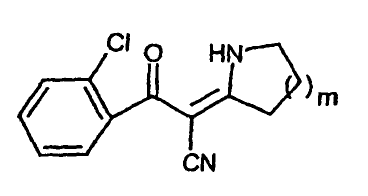 m = 1, 2, 3.

2. Compounds according to Claim 1, **characterized in that**
K represents oxygen or sulphur,
Ar represents Ar¹, where Ar¹ represents phenyl, naphthyl, quinolinyl, thienyl, pyrimidyl, furanyl, thiazolyl, benzothiazolyl, oxazolyl, pyrazolyl or pyridyl, each of which is (but in the case of phenyl and naphthyl not unsubstituted) optionally mono- to tri-substituted by fluorine, chlorine, bromine, C₁-C₆-alkyl, C₂-C₆-alkenyl, C₂-C₆-alkinyl, C₁-C₆-alkoxy, C₃-C₆-alkenyloxy, C₃-C₆-alkinyloxy, C₁-C₆-alkylthio, C₁-C₄-alkylsulfinyl, C₁-C₄-alkylsulfonyl, C₁-C₄-halogenoalkyl, C₁-C₄-halogeno-alkoxy, C₂-C₄-halogenoalkenyloxy, C₁-C₂-alkylidenediyl-dioxy, C₁-C₂-halogenoalkylidenediyl-dioxy, halogeno-C₁-C₂-alkylthio, halogeno-C₁-C₂-alkylsulfinyl, halogeno-C₁-C₂-alkylsulfonyl, hydroxyl, mercapto, nitro, cyano, amino or by one of the following groups 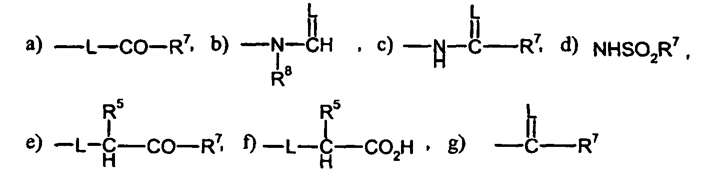 or represents Ar², where Ar² represents Ar¹ which is additionally substituted by phenyl, pyridyl, pyrimidyl, thienyl, furanyl, thiazolyl, tetrazolyl, triazolyl, benzyl, phenoxy, phenyl-S(O)_{g}-, pyridyloxy, pyrimidyloxy, thiazolyloxy, pyridyl-S(O)_{g}-, pyrimidyl-S(O)_{g}- or thiazolyl-S(O)_{g}-, where these substituents for their part are optionally mono- to tri-substituted by fluorine, chlorine, bromine, C₁-C₄-alkyl, C₂-C₄-alkenyl, C₁-C₄-alkoxy, C₁-C₂-halogenoalkyl, C₁-C₂-halogenoalkoxy, nitro or cyano, where g represents 0 to 2, and
L represents oxygen or sulphur,
X represents CN, 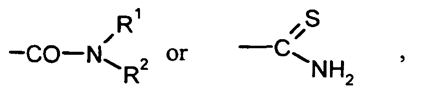
Y represents fluorine, C₁-C₄-alkyl, C₁-C₂-halogenoalkyl, C₁-C₄-alkoxy, represents phenyl, phenyl-C₁-C₂-alkyl, thiazolylmethyl, pyridylmethyl, each of which is optionally mono- to trisubstituted by fluorine, chlorine, bromine, C₁-C₄-alkyl, C₁-C₄-alkoxy, C₁-C₂-halogenoalkyl, C₁-C₂-halogenoalkoxy, cyano or nitro, or represents the groups
―CO₂R¹
or 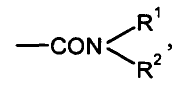 or
two adjacent Yₙ furthermore represent a 5- to 6-membered, saturated or unsaturated cycle which may be interrupted by a heteroatom from the group consisting of N, O, S and which may optionally be mono- to disubstituted by fluorine, chlorine, bromine, methyl, t-butyl, methoxy, trifluoromethyl, trifluoromethoxy, cyano or nitro, and
Z represents hydrogen, represents in each case optionally fluorine- and/or chlorine-substituted C₁-C₆-alkyl, cyano-C₁-C₃-alkyl, C₃-C₆-alkenyl, C₃-C₈-alkinyl, C₃-C₆-cycloalkyl, C₃-C₆-cycloalkyl-C₁-C₂-alkyl, C₁-C₄-alkoxy-C₁-C₂-alkyl, C₁-C₄-halogeno-C₁-C₂-alkyl, represents phenoxy-C₁-C₂-alkyl, phenyl-C₁-C₂-alkyloxy-C₁-C₂-alkyl, phenylthio-C₁-C₂-alkyl, phenyl-C₁-C₂-alkylthio-C₁-C₂-alkyl, phenyl-C₁-C₂-alkyl, phenyl, pyridyl-C₁-C₂-alkyl, thiazolyl-C₁-C₂-alkyl, each of which is optionally mono- to trisubstituted by fluorine, chlorine, bromine, C₁-C₄-alkyl, C₁-C₄-alkoxy, C₁-C₂-halogenoalkyl, C₁-C₂-halogenoalkoxy, nitro or cyano, or represents the groups
―CO₂R¹ ; 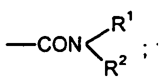
―SO₂R¹;
―COR¹; 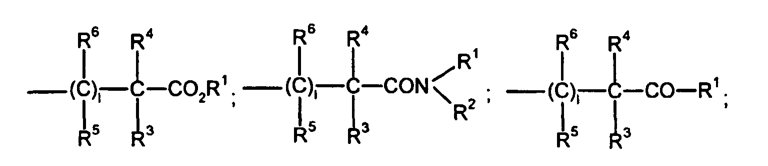
or cyano,
l represents 0 to 1,
R¹ represents hydrogen (but not for the radicals -CO₂R¹ and -SO₂R¹), represents in each case optionally fluorine- and/or chlorine-substituted C₁-C₆-alkyl, C₂-C₆-alkenyl, C₃-C₄-alkinyl, represents optionally fluorine-, chlorine-, C₁-C₂-alkyl-, C₁-C₂-alkoxy-substituted C₃-C₆-cycloalkyl or represents phenyl or benzyl, each of which is optionally mono- to disubstituted by fluorine, chlorine, bromine, C₁-C₄-alkyl, C₁-C₄-alkoxy, C₁-C₂-halogenoalkyl, C₁-C₂-halogenoalkoxy, cyano or nitro,
R² represents hydrogen, represents in each case optionally fluorine- and/or chlorine-substituted C₁-C₄-alkyl, C₃-C₄-alkenyl, C₃-C₄-alkinyl, C₁-C₄-alkoxy, C₃-C₄-alkenyloxy or represents phenyl, benzyl or benzyloxy, each of which is optionally mono- to disubstituted by fluorine, chlorine, bromine, C₁-C₄-alkyl, C₁-C₄-alkoxy, C₁-C₂-halogenoalkyl, C₁-C₂-halogenoalkoxy, cyano or nitro, or
R¹, R² together with the nitrogen atom to which they are attached represent an optionally C₁-C₂-alkyl-substituted five- or six-membered cycle in which optionally one methylene group may be replaced by oxygen,
R³ represents hydrogen, represents C₁-C₄-alkyl or represents phenyl or benzyl, each of which is optionally mono- to disubstituted by fluorine, chlorine, bromine, C₁-C₄-alkyl, C₁-C₄-alkoxy, trifluoromethyl, difluoromethoxy, trifluoromethoxy, cyano or nitro,
R⁴, R⁵, R⁶ each represents hydrogen, methyl or ethyl,
R⁷ represents in each case optionally fluorine- and/or chlorine-substituted C₁-C₆-alkyl, C₂-C₆-alkenyl, C₃-C₆-alkinyl, C₁-C₆-alkoxy, C₁-C₄-alkoxy-C₁-C₂-alkyl, C₁-C₄-alkylthio-C₁-C₂-alkyl, in each case optionally fluorine- and/or chlorine-, C₁-C₂-alkyl-, C₁-C₂-alkoxy-substituted C₃-C₆-cycloalkyl in which optionally one methylene group may be replaced by oxygen, represents phenyl, phenoxy, benzyloxy, thienyl, furanyl, pyridyl, pyrimidyl, thiazolyl, pyrazolyl or phenyl-C₁-C₂-alkyl, each of which is optionally mono- to disubstituted by fluorine, chlorine, bromine, C₁-C₄-alkyl, C₁-C₄-alkoxy, trifluoromethyl, difluoromethoxy, trifluoromethoxy, cyano or nitro, or, in the case of the radicals a) and c) mentioned under Ar, also represents a group 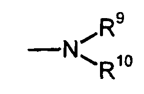 or, for the radical g), also represents hydroxyl,
R⁸ represents hydrogen,
R⁹ represents hydrogen, in each case optionally fluorine- and/or chlorine-substituted C₁-C₆-alkyl, C₃-C₆-alkenyl, C₃-C₆-alkinyl, C₁-C₆-alkoxy, C₃-C₆-cycloalkyl, in which optionally one methylene group may be replaced by oxygen, represents phenyl or phenyl-C₁-C₂-alkyl, each of which is optionally mono- to disubstituted by fluorine, chlorine, bromine, C₁-C₄-alkyl, C₁-C₄-alkoxy, trifluoromethyl, difluoromethoxy, trifluoromethoxy, cyano or nitro,
R¹⁰ represents hydrogen or C₁-C₄-alkyl, or
R⁹, R¹⁰ together with the nitrogen atom to which they are attached represent an optionally C₁-C₂-alkyl-substituted five- to six-membered cycle in which optionally one methylene group may be replaced by oxygen, and
m represents 0 to 1, and
n depending on m, represents 0 to 2.

3. Compounds according to Claim 1, **characterized in that**
K represents oxygen or sulphur,
Ar represents Ar¹, where Ar¹ represents phenyl, thienyl, pyrimidyl, furanyl or pyridyl, each of which is (but in the case of phenyl and naphthyl not unsubstituted) optionally mono- to trisubstituted by fluorine, chlorine, bromine, methyl, ethyl, propyl, i-propyl, s-, n-, i- or t-butyl, methoxy, ethoxy, propoxy, i-propoxy, s-, n-, i- or t-butoxy, allyloxy, methallyloxy, 2-butenyloxy, propargyloxy, 2-butinyloxy, methylthio, ethylthio, methylsulfinyl, ethylsulfinyl, methylsulfonyl, ethylsulfonyl, trifluoromethyl, difluoromethoxy, trifluoromethoxy, methylenedioxy, difluoromethylenedioxy, tetrafluoroethylenedioxy, difluoromethylthio, trifluoromethylthio, trifluoromethylsulfinyl, trifluoromethylsulfonyl, hydroxyl, mercapto, nitro, cyano, amino or by one of the following groups 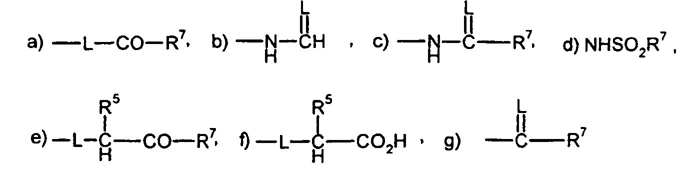 or represents Ar², where Ar² represents Ar¹ which is additionally substituted by phenyl, pyridyl, thienyl, tetrazolyl, triazolyl or phenoxy, where these substituents for their part are optionally mono- to disubstituted by fluorine, chlorine, bromine, methyl, ethyl, n- or i-propyl, s-, n-, i- or t-butyl, methoxy, ethoxy, i-propoxy, s-, n- or t-butoxy, trifluoromethyl, trifluoromethoxy, nitro or cyano, and
L represents oxygen or sulphur,
X represents
―CN,
―CO―NH₂, 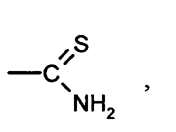
Y represents methyl, phenyl which is optionally mono- to trisubstituted by fluorine, chlorine, bromine, methyl, methoxy, trifluoromethyl or trifluoromethoxy, or represents the group
―CO₂R¹,
or two adjacent Yₙ represent a six-membered unsaturated cycle which may optionally be monosubstituted by fluorine, chlorine, bromine, methyl, methoxy, trifluoromethyl, trifluoromethoxy, cyano or nitro, and
Z represents hydrogen, methyl, ethyl, allyl, propargyl, cyclopropyl, cyclopentyl, cyclohexyl, cyclopropylmethyl, cyclopentylmethyl, cyclohexylmethyl, methoxymethyl, ethoxymethyl, represents phenyl, benzyl, pyridylmethyl, thiazolylmethyl, each of which is optionally mono- to disubstituted by fluorine, chlorine, bromine, methyl, methoxy, trifluoromethyl or trifluoromethoxy, cyano or nitro, or represents the groups
―CO₂R¹, 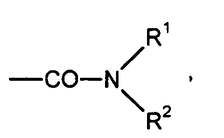
―SO₂R¹ ,
―CO―R¹ , 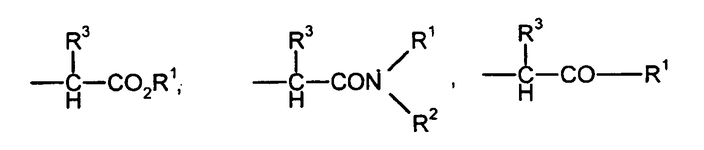
or cyano,
R¹ represents hydrogen (but not for the radicals -CO₂R¹ and -SO₂R¹), methyl, ethyl, propyl, isopropyl, n-, s-, i- or t-butyl, allyl, propargyl, cyclopropyl, cyclopentyl, cyclohexyl, represents phenyl or benzyl, each of which is optionally mono- to disubstituted by fluorine, chlorine, bromine, methyl, methoxy, trifluoromethyl, trifluoromethoxy, cyano or nitro,
R² represents hydrogen, methyl, ethyl, propyl, isopropyl, allyl, propargyl, methoxy, ethoxy, allyloxy or represents benzyloxy which is optionally mono- to disubstituted by fluorine, chlorine, bromine, methyl, methoxy, trifluoromethyl, trifluoromethoxy, cyano or nitro, or
R¹, R² together with the nitrogen atom to which they are attached represent a pyrrolidine, piperidine, thiazine or morpholine radical,
R³, R⁵ each represent hydrogen, methyl or ethyl,
R⁷ represents methyl, ethyl, propyl, isopropyl, n-, s-, i- or t-butyl, vinyl, trifluoromethyl, methoxy, ethoxy, propoxy, isopropoxy, n-, s-, i- or t-butyloxy, cyclopropyl, cyclopentyl, cyclohexyl, cyclopentyloxy, cyclohexyloxy, represents phenyl, pyridyl or benzyl, each of which is optionally mono- to disubstituted by fluorine, chlorine, bromine, methyl, n-, s-, i- or t-butyl, methoxy, trifluoromethyl, trifluoromethoxy, cyano or nitro, or, in the case of the radicals a) and c) mentioned under Ar, also represents a group 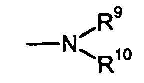 or, for the radical g), also represents hydroxyl,
R⁹ represents hydrogen, methyl, ethyl, propyl, isopropyl, n-, s-, i- or t-butyl, cyclopropyl, cyclopentyl, cyclohexyl, represents phenyl which is optionally mono- to disubstituted by fluorine, chlorine, bromine, methyl, methoxy, trifluoromethyl, trifluoromethoxy, cyano or nitro,
R¹⁰ represents hydrogen, methyl or ethyl, or
R⁹, R¹⁰ together with the nitrogen atom to which they are attached represent a pyrrolidine, piperidine or morpholine radical, and
m represents 1 to 3, and
n depending on m, represents 0 to 1.

4. Compounds according to Claim 1, **characterized in that**
K represents oxygen or sulphur,
Ar represents Ar¹, where Ar¹ represents phenyl, thienyl, pyrimidyl or pyridyl, each of which is optionally mono- to trisubstituted by fluorine, chlorine, bromine, methyl, ethyl, propyl, isopropyl, n-, s-, i-or t-butyl, methoxy, ethoxy, propoxy, isopropoxy, tert-butoxy, allyloxy, methallyloxy, 2-butenyloxy, propargyloxy, 2-butinyloxy, methylthio, ethylthio, methylsulphinyl, ethylsulphinyl, methylsulphonyl, ethylsulphonyl, trifluoromethyl, difluoromethoxy, trifluoromethoxy, methylenedioxy, difluoromethylenedioxy, tetrafluoroethylenedioxy, difluoromethylthio, trifluoromethylthio, trifluoromethylsulphinyl, trifluoromethylsulphonyl, hydroxyl, mercapto, nitro, cyano, amino,
or represents Ar², where Ar² represents Ar¹ which is additionally substituted by phenyl or phenoxy, where these substituents for their part are optionally mono- to disubstituted by fluorine, chlorine, bromine, methyl, ethyl, isopropyl, n-, s-, i- or t-butyl, methoxy, ethoxy, isopropoxy, n-, s-, i- or t-butoxy, trifluoromethyl, trifluoromethoxy, nitro or cyano, and
X represents CN,
Z represents hydrogen or methyl,
m represents 1 to 3, and
n represents 0.

5. Process for preparing compounds according to any of Claims 1 to 4, **characterized in that**
(A) in the case where K represents oxygen and Z represents hydrogen,
compounds of the formula (II) 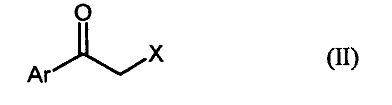 in which
Ar and X are each as defined in any of Claims 1 to 4,
are reacted with compounds of the formula (III) 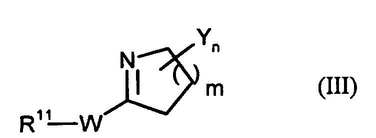 in which
Y, m, n are each as defined in any of Claims 1 to 4,
and
W represents O or S(O)_{g}, where g represents 0 or 2, and
R¹¹ represents alkyl or benzyl,
if appropriate in the presence of a diluent and if appropriate in the presence of a base or a metal compound of the formula (IIIa)
Me(V)₂ (IIIa)
in which
Me represents a divalent transition metal atom and
V represents a chelate ligand,
or **in that**
(B) in the case where K represents oxygen and Z represents hydrogen,
compounds of the formula (IV) 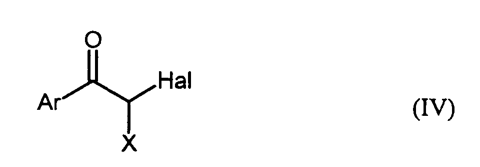 in which
Ar and X are each as defined in any of Claims 1 to 4 and
Hal represents halogen,
are reacted with compounds of the formula (V) 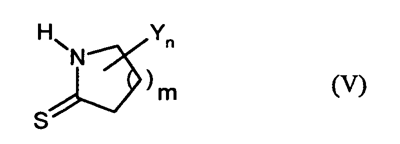
in which
Y, m and n are each as defined in any of Claims 1 to 4,
if appropriate in the presence of a diluent to give compounds of the formula (VI) 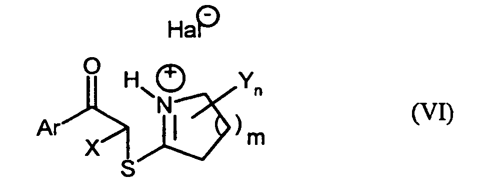 in which
Ar, X, Y, m and n are each as defined in any of Claims 1 to 4,
which are reacted further, if appropriate in the presence of a base and if appropriate in the presence of a trivalent phosphorus compound, with elimination of sulphur and hydrogen halide, to give compounds of the formula (I)
in which
Ar, X, Y, m and n are each as defined in any of Claims 1 to 4 and
Z represents hydrogen,
or **in that**
(C) in the case where K represents oxygen and Z does not represent hydrogen
compounds of the formula (VII) 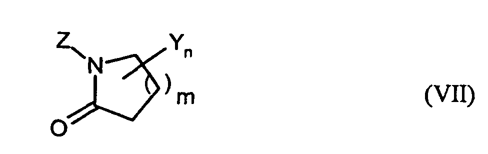 in which
Y, m and n are each as defined in any of Claims 1 to 4,
are reacted with halogenating agents, if appropriate in the presence of a diluent, to give compounds of the formula (VIII) 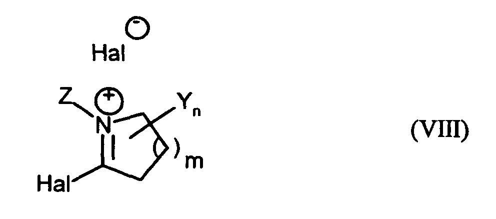 in which
Y, Z, m and n are each as defined in any of Claims 1 to 4
and Z does not represent hydrogen, and
Hal represents halogen,
which are then reacted with compounds of the formula (II) 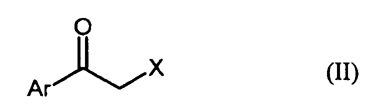 in which
Ar, X are each as defined in any of Claims 1 to 4,
if appropriate in the presence of a diluent and if appropriate in the presence of an acid acceptor,
or **in that**
(D) in the case where K represents oxygen and Z does not represent hydrogen,
compounds of the formula (I-a) 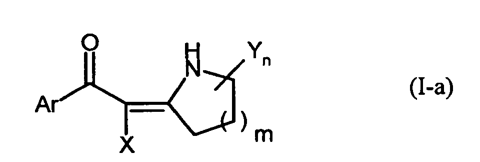 in which
Ar, X, Y, m and n are each as defined in any of Claims 1 to 4 are reacted with
alkylating agents, acylating agents, sulphonylating agents or condensing agents of the formula (IX)
Z-G (IX),
in which
G represents a leaving group such as halogen, sulphonate or alkoxy,
if appropriate in the presence of a solvent and if appropriate in the presence of a base,
or **in that**
(E) in the case where Ar represents Ar² according to any of Claims 1 to 4,
compounds of the formula (I¹) 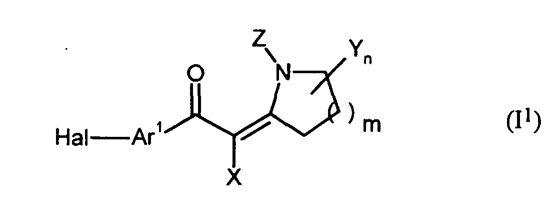 in which
Ar¹, X, Y, Z, m and n are each as defined in any of Claims 1 to 4 and
Hal represents halogen,
are reacted with boronic acids of the formula (X)
Ar^{2'}―B(OH)₂ (X),
in which
Ar² is as defined in any of Claims 2 to 4,
Ar^{2'} represents the substituents which were mentioned in Claim 1 as additional substituents for Ar¹,
in the presence of a solvent, if appropriate in the presence of a base and/or a noble metal complex,
or **in that**
(F) compounds of the formula (I) 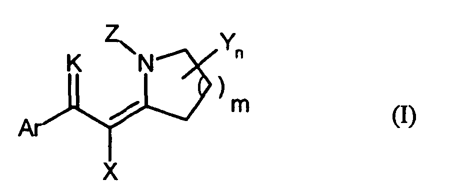 in which
Ar, X, Y, Z, m and n are each as defined in any of Claims 1 to 4, and K represents oxygen,
are reacted in the presence of a sulphurizing agent in the presnce of a solvent.

6. Herbicidal, acaricidal and/or insecticidal compositions, **characterized in that** they comprise at least one compound according to any of Claims 1 to 4.

7. Use of compounds according to any of Claims 1 to 4 for controlling undesirable vegetation and/or animal pests.

8. Compounds of the formula (V) 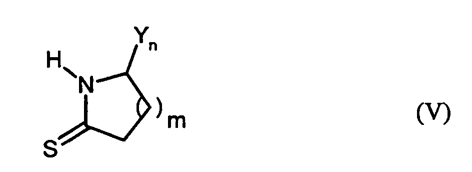 in which Yₙ represents optionally substituted phenyl and m = 1 or 2,
except for the compounds 5-phenyl-pyrrolidine-2-thione and 5-(3,4-dimethoxyphenyl)-pyrrolidine-2-thione.

9. Compounds of the formula (II-1-b) 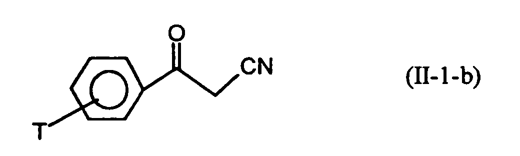 where
T is as defined in the table below:

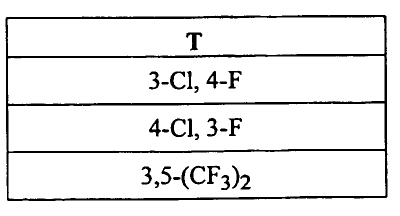

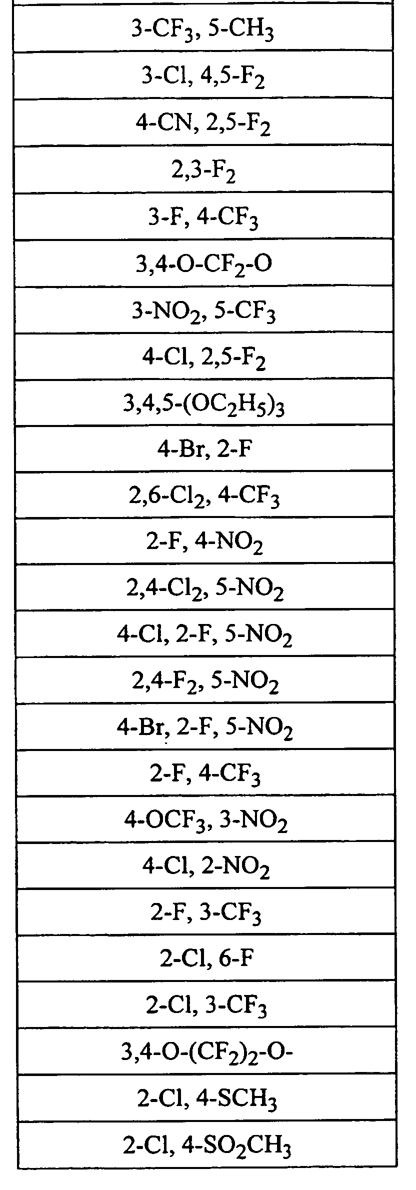

10. Compounds of the formula (II-2-b) 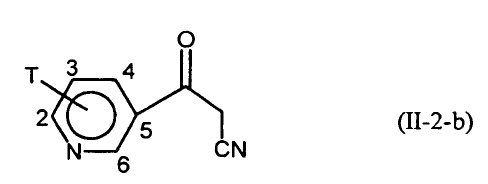 where T is as defined in the table below:

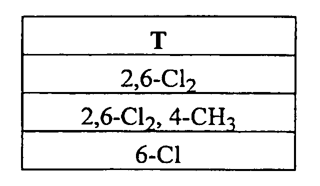

## Revendications

1. Composés de formule générale (I) 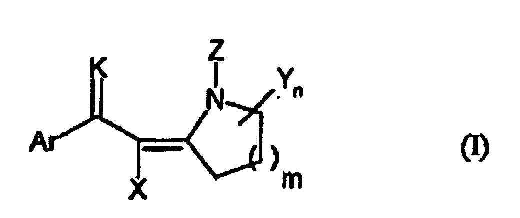 dans laquelle
Ar représente Ar¹, auquel cas Ar¹ représente un reste phényle, naphtyle ou hétaryle monocyclique ou bicyclique pentagonal à décagonal, chacun éventuellement substitué (toutefois non exempt de substituant dans le cas de phényle et naphtyle) une à cinq fois par un radical halogéno, alkyle en C₁ à C₈, alcényle en C₂ à C₈, alcynyle en C₂ à C₈, alkoxy en C₁ à C₈, alcényloxy en C₂ à C₈, alcynyloxy en C₃ à C₈, alkylthio en C₁ à C₈, alkylsulfinyle en C₁ à C₆, alkylsulfonyle en C₁ à C₆, halogénalkyle en C₁ à C₆, halogénalkoxy en C₁ à C₆, halogénalcényloxy en C₂ à C₈, alkylidènediyldioxy en C₁ ou C₂, halogénalkylidènediyldioxy en C₁ ou C₂, halogénalkylthio en C₁ à C₄, halogénalkylsulfinyle en C₁ à C₄, halogénalkylsulfonyle en C₁ à C₄, hydroxy, mercapto, nitro, cyano, amino ou par les groupes 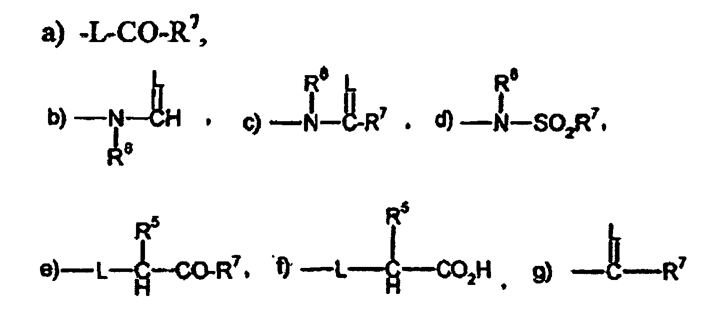 ou représente Ar², auquel cas Ar² est un reste Ar¹ qui est substitué en outre par un reste phényle, naphtyle, hétaryle pentagonal ou hexagonal, phényl-(alkyle en C₁ à C₄), phénoxy, phényl-S(O)g-, hétaryloxy pentagonal
ou hexagonal ou hétaryl-S(O)_{g}, ses substituants étant eux-mêmes éventuellement substitués une à quatre fois par un radical halogéno, alkyle en C₁ à C₆, alkoxy en C₁ à C₆, halogénalkyle en C₁ à C₄, halogénalkoxy en C₁ à C₄, nitro ou cyano, et
K représente l'oxygène ou le soufre,
L représente l'oxygène ou le soufre,
X représente CN, 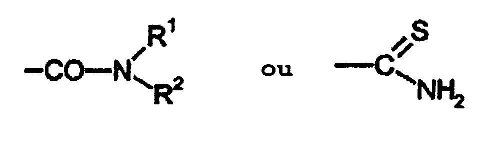
Y est un halogène, un reste alkyle en C₁ à C₆, halogénalkyle en C₁ à C₄, alkoxy en C₁ à C₆, un reste phényle, phényl-(alkyle en C₁ à C₄), hétaryle pentagonal ou hexagonal ou hétaryl-(alkyle en C₁ à C₄) pentagonal ou hexagonal, chacun éventuellement substitué une à quatre fois par un radical halogéno, alkyle en C₁ à C₆, alkoxy en C₁ à C₆, halogénalkyle en C₁ à C₄, halogénalkoxy en C₁ à C₄, cyano ou nitro, ou les groupes
**―CO**_{**2**}**R**^{**1**}
ou 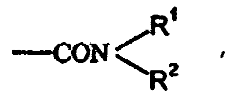 ou bien
deux restes Yₙ contigus représentent un cycle pentagonal à octogonal saturé ou non saturé, qui peut être interrompu par 1 à 3 hétéroatomes de la série N, O ou S et qui peut éventuellement être substitué une à trois fois par un radical halogéno, alkyle en C₁ à C₄, alkoxy en C₁ à C₄, halogénalkyle en C₁ à C₄, halogénalkoxy en C₁ à C₄, cyano ou nitro, et
Z représente l'hydrogène, un reste alkyle en C₁ à C₈, cyano- (alkyle en C₁ à C₆), alcényle en C₃ à C₈, alcynyle en C₃ à C₈, cycloalkyle en C₃ à C₈, (cycloalkyle en C₃ à C₈) - (alkyle en C₁ ou C₂), (alkoxy en C₁ à C₄) - (alkyle en C₁ à C₄), (halogénalkoxy en C₁ à C₄) - (alkyle en C₁ à C₄), chacun éventuellement substitué par du fluor et/ou du chlore, un reste phénoxy- (alkyle en C₁ à C₄), phényl-(alkyloxy en C₁ à C₄) - (alkyle en C₁ à C₄), phénylthio- (alkyle en C₁ à C₄), phényl-(alkylthio en C₁ à C₄)-(alkyle en C₁ à C₄), phényle, phényl-(alkyle en C₁ à C₄), hétaryle pentagonal ou hexagonal, hétaryl-(alkyle en C₁ à C₄) pentagonal ou hexagonal, chacun éventuellement substitué une à quatre fois par un radical halogéno, alkyle en C₁ à C₆, alkoxy en C₁ à C₆, halogénalkyle en C₁ à C₄, halogénalkoxy en C₁ à C₄, nitro ou cyano, ou les groupes
**―CO**_{**2**}**R**^{**1**} **;** 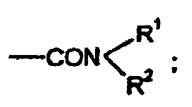
**―SO**_{**2**}**R**^{**1**}**;**
**―COR**^{**1**}**;** 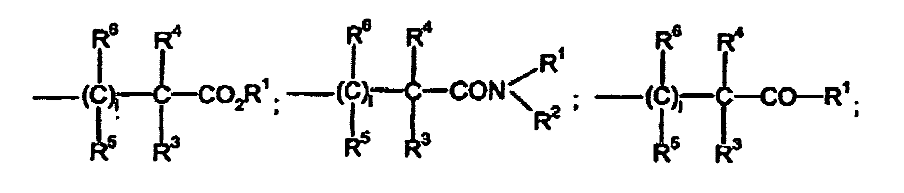
ou le groupe cyano,
g a une valeur de 0 à 2,
l a une valeur de 0 à 2,
R¹ représente l'hydrogène (sauf toutefois pour les restes -CO₂R¹ et -SO₂R¹), un reste alkyle en C₁ à C₁₀, alcényle en C₂ à C₁₀, alcynyle en C₃ à C₆, chacun éventuellement substitué par du fluor et/ou du chlore, un reste cycloalkyle en C₃ à C₈ ou cycloalcényle en C₅ à C₈ dont, le cas échéant, un groupe méthylène peut être interrompu par de l'oxygène ou du soufre et chacun éventuellement substitué par un radical fluoro, chloro, alkyle en C₁ à C₄, alkoxy en C₁ à C₄, ou bien un reste phényle, pyridyle, thiényle, pyrimidyle, thiazolyle, phényl-(alkyle en C₁ à C₄), pyridyl(alkyle en C₁ ou C₂), thiazolyl- (alkyle en C₁ ou C₂), chacun éventuellement substitué une à quatre fois par un radical halogéno, alkyle en C₁ à C₆, alkoxy en C₁ à C₆, halogénalkyle en C₁ à C₄, halogénalkoxy en C₁ à C₄, cyano ou nitro,
R² représente l'hydrogène, un reste alkyle en C₁ à C₆, alcényle en C₃ à C₆, alcynyle en C₃ à C₆, alkoxy en C₁ à C₆, alcényloxy en C₃ à C₆, chacun éventuellement substitué par du fluor et/ou du chlore, ou un reste phényle, phényl-(alkyle en C₁ à C₄) ou phényl-(alkoxy en C₁ à C₄), chacun éventuellement substitué une à quatre fois par un radical halogéno, alkyle en C₁ à C₆, alkoxy en C₁ à C₆, halogénalkyle en C₁ à C₄, halogénalkoxy en C₁ à C₄, cyano ou nitro, ou bien
R¹, R² forment, conjointement avec l'atome d'azote auquel ils sont liés, un cycle pentagonal à octogonal éventuellement substitué par un radical alkyle en C₁ à C₄ et dans lequel, le cas échéant, un groupe méthylène peut être remplacé par de l'oxygène ou du soufre,
R³ représente l'hydrogène, un reste alkyle en C₁ à C₆ éventuellement substitué par un halogène ou un reste phényle ou phényl-(alkyle en C₁ ou C₂) éventuellement substitué une à quatre fois par un radical halogéno, alkyle en C₁ à C₆, alkoxy en C₁ à C₆, halogénalkyle en C₁ à C₄, halogénalkoxy en C₁ à C₄, cyano ou nitro,
R⁴ représente l'hydrogène ou un reste alkyle en C₁ à C₆,
R⁵, R⁶ représentent indépendamment l'un de l'autre l'hydrogène ou un reste alkyle en C₁ à C₄ éventuellement substitué par du fluor et/ou du chlore,
R⁷ représente un reste alkyle en C₁ à C₁₀, alcényle en C₂ à C₁₀, alcynyle en C₃ à C₁₀, alkoxy en Ci à C₁₀, (alkoxy en C₁ à C₄)-(alkyle en C₁ à C₄), (alkylthio en C₁ à C₄)-(alkyle en C₁ à C₄), chacun éventuellement substitué par du fluor et/ou du chlore, un reste cycloalkyle en C₃ à C₈ ou cycloalkyloxy en C₃ à C₈ dont, le cas échéant, un groupe méthylène peut être remplacé par de l'oxygène ou du soufre et chacun étant éventuellement substitué par du fluor et/ou du chlore, un reste phényle, phénoxy, benzyloxy, hétaryle pentagonal ou hexagonal ou phényl-(alkyle en C₁ à C₄) chacun éventuellement substitué une à quatre fois par un radical halogéno, alkyle en C₁ à C₆, alkoxy en C₁ à C₆, halogénalkyle en C₁ à C₄, halogénalkoxy en C₁ à C₄, cyano ou nitro, ou bien dans le cas des restes a) et c) mentionnés pour Ar, également un groupe 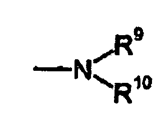 ou, pour le reste g), également un groupe hydroxy,
R⁸ représente l'hydrogène ou un reste alkyle en C₁ à C₄,
R⁹ représente l'hydrogène, un reste alkyle en C₁ à C₁₀, alcényle en C₃ à C₈, alcynyle en C₃ à C₈, alkoxy en C₁ à C₁₀, alcényloxy en C₃ à C₈, chacun éventuellement substitué par du fluor et/ou du chlore, un reste cycloalkyle en C₃ à C₈ éventuellement substitué par un radical fluoro, chloro, alkyle en C₁ à C₄ ou alkoxy en C₁ à C₄ et dont, le cas échéant, un groupe méthylène peut être remplacé par de l'oxygène ou du soufre, un reste phényle, phényl-(alkyle en C₁ à C₄) ou phényl-(alkoxy en C₁ ou C₂), chacun éventuellement substitué une à quatre fois par un radical halogéno, alkyle en C₁ à C₆, alkoxy en C₁ à C₆, halogénalkyle en C₁ à C₄, halogénalkoxy en C₁ à C₄, cyano ou nitro,
R¹⁰ représente l'hydrogène, un reste alkyle en C₁ à C₆ ou alcényle en C₃ à C₆, ou bien
R⁹, R¹⁰ forment, conjointement avec l'atome d'azote auquel ils sont liés, un cycle pentagonal à octogonal éventuellement substitué par un radical alkyle en C₁ à C₄ et dans lequel, le cas échéant, un groupe méthylène peut être remplacé par de l'oxygène ou du soufre, et
m a une valeur de 1 à 3, et
n a, en fonction de m, une valeur de 0 à 3,
excepté les composés de formule suivante 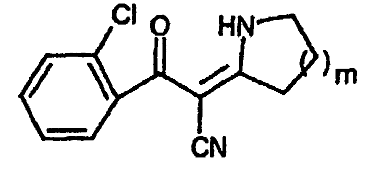 m = 1, 2, 3.

2. Composés suivant la revendication 1, **caractérisés en ce que**
K représente l'oxygène ou le soufre,
Ar représente Ar¹, auquel cas Ar¹ représente un reste phényle, naphtyle, quinolinyle, thiényle, pyrimidyle, furannyle, thiazolyle, benzothiazolyle, oxazolyle, pyrazolyle ou pyridyle, chacun éventuellement substitué (toutefois non exempt de substituant dans le cas de phényle et naphtyle) une à trois fois par un radical fluoro, chloro, bromo, alkyle en C₁ à C₆, alcényle en C₂ à C₆, alcynyle en C₂ à C₆, alkoxy en C₁ à C₆, alcényloxy en C₃ à C₆, alcynyloxy en C₃ à C₆, alkylthio en C₁ à C₆, alkylsulfinyle en C₁ à C₄, alkylsulfonyle en C₁ à C₄, halogénalkyle en C₁ à C₄, halogénalkoxy en C₁ à C₄, halogénalcényloxy en C₂ à C₄, alkylidènediyldioxy en C₁ ou C₂, halogénalkylidènediyldioxy en C₁ ou C₂, halogénalkylthio en C₁ ou C₂, halogénalkylsulfinyle en C₁ ou C₂, halogénalkylsulfonyle en C₁ ou C₂, hydroxy, mercapto, nitro, cyano, amino ou par l'un des groupes suivants 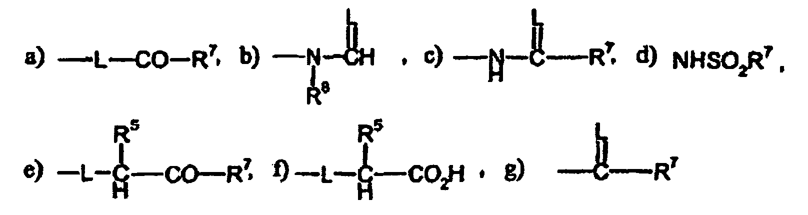 ou représente Ar², auquel cas Ar² est un reste Ar¹ qui est en outre substitué par un radical phényle, pyridyle, pyrimidyle, thiényle, furannyle, thiazolyle, tétrazolyle, triazolyle, benzyle, phénoxy, phényl-S(O)_{g}-, pyridyloxy, pyrimidyloxy, thiazolyloxy, pyridyl-S(O)_{g}-, pyrimidyl-S(O)_{g}- ou thiazolyl-S(O)_{g}-, ses substituants étant eux-mêmes substitués le cas échéant une à trois fois par un radical fluoro, chloro, bromo, alkyle en C₁ à C₄, alcényle en C₂ à C₄, alkoxy en C₁ à C₄, halogénalkyle en C₁ ou C₂, halogénalkoxy en C₁ ou C₂, nitro ou cyano, g ayant une valeur de 0 à 2, et
L représente l'oxygène ou le soufre,
X est un groupe CN, 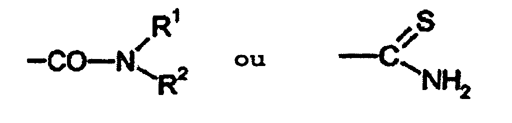
Y représente le fluor, un reste alkyle en C₁ à C₄, halogénalkyle en C₁ ou C₂, alkoxy en C₁ à C₄, un reste phényle, phényl-(alkyle en C₁ ou C₂), thiazolylméthyle, pyridyln-éthyle, chacun éventuellement substitué une à trois par un radical fluoro, chloro, bromo, alkyle en C₁ à C₄, alkoxy en C₁ à C₄, halogénalkyle en C₁ ou C₂, halogénalkoxy en C₁ ou C₂, cyano ou nitro, ou représente les groupes 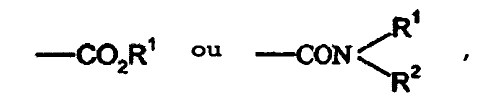 ou bien
deux restes Yₙ contigus représentent en outre un cycle pentagonal ou hexagonal saturé ou non saturé qui peut être interrompu par un hétéroatome de la série N, O, S et qui peut éventuellement être substitué une ou deux fois par un radical fluoro, chloro, bromo, méthyle, tertiobutyle, méthoxy, trifluorométhyle, trifluorométhoxy, cyano ou nitro, et
Z représente l'hydrogène, un reste alkyle en C₁ à C₆, cyano(alkyle en C₁ à C₃), alcényle en C₃ à C₆, alcynyle en C₃ à C₈, cycloalkyle en C₃ à C₆, (cycloalkyle en C₃ à C₆)-(alkyle en C₁ ou C₂), (alkoxy en C₁ à C₄)-(alkyle en C₁ ou C₂), (halogène en C₁ à C₄)-(alkyle en C₁ ou C₂), un reste phénoxy-(alkyle en C₁ ou C₂), phényl-(alkyloxy en C₁ ou C₂) - (alkyle en C₁ ou C₂), phénylthio-(alkyle en C₁ ou C₂), phényl-(alkylthio en C₁ ou C₂) - (alkyle en C₁ ou C₂), phényl-(alkyle en C₁ ou C₂), phényle, pyridyl-(alkyle en C₁ ou C₂), thiazolyl-(alkyle en C₁ ou C₂), chacun éventuellement substitué une à trois fois par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano, ou les groupes
**―CO**_{**2**}**R**^{**1**} **;** 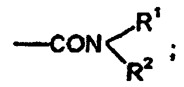
**―SO**_{**2**}**R**^{**1**} **;**
**―COR**^{**1**}**;** 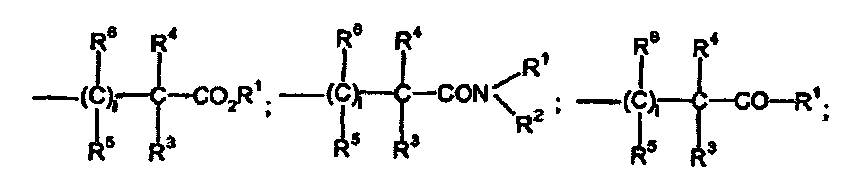
ou le groupe cyano,
l a la valeur 0 ou 1,
R¹ représente l'hydrogène (non toutefois dans les restes -CO₂R¹ et -SO₂R¹), un reste alkyle en C₁ à C₆, alcényle en C₂ à C₆, alcynyle en C₃ ou C₄, chacun éventuellement substitué par du fluor et/ou du chlore, un reste cycloalkyle en C₃ à C₆ éventuellement substitué par un radical fluoro, chloro, alkyle en C₁ ou C₂, alkoxy en C₁ ou C₄, ou un reste phényle ou benzyle, chacun éventuellement substitué une ou deux fois par un radical fluoro, chloro, bromo, alkyle en C₁ à C₄, alkoxy en C₁ à C₄, halogénalkyle en C₁ ou C₂, halogénalkoxy en C₁ ou C₂, cyano ou nitro,
R² représente l'hydrogène, un reste alkyle en C₁ à C₄, alcényle en C₃ ou C₄, alcynyle en C₃ ou C₄, alkoxy en C₁ à C₄, alcényloxy en C₃ ou C₄, chacun éventuellement substitué par du fluor et/ou du chlore, ou un reste phényle, benzyle ou benzyloxy, chacun éventuellement substitué une ou deux fois par un radical fluoro, chloro, bromo, alkyle en C₁ à C₄, alkoxy en C₁ à C₄, halogénalkyle en C₁ ou C₂, halogénalkoxy en C₁ ou C₂, cyano ou nitro, ou bien
R¹, R² forment conjointement avec l'atome d'azote auquel ils sont liés un cycle pentagonal ou hexagonal éventuellement substitué par un radical alkyle en C₁ ou C₂ et dans lequel un groupe méthylène peut éventuellement être remplacé par de l'oxygène,
R³ représente l'hydrogène, un reste alkyle en C₁ à C₄ ou un reste phényle ou benzyle, chacun éventuellement substitué une ou deux fois par un radical fluoro, chloro, bromo, alkyle en C₁ à C₄, alkoxy en C₁ à C₄, trifluorométhyle, difluorométhoxy, trifluorométhoxy, cyano ou nitro,
R⁴, R⁵, R⁶ représentent l'hydrogène, un reste méthyle ou éthyle,
R⁷ représente un reste alkyle en C₁ à C₆, alcényle en C₂ à C₆, alcynyle en C₃ à C₆, alkoxy en C₁ à C₆, (alkoxy en C₁ à C₄)-(alkyle en C₁ ou C₂), (alkylthio en C₁ à C₄)-(alkyle en C₁ ou C₂), chacun éventuellement substitué par du fluor et/ou du chlore, un reste cycloalkyle en C₃ à C₆ éventuellement substitué par du fluor et/ou du chlore, un radical alkyle en C₁ ou C₂, alkoxy en C₁ ou C₂, et dont un groupe méthylène peut éventuellement être remplacé par de l'oxygène, un reste phényle, phénoxy, benzyloxy, thiényle, furannyle, pyridyle, pyrimidyle, thiazolyle, pyrazolyle ou phényl-(alkyle en C₁ ou C₂), chacun éventuellement substitué une ou deux fois par un radical fluoro, chloro, bromo, alkyle en C₁ à C₄, alkoxy en C₁ à C₄, trifluorométhyle, difluorométhoxy, trifluorométhoxy, cyano ou nitro, ou bien, dans le cas d'un reste a) et c) mentionné pour Ar, également un groupe 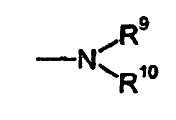 ou, pour le reste g), également un groupe hydroxy,
R⁸ représente l'hydrogène,
R⁹ représente l'hydrogène, un reste, éventuellement substitué par du fluor et/ou du chlore, alkyle en C₁ à C₆, alcényle en C₃ à C₆, alcynyle en C₃ à C₆, alkoxy en C₁ à C₆, cycloalkyle en C₃ à C₆ dans lequel, le cas échéant, un groupe méthylène peut être remplacé par de l'oxygène, un reste phényle ou phényl-(alkyle en C₁ ou C₂), chacun éventuellement substitué une ou deux fois par un radical fluoro, chloro, bromo, alkyle en C₁ à C₄, alkoxy en C₁ à C₄, trifluorométhyle, difluorométhoxy, trifluorométhoxy, cyano ou nitro,
R¹⁰ représente l'hydrogène ou un reste alkyle en C₁ à C₄, ou bien
R⁹, R¹⁰ forment, conjointement avec l'atome d'azote auquel ils sont liés, un cycle pentagonal ou hexagonal éventuellement substitué par un radical alkyle en C₁ ou C₂ et dans lequel, le cas échéant, un groupe méthylène peut être remplacé par de l'oxygène, et
m a une valeur de 0 à 1, et
n a, en fonction de m, une valeur de 0 à 2.

3. Composés suivant la revendication 1, **caractérisés en ce que**
K représente l'oxygène ou le soufre,
Ar représente Ar¹, auquel cas Ar¹ représente un reste phényle, thiényle, pyrimidyle, furannyle ou pyridyle, chacun éventuellement substitué (toutefois non exempt de substituant dans le cas de phényle et naphtyle) une à trois fois par un radical fluoro, chloro, bromo, méthyle, éthyle, propyle, isopropyle, sec.-butyle, n-butyle, isobutyle, tertiobutyle, méthoxy, éthoxy, propoxy, isopropoxy, sec.-butoxy, n-butoxy, isobutoxy, tertiobutoxy, allyloxy, métallyloxy, 2-butényloxy, propargyloxy, 2-butynyloxy, méthylthio, éthylthio, méthylsulfinyle, éthylsulfinyle, méthylsulfonyle, éthylsulfonyle, trifluorométhyle, difluorométhoxy, trifluorométhoxy, méthylènedioxy, difluorométhylènedioxy, tétrafluoréthylènedioxy, difluorométhylthio, trifluorométhylthio, trifluorométhylsulfinyle, trifluorométhylsulfonyle, hydroxy, mercapto, nitro, cyano, amino ou par l'un des groupes suivants 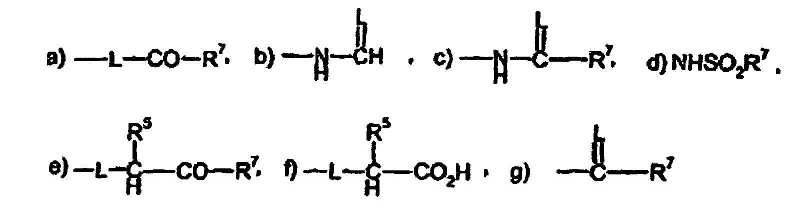 ou représente Ar², auquel cas Ar² est un reste Ar¹ qui est en outre substitué par un radical phényle, pyridyle, thiényle, tétrazolyle, triazolyle ou phénoxy, ses substituants étant eux-mêmes éventuellement substitués une ou deux fois par un radical fluoro, chloro, bromo, méthyle, éthyle, n-propyle, isopropyle, sec.-butyle, n-butyle, isobutyle, tertiobutyle, méthoxy, éthoxy, isopmpoxy, sec. -butoxy, n-butoxy, tertiobutoxy, trifluorométhyle, trifluorométhoxy, nitro ou cyano, et
L représente l'oxygène ou le soufre,
X représente
**―CN,**
**―CO―NH**_{**2**}**,** 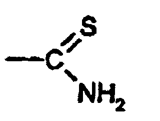
Y est un reste méthyle, un reste phényle éventuellement substitué une à trois fois par un radical fluoro, chloro, bromo, méthyle, méthoxy, trifluorométhyle ou trifluorométhoxy, ou le groupe 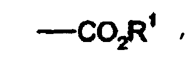 ou bien
deux restes Yₙ contigus représentent un cycle hexagonal non saturé qui peut être éventuellement substitué une fois par un radical fluoro, chloro, bromo, méthyle, méthoxy, trifluorométhyle, trifluorométhoxy, cyano ou nitro, et
Z représente l'hydrogène, un reste méthyle, éthyle, allyle, propargyle, cyclopropyle, cyclopentyle, cyclohexyle, cyclopropylméthyle, cyclopentylméthyle, cyclohexylméthyle, méthoxyméthyle, éthoxyméthyle, un reste phényle, benzyle, pyridylméthyle, thiazolylméthyle, éventuellement substitué une ou deux fois par un radical fluoro, chloro, bromo, méthyle, méthoxy, trifluorométhyle, trifluorométhoxy, cyano ou nitro, ou les groupes
**―CO**_{**2**}**R**^{**1**}**,** 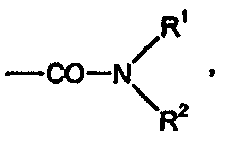
**―SO**_{**2**}**R**^{**1**} **,**
**―CO―R**^{**1**} **,** 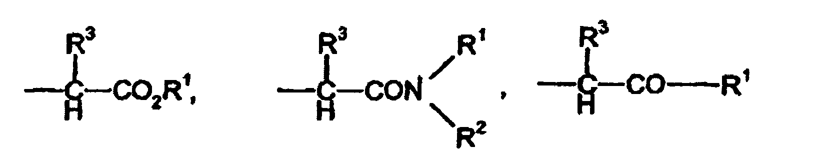
ou cyano,
R¹ représente l'hydrogène (non toutefois dans les restes -CO₂R¹ et -SO₂R¹), un reste méthyle, éthyle, propyle, isopropyle, n-butyle, sec.-butyle, isobutyle, tertiobutyle, allyle, propargyle, cyclopropyle, cyclopentyle, cyclohexyle, un reste phényle ou benzyle, chacun éventuellement substitué une ou deux fois par un radical fluoro, chloro, bromo, méthyle, méthoxy, trifluorométhyle, trifluorométhoxy, cyano ou nitro,
R² représente l'hydrogène, un reste méthyle, éthyle, propyle, isopropyle, allyle, propargyle, méthoxy, éthoxy, allyloxy, ou un reste benzyloxy éventuellement substitué une ou deux fois par un radical fluoro, chloro, bromo, méthyle, méthoxy, trifluorométhyle, trifluorométhoxy, cyano ou nitro, ou bien
R¹, R² forment, conjointement avec l'atome d'azote auquel ils sont liés, un reste pyrrolidine, pipéridine, thiazine ou morpholine,
R³, R⁵ représentent l'hydrogène, un reste méthyle ou éthyle,
R⁷ représente un reste méthyle, éthyle, propyle, isopropyle, n-butyle, sec.-butyle, isobutyle, tertiobutyle, vinyle, trifluorométhyle, méthoxy, éthoxy, propoxy, isopropoxy, n-butyloxy, sec.-butylaxy, isobutyloxy, tertiobutyloxy, cyclopropyle, cyclopentyle, cyclohexyle, cyclopentyloxy, cyclohexyloxy, un reste phényle, pyridyle ou benzyle, chacun éventuellement substitué une ou deux fois par un radical fluoro, chloro, bromo, méthyle, n-butyle, sec.-butyle, isobutyle, tertiobutyle, méthoxy, trifluorométhyle, trifluorométhoxy, cyano ou nitro ou bien, dans le cas des restes a) et c) mentionnés pour Ar, également un groupe 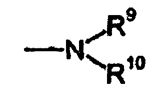 ou, pour le reste g), également un groupe hydroxy,
R⁹ représente l'hydrogène, un reste méthyle, éthyle, propyle, isopropyle, n-butyle, sec.-butyle, isobutyle, tertiobutyle, cyclopropyle, cyclopentyle, cyclohexyle, un reste phényle éventuellement substitué une ou deux fois par un radical fluoro, chloro, bromo, méthyle, méthoxy, trifluorométhyle, trifluorométhoxy, cyano ou nitro,
R¹⁰ représente l'hydrogène, un reste méthyle ou éthyle, ou bien
R⁹, R¹⁰ forment, conjointement avec l'atome d'azote auquel ils sont liés, un reste pyrrolidine, pipéridine ou morpholine, et
m a une valeur de 1 à 3, et
n indépendamment de m, a une valeur de 0 à 1.

4. Composés suivant la revendication 1, **caractérisés en ce que**
K représente l'oxygène ou le soufre,
Ar représente un reste Ar¹, auquel cas Ar¹ est un reste phényle, thiényle, pyrimidyle ou pyridyle, chacun éventuellement substitué une à trois fois par un radical fluoro, chloro, bromo, méthyle, éthyle, propyle, isopropyle, n-butyle, sec.-butyle, isobutyle, tertiobutyle, méthoxy, éthoxy, propoxy, isopropoxy, tertiobutoxy, allyloxy, métallyloxy, 2-butényloxy, propargyloxy, 2-butinyloxy, méthylthio, éthylthio, méthylsulfinyle, éthylsulfinyle, méthylsulfonyle, éthylsulfonyle, trifluorométhyle, difluorométhoxy, trifluorométhoxy, méthylènedioxy, difluorométhylènedioxy, tétrafluoréthylènedioxy, difluorométhylthio, trifluorométhylthio, trifluorométhylsulfinyle, trifluorométhylsulfonyle, hydroxy, mercapto, nitro, cyano, amino,
ou représente Ar², auquel cas Ar² est un reste Ar¹ qui porte en outre un substituant phényle ou phénoxy, ces substituants étant eux-mêmes éventuellement substitués une ou deux fois par un radical fluoro, chloro, bromo, méthyle, éthyle, isopropyle, n-butyle, sec.-butyle, isobutyle, tertiobutyle, méthoxy, éthoxy, isopropoxy, n-butoxy, sec.-butoxy, isobutoxy, tertiobutoxy, trifluorométhyle, trifluorométhoxy, nitro ou cyano, et
X représente CN,
Z représente l'hydrogène ou le reste méthyle,
m a une valeur de 1 à 3, et
n a la valeur 0.

5. Procédé de production de composés suivant l'une des revendications 1 à 4, **caractérisé en ce que** :
(A) au cas où K représente l'oxygène et Z représente l'hydrogène,
on fait réagir des composés de formule (II), 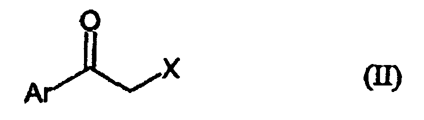 dans laquelle
Ar et X ont les définitions indiquées dans l'une des revendications 1 à 5
avec des composés de formule (III), 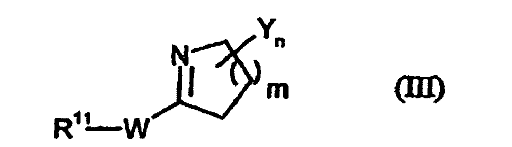 dans laquelle
Y, m, n ont les définitions indiquées dans l'une des revendications 1 à 5,
et
W représente O ou S(O)_{g}, où g a la valeur 0 ou 2, et
R¹¹ est un reste alkyle ou benzyle,
éventuellement en présence d'un diluant et le cas échéant en présence d'une base ou d'un composé métallique de formule (IIIa),
**Me(V)**_{**2**} **(IIIa)**
dans laquelle
Me est un atome de métal de transition divalent, et
V représente un ligand de chélation,
ou bien
(B) au cas où K représente l'oxygène et Z représente l'hydrogène,
on fait réagir des composés de formule (IV), 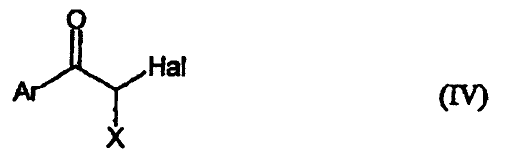 dans laquelle
Ar et X on la définition indiquée dans l'une des revendications 1 à 5, et
Hal représente un halogène,
avec des composés de formule (V), 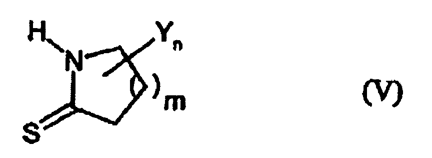
dans laquelle
Y, m et n ont les définitions indiquées dans l'une des revendications 1 à 5,
éventuellement en présence d'un diluant, pour obtenir des composés de formule (VI), 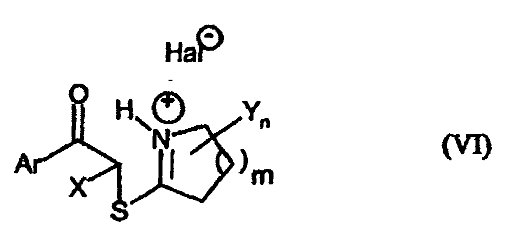 dans laquelle
Ar, X, Y, et n m ont les définitions indiquées dans les revendications 1 à 5,
qu'on fait ensuite réagir éventuellement en présence d'une base et, le cas échéant, en présence d'un composé de phosphore trivalent avec élimination de soufre et d'halogénure d'hydrogène pour obtenir des composés de formule (I)
dans laquelle
Ar, X et Y, m et n ont les définitions indiquées dans l'une des revendications 1 à 5 et
Z représente l'hydrogène,
ou bien
(C) au cas où K représente l'oxygène et Z ne représente pas l'hydrogène,
on fait réagir des composés de formule (VII), 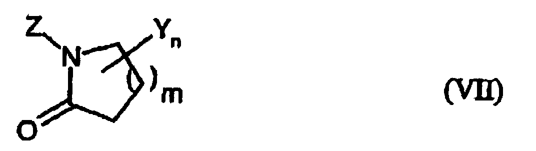 dans laquelle
Y, m et n ont les définitions indiquées dans l'une des revendications 1 à 5,
avec des agents d'halogénation, éventuellement en présence d'un diluant, pour obtenir des composés de formule (VIII), 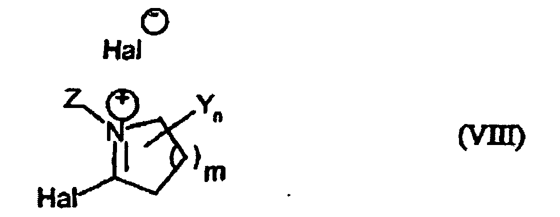 dans laquelle
Y, Z, m et n ont les définitions indiquées dans l'une des revendications 1 à 5,
et Z ne représente pas l'hydrogène, et
Hal est un halogène,
qu'on fait ensuite réagir avec des composés de formule (II), 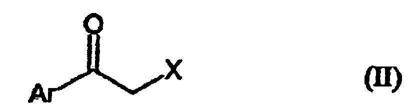 dans laquelle
Ar, X ont les définitions indiquées dans l'une des revendications 1 à 5,
éventuellement en présence d'un diluant et, le cas échéant, en présence d'un accepteur d'acide,
ou bien
(D) au cas où K représente l'oxygène et Z ne représente pas l'hydrogène,
on fait réagir des composés de formule (I-a), 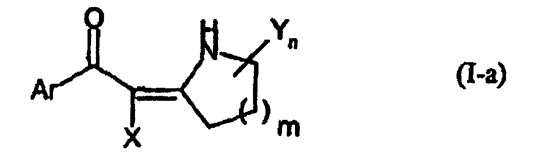 dans laquelle
Ar, X, Y, m et n ont les définitions indiquées dans l'une des revendications 1 à 5, avec
un agent d'alkylation, des agents d'acylation, des agents de sulfonylation ou des agents de condensation de formule (IX)
**Z-G (IX),**
dans laquelle
G est un groupe partant tel qu'halogène, sulfonate ou alkoxy,
éventuellement en présence d'un solvant et, le cas échéant, en présence d'une base,
ou bien
(E) au cas où Ar représente Ar² conformément à l'une des revendications 1 à 5,
on fait réagir des composés de formule (I¹) 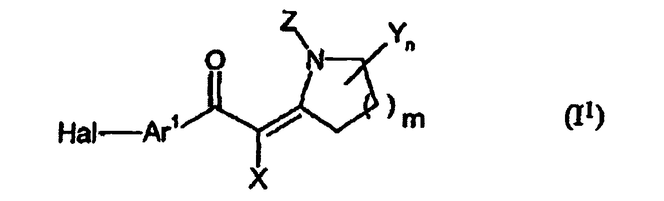 dans laquelle
Ar¹, X, Y, Z, m et n ont les définitions indiquées dans l'une des revendications 1 à 5, et
Hal représente un halogène,
avec des acides boroniques de formule (X)
**Ar**^{**2'**}**―B(OH)**_{**2**} **(X),**
dans laquelle
Ar² est tel que défini dans l'une des revendications 2 à 5,
Ar^{2'} représente les substituants qui ont été mentionnés dans la revendication 1 comme substituants supplémentaires pour Ar¹
en présence d'un solvant, le cas échéant en présence d'une base et/ou d'un complexe de métal noble,
ou bien
(F) on fait des composés de formule (I) 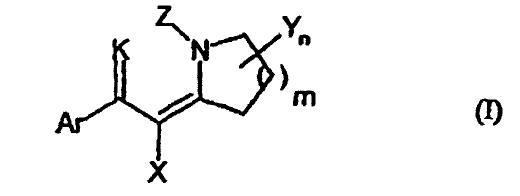 dans laquelle
Ar, X, Y, Z, m et n ont la définition indiquée dans l'une des revendications 1 à 5 et K représente l'oxygène,
en présence d'un réactif de sulfuration et en présence d'un solvant.

6. Compositions herbicides, acaricides et/ou insecticides, **caractérisées par** une teneur en au moins un composé selon l'une des revendications 1 à 5.

7. Utilisation de composés selon l'une des revendications 1 à 5 pour combattre une végétation indésirable et/ou des parasites animaux.

8. Composés de formule (V), 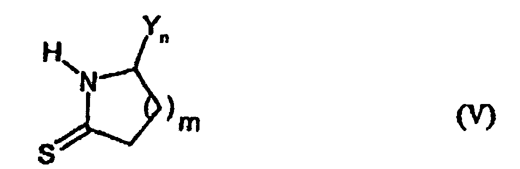 dans laquelle Yₙ est un reste phényle éventuellement substitué, et m a la valeur 1 ou 2,
excepté les composés 5-phénylpyrrolidine-2-thione et 5-(3,4-diméthoxyphényl)-pyrrolidine-2-thione.

9. Composés de formule (II-1-b), 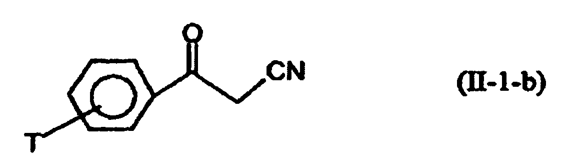 dans laquelle
T a la définition indiquée dans le tableau suivant :

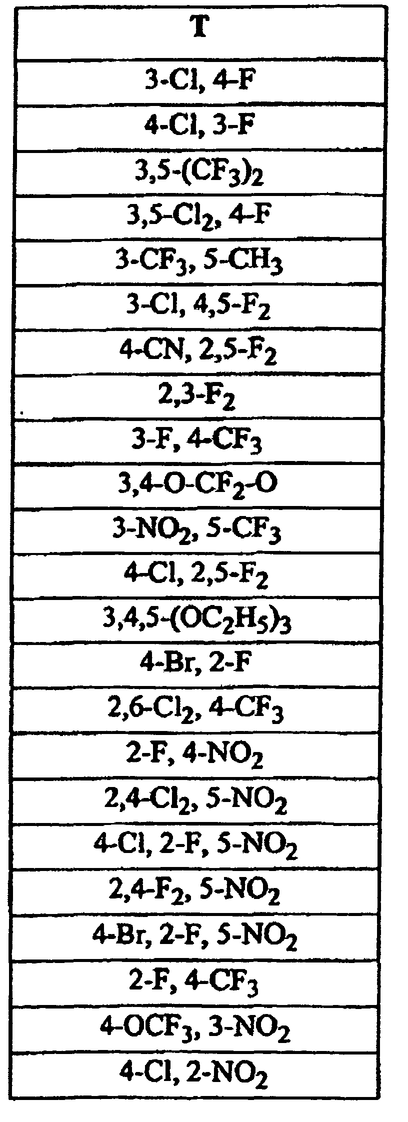

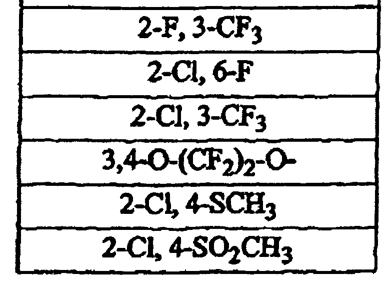

10. Composés de formule (II-2-b), 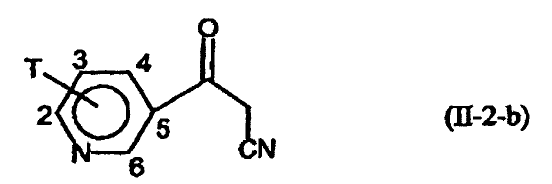 dans laquelle T a la définition indiquée dans le tableau suivant :
